(19) Europäisches Patentamt
European Patent Office
Office européen des brevets

(11) Publication number: **0 254 951 B1**

# (12) EUROPEAN PATENT SPECIFICATION

(45) Date of publication of patent specification: **20.09.95**

(51) Int. Cl.[6]: **C07D 401/04**, C07D 471/04, C07D 471/14, C07D 213/81, C07D 405/04, C07D 409/04, C07D 405/14, C07D 409/14, A01N 43/40, A01N 43/90, //(C07D471/04,221:00,209:00), (C07D471/14,235:00,221:00, 209:00)

(21) Application number: **87110150.7**

(22) Date of filing: **14.07.87**

The file contains technical information submitted after the application was filed and not included in this specification

## (54) 5(and/or 6)substituted 2-(2-imidazolin-2-yl)nicotinic acids, esters and salts, useful as herbicidal agents and intermediates for the preparation of said nicotinic acids, esters and salts.

(30) Priority: **28.07.86 US 889999**

(43) Date of publication of application:
**03.02.88 Bulletin 88/05**

(45) Publication of the grant of the patent:
**20.09.95 Bulletin 95/38**

(84) Designated Contracting States:
**AT BE CH DE ES FR GB GR IT LI LU NL SE**

(56) References cited:
**EP-A- 0 133 309**
**EP-A- 0 166 907**
**EP-A- 0 296 109**
**GB-A- 2 192 877**

**CHEMICAL ABSTRACTS, vol. 108, 1988, page 663, abstract no. 94556j, Columbus,Ohio, US; & JP-A-62 174 069**

(73) Proprietor: **AMERICAN CYANAMID COMPANY**
**One Cyanamid Plaza**
**Wayne, NJ 07470-8426 (US)**

(72) Inventor: **Doehner, Robert Francis, Jr.**
**733 Windsor-Perrineville Road**
**East Windsor**
**New Jersey 08520 (US)**

(74) Representative: **Wächtershäuser, Günter, Prof. Dr.**
**Patentanwalt,**
**Tal 29**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 254 951 B1

## Description

SUMMARY OF THE INVENTION

This invention relates to herbicidal, 5(and/or 6) substituted 2-(2-imidazolin-2-yl)nicotinic acids, esters and salts depicted by formula I and 5(and/or 6)substituted 2-(2-imidazolidinyl)nicotinic acids, esters and salts depicted by formula II,

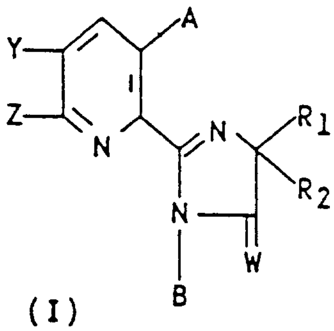

(I)

and

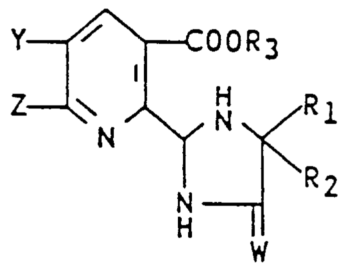

(II) ;

wherein

| | |
|---|---|
| $R_1$ | is $C_1$-$C_4$ alkyl; |
| $R_2$ | is $C_1$-$C_4$ alkyl; |
| A | is $COOR_3$, $CONHR_6$, CHO, $CH_2OH$, $COCH_3$, $COC_6H_5$, CN, $CH_3$, CH = NOH, $CH_2COOH$, CONHOH, $CH_2CH_2COOH$, $CHR_8OH$, |

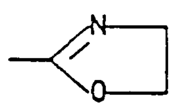

or

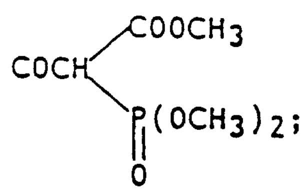

$R_3$ is hydrogen,

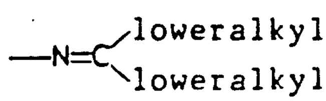

$C_1$-$C_{12}$ alkyl optionally substituted with one of the following groups: $C_1$-$C_3$ alkoxy, $C_1$-$C_3$ alkylthio, halogen, hydroxyl, $C_3$-$C_6$ cycloalkyl, benzyloxy, furyl, phenyl, halophenyl, loweralkylphenyl, loweralkoxyphenyl, nitrophenyl, carboxyl, loweralkoxycarbonyl, cyano or triloweralkylammonium halide;

$C_3$-$C_{12}$ alkenyl optionally substituted with one of the following groups: $C_1$-$C_3$ alkoxy, phenyl, halogen or loweralkoxycarbonyl or with two $C_1$-$C_3$ alkoxy groups or two halogen groups;

$C_3$-$C_6$ cycloalkyl optionally substituted with one or two $C_1$-$C_3$ alkyl groups;

$C_3$-$C_{16}$ alkynyl optionally substituted with one or two $C_1$-$C_3$ alkyl groups; or

a cation;

| | |
|---|---|
| $R_6$ | is hydrogen, hydroxyl, $C_3$-alkenyl, $C_3$-alkynyl or $C_1$-$C_4$ alkyl optionally substituted with one hydroxyl or one chlorine group; |
| B | is H, $COR_4$ or $SO_2R_5$, provided that when B is $COR_4$ or $SO_2R_5$; A is $COOR_3$ in which $R_3$ is other than H or a cation, $CH_3$ or CN; W is O; and Y and Z are not alkylamino, hydroxyl, or hydroxyloweralkyl; |

$R_4$ is $C_1$-$C_{11}$ alkyl, chloromethyl or phenyl optionally substituted with one chloro, one nitro or one methoxy group;
$R_5$ is $C_1$-$C_4$ alkyl or phenyl optionally substituted with one methyl group;
W is O;
$R_8$ is $C_1$-$C_4$-alkyl or phenyl;
Y and Z are each hydrogen, halogen, $C_1$-$C_6$ alkyl, $C_1$-$C_4$ hydroxyloweralkyl, $C_1$-$C_6$ alkoxy, $C_1$-$C_4$ alkylthio, formyl, phenoxy, $C_1$-$C_4$ haloalkyl, nitro, cyano, $C_1$-$C_4$ alkylamino, di-$C_1$-$C_4$ loweralkylamino, $C_1$-$C_4$ alkylsulfonyl, difluoromethoxy, trifluoromethoxy, 1,1,2,2-tetra-fluoroethoxy, phenyl optionally substituted with one
$C_1$-$C_4$ alkyl, $C_1$-$C_4$ alkoxy or halogen;
$C_3$-$C_8$ straight or branched alkenyloxy optionally substituted with one to three halogens;
$C_3$-$C_8$ straight or branched alkylnyloxy optionally substituted with one to three halogens;
$C_2$-$C_6$ straight or branched alkenyl optionally substituted with phenyl, $C_1$-$C_4$ alkoxy or 1 to 3 halogens;
$C_2$-$C_6$ straight or branched alkynyl optionally substituted with phenyl, $C_1$-$C_4$ alkoxy or 1 to 3 halogens;
$C_3$-$C_6$ cycloalkyl optionally substituted with methyl, halogen or $C_1$-$C_4$ alkoxy;
$C_1$-$C_4$ alkylcarbonyl optionally substituted with $C_1$-$C_4$ alkoxy or 1 to 3 halogens;
$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;
$C_1$-$C_4$ alkylcarbonyloxy optionally substituted with $C_1$-$C_4$ alkoxy or 1 to 3 halogens;
$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;
2(or 3)-furyl optionally substituted with halogen or $C_1$-$C_3$ alkyl;
2(or 3)-thienyl optionally substituted with halogen or $C_1$-$C_3$ alkyl;
N-methyl-2(or 3)-pyrrolyl;
hydroxy;
amino optionally substituted with $C_1$-$C_4$ alkoxy or $C_1$-$C_2$ alkyl and $C_1$-$C_4$ alkoxy;
2(or 4)-pyridyloxy optionally substituted with $C_1$-$C_4$ alkyl, trifluoromethyl or 1 or 2 halogens;
$C_1$-$C_4$ mono or dialkylaminocarbonylamino, optionally substituted on the nitrogen attached to the ring with $C_1$-$C_2$ alkyl or $C_1$-$C_4$ alkoxy;
$C_1$-$C_4$ alkoxycarbonylamino, optionally substituted on nitrogen with $C_1$-$C_2$ alkyl or $C_1$-$C_4$ alkoxy;
$C_1$-$C_4$ mono or dialkylaminocarbonyloxy;

Provided that at least one of Y and Z is:
$C_2$-$C_6$ straight or branched alkenyl optionally substituted with phenyl, $C_1$-$C_4$ alkoxy or 1 to 3 halogens;
$C_2$-$C_6$ straight or branched alkynyl optionally substituted with phenyl, $C_1$-$C_4$ alkoxy or 1 to 3 halogens;
$C_3$-$C_6$ cycloalkyl optionally substituted with methyl, halogen or $C_1$-$C_4$ alkoxy;
$C_1$-$C_4$ alkylcarbonyl optionally substituted with $C_1$-$C_4$ alkoxy or 1 to 3 halogens;
$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;
$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;
2(or 3)-furyl optionally substituted with halogen or $C_1$-$C_3$ alkyl;
2(or 3)-thienyl optionally substituted with halogen or $C_1$-$C_3$ alkyl;
N-methyl-2(or 3)-pyrrolyl;
hydroxy;
amino optionally substituted with $C_1$-$C_4$ alkoxy, or $C_1$-$C_2$ alkyl and $C_1$-$C_4$ alkoxy; or
2(or 4)-pyridyloxy optionally substituted by $C_1$-$C_4$ alkyl, trifluoromethyl or 1 to 2 halogens; and
$C_1$-$C_4$ mono or dialkylaminocarbonylamino, optionally substituted on the nitrogen attached to the ring with $C_1$-$C_2$ alkyl or $C_1$-$C_4$ alkoxy;
$C_1$-$C_4$ alkoxycarbonylamino, optionally substituted on nitrogen with $C_1$-$C_2$ alkyl or $C_1$-$C_4$ alkoxy;
$C_1$-$C_4$ mono or dialkylaminocarbonyloxy;

Provided also that when Y is hydroxy, Z cannot be hydrogen;

the N-oxides thereof, provided that $R_3$ cannot be unsaturated alkyl and Y and Z cannot be alkylthioalkyl, alkylaminoalkyl, dialkylaminoalkyl, trialkylammoniumalkyl, amino, 2(or 4)-pyridyloxy, alkoxyamino or unsaturated alkyl;

the optical isomers thereof when $R_1$ and $R_2$ are not the same;
the tautomers and geometric isomers thereof; and
the acid addition salts thereof except when $R_3$ is a salt forming cation.

The present invention also relates to substituted pyrrolopyridine acetonitriles depicted by formula III, substituted pyrrolopyridine acetamides illustrated by formula IV, imidazopyrrolopyridinediones illustrated formulas V and VI, and carbamoyl nicotinic acids, esters and salts depicted by formula VII. These compounds have the structures illustrated below and are useful as intermediates for the preparation of the herbicidal nicotinic acids, esters and salts of formulas I and II.

Formula III, IV, V, VI and VII, compounds have the structures:

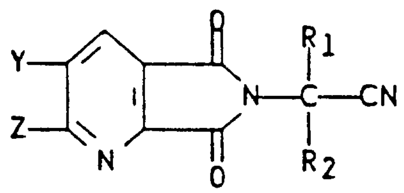

(III)

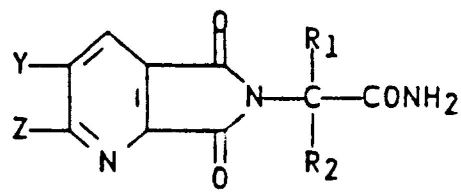

(IV)

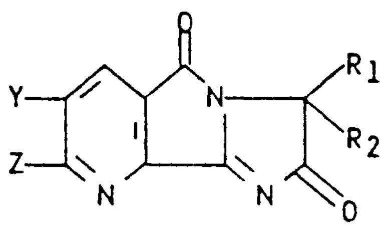

(V)

(VI)

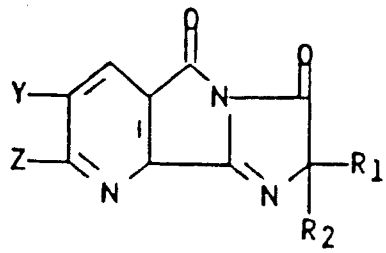

and

(VII)

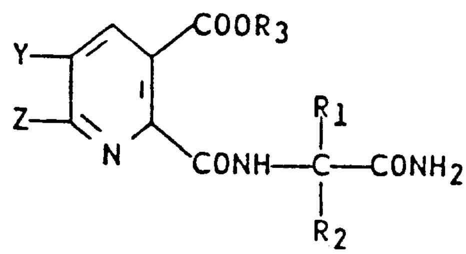

wherein $R_1$, $R_2$, $R_3$, Y and Z are as described above.

DESCRIPTION OF THE PREFERRED EMBODIMENTS

A preferred group of compounds of this invention are the 5(and/or 6)substituted 2-(2-imidazolin-2-yl)-nicotinic acid, esters and salts represented by formula I above, wherein A, B, W, $R_1$ and $R_2$ are as described in said claim 1, and one of Y and Z represents hydrogen or methyl while the other of Y and Z represents

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-furyl optionally substituted with halogen or $C_1$-$C_3$ alkyl;

2(or 3)-thienyl optionally substituted with halogen or $C_1$-$C_3$ alkyl;

N-methyl-2(or 3)-pyrrolyl;

hydroxy;

amino optionally substituted with $C_1$-$C_4$ alkoxy or $C_1$-$C_2$ alkyl and $C_1$-$C_4$ alkoxy;

2(or 4)-pyridyloxy optionally substituted with $C_1$-$C_4$ alkyl, trifluoromethyl or 1 or 2 halogens;

$C_1$-$C_4$ mono or dialkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

the N-oxides thereof, provided that $R_3$ cannot be unsaturated alkyl;

the optical isomers thereof when $R_1$ and $R_2$ are not the same;

the tautomers and geometric isomers thereof; and

the acid addition salts thereof except when $R_3$ is a salt forming cation.

In this application the term cation means all cations, but preferably alkali metals, alkaline earth metals, ammonium, organic ammonium and inorganic metals such as copper, nickel, zinc, silver, lead, manganese, cobalt or iron.

The term organic ammonium means a positively charged nitrogen atom joined to from one to four aliphatic groups, each containing from one to twenty carbon atoms and preferably one to six carbon atoms.

The compounds of the present invention are analogs of 2-(2-imidazolin-2-yl)pyridines of M. Los, described in the EPO application 81103638.3 published December 16, 1981 and EPO patent specification publication number 0,041,623; published July 28, 1985. However, the compounds of the present invention differ from those of M. Los in the 5(and/or 6) substitution of the pyridine ring. These differences may appear to be minimal but, in fact, are significant since they represent novel substitution of the imidazolinones and can provide new avenues to other herbicidally useful products or plant growth regulating agents.

Heretofore, 5(and/or 6) substitution on the pyridine ring was limited by the unavailability of methods for the preparation of substituted intermediates that would yield the 5(and/or 6) alkenyl, alkynyl, cycloalkyl, alkylcarbonyl, alkylcarbonylamino, 2(or 3)-furyl, 2(or 3)-thienyl or 2(or 4) pyridyloxy formula I-VII compounds of the present invention.

It is therefore an object of the present invention to provide 5(and/or 6)substituted 2-(2-imidazolin-2-yl)-nicotinic acids, esters and salts and 5(and/or 6)substituted intermediates therefore.

In the following descriptions for the preparation of the formula I 5(and/or 6) substituted 2-(2-imidazolin-2-yl)nicotinic acids, esters and salts, the imidazolinyl function, has the following structure:

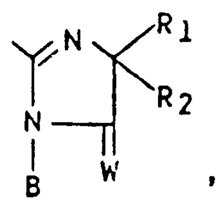

wherein $R_1$, $R_2$, B and W are as described above; however, to avoid the repeated use of this structure in the specification, a specific imidazolinyl function is used as representative of the above structure. The specific imidazolinyl function is (4-isopropyl-4-methyl-5-oxo-2-imidazolin-2-yl), and is used in the specification simply as a convenience in describing the reactions in which the above illustrated function can be involved. In practice, the structure can be substituted for the above-said terminology and the substituents identified as $R_1$, $R_2$, B and W, can be any of those selected from definitions for $R_1$, $R_2$, B and W mentioned above.

In accordance with this invention, 6-(4-isopropyl-4-methyl-5-oxo-2-imidazolin-2-yl)pyridine-3-carboxaldehyde is prepared by oxidation of 2-(5-hydroxymethyl-2-pyridyl)-4-isopropyl-4-methyl-2-imidazolin-5-one with manganese dioxide at an elevated temperature in the presence of an inert organic solvent such as a chlorinated hydrocarbon.

The thus prepared carboxaldehyde can then be converted to the desired 2-(5-alkenyl-2-pyridyl)-4-isopropyl-4-methyl-5-oxo-2-imidazolin-4-one by reaction of said aldehyde with the appropriate alkyl triphenylphosphonium halide in the presence of an alkali metal hydride, mineral oil and dimethylsulfoxide. Lithiation of this 5-alkenyl derivative, using methyl lithium and carbon dioxide in the presence of tetrahydrofuran, affords the corresponding, herbicidally active, 2-(4-isopropyl-4-methyl-5-oxo-2-imidazolin-2-yl)-5-alkenylnicotinic acid.

Esterification of the thus prepared nicotinic acid can then be achieved by reaction of said acid with ethereal diazoalkane, preferably in the presence of an inert organic solvent such as chlorinated hydrocarbon.

Preparation of the 5(and/or 6)substituted nicotinic acids wherein the 5(and/or 6)substitution is alkenyl, alkylcarbonyl, 2(or 3)-furyl or 2(or 3)-thienyl, can be accomplished from the appropriate 5(and/or 6)-substituted-2,3-dicarboxylate.

In this method the appropriately substituted 5(and/or 6)pyridine-2,3-dicarboxylate is converted to the corresponding acid by heating with a strong base, such as an alkali metal hydroxide, and then acidifying the reaction mixture with a strong mineral acid. Heating of the thus formed acid with acetic anhydride, preferably in the presence of pyridine and 1,2-dimethoxyethane, yields the 5(and/or 6)substituted pyridine-2,3-dicarboxylic anhydride. The 5(and/or 6)substituted pyridine-2,3-dicarboxylic anhydride is then reacted with an aminocarboxamide to yield an isomeric mixture of the 5(and/or 6)substituted carbamoylnicotinic acid and the 5(and/or 6)substituted carbamoylpicolinic acid. This reaction is carried out using approximately equimolar amounts of the anhydride and the carboxamide, in the presence of an inert organic solvent such

as a chlorinated hydrocarbon or a low-boiling ether such as THF, diethyl ether, or dimethoxyethane. When the reaction is essentially complete, the product mixture is heated with base to give a mixture of the 2-(4-isopropyl-4-methyl-5-oxo-2-imidazolin-2-yl) 5-(and/or 6) substituted nicotinic acid and the 3-(4-isopropyl-4-methyl-5-oxo-2-imidazolin-2-yl)-5-(and/or 6)substituted picolinic acid. Conversion of this acid mixture to the corresponding esters can then be achieved by reaction of said mixture with acetic anhydride, preferably in the presence of an inert organic solvent such as toluene, xylene, benzene or the like. The reaction provides a mixture of the tricyclic diones of formulas V and VI which can be ring opened with an alkali metal alkoxide to yield a mixture of the 2-(4-isopropyl-4-methyl-5-oxo-2-imidazolin-2-yl) 5-(and/or 6) substituted nicotinate and the 3-(4-isopropyl-4-methyl-5-oxo-2-imidazolin-2-yl) 5-(and/or 6)substituted picolinate.

Conversion of the alkyl 2-(4-isopropyl-4-methyl-5-oxo-2-imidazolin-2-yl) 5-(and/or 6)alkenyl nicotinates or the corresponding 5-(and/or 6)alkylthioalkyl or alkoxyalkylnicotinates to the corresponding acid can be achieved by reaction of the ester with alcoholic alkali metal hydroxide at an elevated temperature. After heating the reaction mixture is cooled and acidified with a strong mineral acid. The reaction is concentrated and then partitioned between a chlorinated hydrocarbon solvent and water.

Preparation of formula I, 5(and/or 6)haloalkyl 2-(2-imidazolin-2-yl)nicotinates can be achieved by reacting an alkyl 2-(4-isopropyl-4-methyl-5-oxo-2-imidazolin-2-yl)-5-alkylnicotinate with an N-halosuccinimide and benzoyl peroxide in the presence of an inert organic solvent such a chlorinated hydrocarbon. This reaction is generally conducted at an elevated temperature, preferably under a blanket of inert gas. The reaction yields the alkyl 2-(4-isopropyl-4-methyl-5-oxo-2-imidazolin-2-yl)-5(and/or 6)-(1-haloalkyl)nicotinate If this reaction is conducted using an imidazolinyl function in which B is $COR_4$ as described above, the $COR_4$ function is retained during the reaction but can be removed from the product ester by reaction thereof with aqueous alcohol and a strong mineral acid. These reactions are exemplified by examples 9 and 10 described in detail below. The thus obtained alkyl 2-(4-isopropyl-4-methyl-5-oxo-2-imidazolin-2-yl)-5-(and/or 6)-(1-haloalkyl)nicotinate can then be used as the starting material for the preparation of the corresponding formula I 5(and/or 6)alkyl substituted with alkylcarbonyloxy 2-(2-imidazolin-2-yl)nicotinate. The reaction involves the treatment of the 5(and/or 6)-1-(haloalkyl)nicotinate referred to above, with an organic acid and an alkali metal salt of said organic acid.

Treatment of the thus prepared formula I 2-(2-imidazolin-2-yl)nicotinate, having a 5(and/or 6)alkyl substituent substituted with an alkylcarbonyloxy function, with an alkali metal alkoxide in a lower aliphatic alcohol provides the corresponding 5(and/or 6)hydroxyloweralkyl function on the pyridine ring of the 2-(2-imidazolin-2 yl)nicotinate.

Treatment of this 5-(and/or 6)hydroxy loweralkyl 2-(2-imidazolin-2-yl)nicotinate with pyridinium dichromate and pyridinium trifluoroacetate in the presence of an inert organic solvent such as a chlorinated hydrocarbon, then yields the corresponding 5(and/or 6)alkylcarbonyl 2-(2-imidazolin-2-yl)nicotinate.

To obtain the formula I, 5(and/or 6)alkylthioalkyl substituted 2-(2-imidazolin-2-yl)nicotinates of this invention, a formula I 5(and/or 6)haloalkyl 2-(2-imidazolin-2-yl)nicotinate is dissolved in absolute methanol, treated with a 50% oil dispersion of alkali metal hydride and then with an alkyl mercaptan. These additions are generally made while maintaining the temperature of the mixture below about -5°C. Thereafter, the mixture is acidified with acetic acid to pH 4 and then concentrated in vacuo. The residue can be dissolved in methylene chloride, washed with aqueous sodium bicarbonate, reconcentrated in vacuo and the product recovered.

Additional methods for the preparation of the compounds of this invention are described in the examples below.

Among the compounds of this invention that can be prepared by the methods described above and/or exemplified below are those having the following structure:

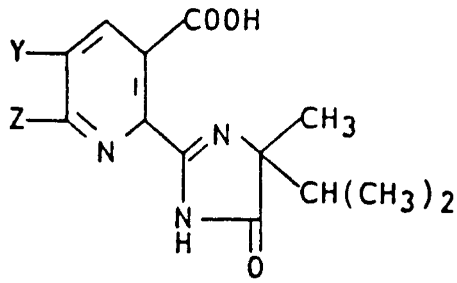

| Y | Z |
|---|---|
| vinyl | hydrogen |
| 1-propenyl | hydrogen |
| allyl | hydrogen |
| allyl | allyl |
| 3-methoxy propenyl | hydrogen |
| 3-chloropropenyl | methoxy |
| 2-trichloromethylvinyl | hydrogen |
| 1-chlorovinyl | dimethylamino |
| 2-chlorovinyl | hydrogen |
| isopropenyl | hydrogen |
| 2-phenylvinyl (cis) | hydrogen |
| 2-phenylvinyl (trans) | hydrogen |
| ethynyl | hydrogen |
| 1-propynyl | hydrogen |
| 2-propynyl | ethoxy |
| 3-chloro-1-propynyl | hydrogen |
| 3-methoxy-1-propynyl | hydrogen |
| 3-phenyl-1-propynyl | hydrogen |
| 2-trichloromethyl-1-ethynyl | cyano |
| 2-chloro-1-ethynyl | hydrogen |
| cyclopropyl | p-tolyl |
| cyclopropyl | hydrogen |

| Y | Z |
|---|---|
| cyclopropyl | methoxy |
| cyclobutyl | hydrogen |
| cyclobutyl | phenyl |
| cyclopentyl | hydrogen |
| cyclohexyl | hydrogen |
| 2,2-dimethyl-1-cyclopropyl | hydrogen |
| 2-methyl-1-cyclohexyl | hydrogen |
| 2-chloro-1-cyclohexyl | hydrogen |
| 2-chloro-1-cyclopropyl | hydrogen |
| 2-methoxy-1-cyclohexyl | hydrogen |
| 2-methoxy-1-cyclopropyl | hydrogen |
| acetyl | hydrogen |
| propionyl | hydrogen |
| butyryl | hydrogen |
| methoxyacetyl | hydrogen |
| butoxy acetyl | hydrogen |
| chloroacetyl | hydrogen |
| dichloroacetyl | hydrogen |
| trichloroacetyl | hydrogen |
| 2-chloropropionyl | hydrogen |
| 3-chloropropionyl | hydrogen |
| acetamido | hydrogen |
| propionamido | hydrogen |
| butyramido | hydrogen |
| chloroacetamido | hydrogen |
| dichloroacetamido | hydrogen |
| trichloroacetamido | hydrogen |
| methoxyacetamido | hydrogen |
| 2-chloropropionamido | hydrogen |
| 3-chloropropionamido | hydrogen |

| Y | Z |
|---|---|
| N-methylacetamido | hydrogen |
| N-ethylacetamido | hydrogen |
| N-methoxyacetamido | hydrogen |
| N-butoxyacetamido | hydrogen |
| N-methylpropionamido | hydrogen |
| N-methoxypropionamido | hydrogen |
| N-methylchloroacetamido | hydrogen |
| N-methoxychloroacetamido | hydrogen |
| N-methyldichloroacetamido | hydrogen |
| N-methoxydichloroacetamido | hydrogen |
| acetoxy | hydrogen |
| acetoxy | acetamido |
| propionyloxy | hydrogen |
| butyryloxy | hydrogen |
| methoxyacetoxy | hydrogen |
| chloroacetoxy | hydrogen |
| dichloroacetoxy | hydrogen |
| trichloroacetoxy | hydrogen |
| 2-chloropropionyloxy | hydrogen |
| 3-chloropropionyloxy | hydrogen |
| 2,2-dichloropropionyloxy | hydrogen |
| methoxymethyl | hydrogen |
| methoxymethyl | methoxymethyl |
| ethoxymethyl | hydrogen |
| n-propoxymethyl | hydrogen |
| i-propoxymethyl | hydrogen |
| n-butoxymethyl | hydrogen |
| i-butoxymethyl | hydrogen |
| sec-butoxymethyl | hydrogen |
| tert-butoxymethyl | hydrogen |

| Y | Z |
|---|---|
| 2-methoxyethyl | 3-thienyl |
| 2-methoxyethyl | hydrogen |
| 2-ethoxyethyl | hydrogen |
| 2-methoxypropyl | hydrogen |
| 3-methoxypropyl | hydrogen |
| 2-methoxy-2-methylethyl | hydrogen |
| methylthiomethyl | hydrogen |
| methylthiomethyl | 2-furyl |
| ethylthiomethyl | hydrogen |
| propylthiomethyl | hydrogen |
| butylthiomethyl | hydrogen |
| 1-methylthioethyl | hydrogen |
| 2-methylthioethyl | hydrogen |
| 1-methylthiopropyl | hydrogen |
| 2-methyl-2-methylthioethyl | hydrogen |
| methylaminomethyl | hydrogen |
| methylaminomethyl | p-chlorophenyl |
| ethylaminomethyl | hydrogen |
| n-propylaminomethyl | hydrogen |
| i-propylaminomethyl | hydrogen |
| n-butylaminomethyl | hydrogen |
| i-butylaminomethyl | hydrogen |
| sec-butylaminomethyl | hydrogen |
| tert-butylaminomethyl | hydrogen |
| 2-methylaminoethyl | hydrogen |
| 2-methylaminopropyl | hydrogen |
| 2-methylaminobutyl | hydrogen |
| 2-methyl-2-methylaminoethyl | hydrogen |
| 3-methylaminopropyl | hydrogen |

| Y | Z |
|---|---|
| dimethylaminomethyl | hydrogen |
| diethylaminomethyl | hydrogen |
| methyl(ethyl)aminomethyl | hydrogen |
| dipropylaminomethyl | hydrogen |
| methyl(propyl)aminomethyl | hydrogen |
| ethyl(propyl)aminomethyl | hydrogen |
| 1-dimethylaminoethyl | hydrogen |
| 2-dimethylaminoethyl | hydrogen |
| 1-diethylaminoethyl | hydrogen |
| 2-diethylaminoethyl | hydrogen |
| 3-dimethylaminopropyl | hydrogen |
| (trimethylammonium)methyl | hydrogen |
| (triethylammonium)methyl | hydrogen |
| 1-(trimethylammonium)ethyl | hydrogen |
| 2-(trimethylammonium)ethyl | hydrogen |
| 3-(trimethylammonium)propyl | hydrogen |
| acetoxymethyl | hydrogen |
| propionyloxymethyl | hydrogen |
| butyroyloxymethyl | hydrogen |
| 1-acetoxyethyl | hydrogen |
| 2-acetoxyethyl | hydrogen |
| 3-acetoxypropyl | hydrogen |
| 2-acetoxy-2-methylethyl | hydrogen |
| 2-furyl | hydrogen |
| 3-furyl | hydrogen |
| 3-chloro-2-furyl | hydrogen |
| 4-chloro-2-furyl | hydrogen |
| 5-chloro-2-furyl | hydrogen |
| 2-chloro-3-furyl | hydrogen |
| 4-chloro-3-furyl | hydrogen |
| 5-chloro-3-furyl | hydrogen |

| Y | Z |
| --- | --- |
| 3-methyl-2-furyl | hydrogen |
| 5-methyl-2-furyl | hydrogen |
| 2-methyl-3-furyl | hydrogen |
| 5-methyl-3-furyl | hydrogen |
| 2-thienyl | hydrogen |
| 3-thienyl | hydrogen |
| 3-chloro-2-thienyl | hydrogen |
| 4-chloro-2-thienyl | hydrogen |
| 5-chloro-2-thienyl | hydrogen |
| 2-chloro-3-thienyl | hydrogen |
| 4-chloro-3-thienyl | hydrogen |
| 5-chloro-3-thienyl | hydrogen |
| 3-methyl-2-thienyl | hydrogen |
| 5-methyl-2-thienyl | hydrogen |
| 2-methyl-3-thienyl | hydrogen |
| 5-methyl-3-thienyl | hydrogen |
| 5-bromo-2-furyl | hydrogen |
| 5-bromo-2-thienyl | hydrogen |
| N-methyl-2-pyrrolyl | hydrogen |
| N-methyl-3-pyrrolyl | hydrogen |
| amino | hydrogen |
| methoxyamino | hydrogen |
| ethoxyamino | hydrogen |
| n-propyloxyamino | hydrogen |
| i-propyloxyamino | hydrogen |
| n-butoxyamino | hydrogen |
| i-butoxyamino | hydrogen |
| sec-butoxyamino | hydrogen |
| tert-butoxyamino | hydrogen |
| methyl(methoxy)amino | hydrogen |
| ethyl(methoxy)amino | hydrogen |
| methyl(ethoxy)amino | hydrogen |
| ethyl(ethoxy)amino | hydrogen |

| Y | Z |
|---|---|
| 2-pyridyloxy | hydrogen |
| 4-pyridyloxy | hydrogen |
| 3-fluoro-2-pyridyloxy | hydrogen |
| 3-chloro-2-pyridyloxy | hydrogen |
| 3-methyl-2-pyridyloxy | hydrogen |
| 3,5-dichloro-2-pyridyloxy | hydrogen |
| 3,5-difluoro-2-pyridyloxy | hydrogen |
| 5-trifluoromethyl-2-pyridyloxy | hydrogen |
| 3-fluoro-5-trifluoromethyl-2-pyridyloxy | hydrogen |
| hydrogen | vinyl |
| hydrogen | 1-propenyl |
| hydrogen | allyl |
| hydrogen | 3-methoxypropenyl |
| hydrogen | 3-chloropropenyl |
| hydrogen | 2-trichloromethyl vinyl |
| hydrogen | 1-chlorovinyl |
| hydrogen | 2-chlorovinyl |
| hydrogen | isopropenyl |
| hydrogen | 2-phenylvinyl(cis) |
| hydrogen | 2-phenylvinyl(trans) |
| hydrogen | ethynyl |
| hydrogen | 1-propynyl |
| hydrogen | 2-propynyl |
| hydrogen | 3-chloro-1-propynyl |
| hydrogen | 3-methoxy-1-propynyl |
| hydrogen | 3-phenyl-1-propynyl |
| hydrogen | 2-trichloromethyl-ethynyl |
| hydrogen | 2-chloro-1-ethynyl |

| Y | Z |
|---|---|
| trifluoromethoxy | 1-chlorovinyl |
| methylthio | 2-chlorovinyl |
| methylsulfonyl | ethynyl |
| 2-chlorovinyl | 2-chlorovinyl |
| hydrogen | cyclopropyl |
| phenyl | cyclopropyl |
| hydrogen | cyclobutyl |
| hydrogen | cyclopentyl |
| hydrogen | cyclohexyl |
| hydrogen | 2,2-dimethyl-1-cyclopropyl |
| hydrogen | 2-methyl-1-cyclohexyl |
| hydrogen | 2-methoxy-1-cyclopropyl |
| hydrogen | acetyl |
| dimethylamino | acetyl |
| hydrogen | propionyl |
| hydrogen | butyryl |
| hydrogen | methoxyacetyl |
| hydrogen | butoxyacetyl |
| 2-thienyl | chloroacetyl |
| hydrogen | chloroacetyl |
| hydrogen | dichloroacetyl |
| hydrogen | trichloroacetyl |
| hydrogen | 2-chloropropionyl |
| hydrogen | 3-chloropropionyl |
| hydrogen | acetamido |
| hydrogen | propionamido |
| hydrogen | butyramido |
| hydrogen | chloroacetamido |
| hydrogen | dichloroacetamido |
| hydrogen | trichloroacetamido |

| Y | Z |
|---|---|
| hydrogen | methoxyacetamido |
| hydrogen | 2-chloropropionamido |
| hydrogen | 3-chloropropionamido |
| hydrogen | N-methylacetamido |
| hydrogen | N-ethylacetamido |
| hydrogen | N-methoxyacetamido |
| hydrogen | N-butoxyacetamido |
| hydrogen | N-methylpropionamido |
| hydrogen | N-methoxypropionamido |
| hydrogen | N-methylchloroacetamido |
| hydrogen | N-methoxychloroacetamido |
| hydrogen | N-methyldichloroacetamido |
| hydrogen | N-methoxydichloro-acetamido |
| hydrogen | acetoxy |
| hydrogen | propionyloxy |
| hydrogen | butyroyloxy |
| hydrogen | methoxyacetoxy |
| hydrogen | chloroacetoxy |
| hydrogen | dichloroacetoxy |
| hydrogen | trichloroacetoxy |
| hydrogen | 2-chloropropionyloxy |
| hydrogen | 3-chloropropionyloxy |
| hydrogen | 2,2-dichloropropionyloxy |
| hydrogen | methoxymethyl |
| hydrogen | ethoxymethyl |
| hydrogen | n-propoxymethyl |
| hydrogen | i-propoxymethyl |
| hydrogen | n-butoxymethyl |
| hydrogen | i-butoxymethyl |
| hydrogen | sec-butoxymethyl |
| hydrogen | tert-butoxymethyl |

| Y | Z |
|---|---|
| hydrogen | 2-methoxyethyl |
| hydrogen | 2-ethoxyethyl |
| hydrogen | 2-methoxypropyl |
| hydrogen | 3-methoxypropyl |
| hydrogen | 2-methoxy-2-methylethyl |
| hydrogen | methylthiomethyl |
| hydrogen | ethylthiomethyl |
| hydrogen | propylthiomethyl |
| hydrogen | butylthiomethyl |
| hydrogen | 1-methylthioethyl |
| hydrogen | 2-methylthioethyl |
| hydrogen | 1-methylthiopropyl |
| hydrogen | 2-methyl-2-methylthio-ethyl |
| hydrogen | methylaminomethyl |
| hydrogen | ethylaminomethyl |
| hydrogen | n-propylaminomethyl |
| hydrogen | i-propylaminomethyl |
| hydrogen | n-butylaminomethyl |
| hydrogen | i-butylaminomethyl |
| hydrogen | sec-butylaminomethyl |
| hydrogen | tert-butylaminomethyl |
| hydrogen | 2-methylaminoethyl |
| hydrogen | 2-methylaminopropyl |
| hydrogen | 2-methylaminobutyl |
| hydrogen | 2-methyl-2-methylamino-ethyl |
| hydrogen | 3-methylaminopropyl |
| hydrogen | dimethylaminomethyl |
| hydrogen | diethylaminomethyl |
| hydrogen | methyl(ethyl)aminomethyl |
| hydrogen | dipropylaminomethyl |

| Y | Z |
|---|---|
| hydrogen | methyl(propyl)aminomethyl |
| hydrogen | ethyl(propyl)aminomethyl |
| hydrogen | 1-dimethylaminoethyl |
| hydrogen | 2-dimethylaminoethyl |
| hydrogen | 1-diethylaminoethyl |
| hydrogen | 2-diethylaminoethyl |
| hydrogen | 3-dimethylaminopropyl |
| hydrogen | (trimethylammonium)methyl |
| hydrogen | (triethylammonium)methyl |
| hydrogen | 1-(trimethylammonium)-ethyl |
| hydrogen | 2-(trimethylammonium)-ethyl |
| hydrogen | 3-(trimethylammonium)-propyl |
| hydrogen | acetoxymethyl |
| hydrogen | propionyloxymethyl |
| hydrogen | butyroyloxymethyl |
| hydrogen | 1-acetoxyethyl |
| hydrogen | 2-acetoxyethyl |
| hydrogen | 3-acetoxypropyl |
| hydrogen | 2-acetoxy-2-methylethyl |
| hydrogen | 2-furyl |
| hydrogen | 3-furyl |
| hydrogen | 3-chloro-2-furyl |
| hydrogen | 4-chloro-2-furyl |
| hydrogen | 5-chloro-2-furyl |
| hydrogen | 2-chloro-3-furyl |
| hydrogen | 4-chloro-3-furyl |
| hydrogen | 5-chloro-3-furyl |
| hydrogen | 3-methyl-2-furyl |
| hydrogen | 5-methyl-2-furyl |

| Y | Z |
|---|---|
| hydrogen | 2-methyl-3-furyl |
| hydrogen | 5-methyl-3-furyl |
| hydrogen | 2-thienyl |
| hydrogen | 3-thienyl |
| hydrogen | 3-chloro-2-thienyl |
| hydrogen | 4-chloro-2-thienyl |
| hydrogen | 5-chloro-2-thienyl |
| hydrogen | 2-chloro-3-thienyl |
| hydrogen | 4-chloro-3-thienyl |
| hydrogen | 5-chloro-3-thienyl |
| hydrogen | 3-methyl-2-thienyl |
| hydrogen | 5-methyl-2-thienyl |
| hydrogen | 2-methyl-3-thienyl |
| hydrogen | 5-methyl-3-thienyl |
| hydrogen | 5-bromo-2-furyl |
| hydrogen | 5-bromo-2-thienyl |
| hydrogen | N-methyl-2-pyrrolyl |
| hydrogen | N-methyl-3-pyrrolyl |
| hydrogen | amino |
| hydrogen | methoxyamino |
| hydrogen | ethoxyamino |
| hydrogen | n-propyloxyamino |
| hydrogen | i-propyloxyamino |
| hydrogen | n-butoxyamino |
| hydrogen | i-butoxyamino |
| hydrogen | sec-butoxyamino |
| hydrogen | tert-butoxyamino |
| hydrogen | methyl(methoxy)amino |
| hydrogen | ethyl(methoxy)amino |
| hydrogen | methyl(ethoxy)amino |
| hydrogen | ethyl(ethoxy)amino |

| Y | Z |
|---|---|
| hydrogen | 2-pyridyloxy |
| hydrogen | 4-pyridyloxy |
| hydrogen | 3-fluoro-2-pyridyloxy |
| hydrogen | 3-chloro-2-pyridyloxy |
| hydrogen | 3-methyl-2-pyridyloxy |
| hydrogen | 3,5-dichloro-2-pyridyloxy |
| hydrogen | 3,5-difluoro-2-pyridyloxy |
| hydrogen | 5-trifluoromethyl-2-pyridyloxy |
| hydrogen | 3-fluoro-5-trifluoromethyl-2-pyridyloxy |
| hydrogen | hydroxy |
| vinyl | methyl |
| 1-propenyl | methyl |
| allyl | methyl |
| 3-methoxypropenyl | methyl |
| 3-chloropropenyl | methyl |
| 2-trichloromethylvinyl | methyl |
| 1-chlorovinyl | methyl |
| 2-chlorovinyl | methyl |
| isopropenyl | methyl |
| 2-phenylvinyl(cis) | methyl |
| 2-phenylvinyl(trans) | methyl |
| ethynyl | methyl |
| 1-propynyl | methyl |
| 2-propynyl | methyl |
| 3-chloro-1-propynyl | methyl |
| 3-methoxy-1-propynyl | methyl |
| 3-phenyl-1-propynyl | methyl |
| 2-trichloromethyl-1-ethynyl | methyl |
| 2-chloro-1-ethynyl | methyl |

| Y | Z |
|---|---|
| cyclopropyl | methyl |
| cyclobutyl | methyl |
| cyclopentyl | methyl |
| cyclohexyl | methyl |
| 2,2-dimethyl-1-cyclopropyl | methyl |
| 2-methyl-1-cyclohexyl | methyl |
| 2-chloro-1-cyclohexyl | methyl |
| 2-chloro-1-cyclopropyl | methyl |
| 2-methoxy-1-cyclohexyl | methyl |
| 2-methoxy-1-cyclopropyl | methyl |
| acetyl | methyl |
| propionyl | methyl |
| butyryl | methyl |
| methoxyacetyl | methyl |
| butoxyacetyl | methyl |
| chloroacetyl | methyl |
| dichloroacetyl | methyl |
| trichloroacetyl | methyl |
| 2-chloropropionyl | methyl |
| 3-chloropropionyl | methyl |
| acetamido | methyl |
| propionamido | methyl |
| butyramido | methyl |
| chloroacetamido | methyl |
| dichloroacetamido | methyl |
| trichloroacetamido | methyl |
| methoxyacetamido | methyl |
| 2-chloropropionamido | methyl |
| 3-chloropropionamido | methyl |
| N-methylacetamido | methyl |
| N-ethylacetamido | methyl |

| Y | Z |
|---|---|
| N-methoxyacetamido | methyl |
| N-butoxyacetamido | methyl |
| N-methylpropionamido | methyl |
| N-methoxypropionamido | methyl |
| N-methylchloroacetamido | methyl |
| N-methoxychloroacetamido | methyl |
| N-methyldichloroacetamido | methyl |
| N-methoxydichloroacetamido | methyl |
| acetoxy | methyl |
| propionyloxy | methyl |
| butyroyloxy | methyl |
| methoxyacetoxy | methyl |
| chloroacetoxy | methyl |
| dichloroacetoxy | methyl |
| trichloroacetoxy | methyl |
| 2-chloropropionyloxy | methyl |
| 3-chloropropionyloxy | methyl |
| 2,2-dichloropropionyloxy | methyl |
| methoxymethyl | methyl |
| ethoxymethyl | methyl |
| n-propoxymethyl | methyl |
| i-propoxymethyl | methyl |
| n-butoxymethyl | methyl |
| i-butoxymethyl | methyl |
| sec-butoxymethyl | methyl |
| tert-butoxymethyl | methyl |
| 2-methoxyethyl | methyl |
| 2-ethoxyethyl | methyl |
| 2-methoxypropyl | methyl |
| 3-methoxypropyl | methyl |
| 2-methoxy-2-methylethyl | methyl |

| Y | Z |
|---|---|
| methylthiomethyl | methyl |
| ethylthiomethyl | methyl |
| propylthiomethyl | methyl |
| butylthiomethyl | methyl |
| 1-methylthioethyl | methyl |
| 2-methylthioethyl | methyl |
| 1-methylthiopropyl | methyl |
| 2-methyl-2-methylthioethyl | methyl |
| methylaminomethyl | methyl |
| ethylaminomethyl | methyl |
| n-propylaminomethyl | methyl |
| i-propylaminomethyl | methyl |
| n-butylaminomethyl | methyl |
| i-butylaminomethyl | methyl |
| sec-butylaminomethyl | methyl |
| tert-butylaminomethyl | methyl |
| 2-methylaminoethyl | methyl |
| 2-methylaminopropyl | methyl |
| 2-methylaminobutyl | methyl |
| 2-methyl-2-methylaminoethyl | methyl |
| 3-methylaminopropyl | methyl |
| dimethylaminomethyl | methyl |
| diethylaminomethyl | methyl |
| methyl(ethyl)aminomethyl | methyl |
| dipropylaminomethyl | methyl |
| methyl(propyl)aminomethyl | methyl |
| ethyl(propyl)aminomethyl | methyl |
| 1-dimethylaminoethyl | methyl |
| 2-dimethylaminoethyl | methyl |
| 1-diethylaminoethyl | methyl |
| 2-diethylaminoethyl | methyl |
| 3-dimethylaminopropyl | methyl |

| Y | Z |
|---|---|
| (trimethylammonium)methyl | methyl |
| (triethylammonium)methyl | methyl |
| 1-(trimethylammonium)ethyl | methyl |
| 2-(trimethylammonium)ethyl | methyl |
| 3-(trimethylammonium)propyl | methyl |
| acetoxymethyl | methyl |
| propionyloxymethyl | methyl |
| butyroyloxymethyl | methyl |
| 1-acetoxyethyl | methyl |
| 2-acetoxyethyl | methyl |
| 3-acetoxypropyl | methyl |
| 2-acetoxy-2-methylethyl | methyl |
| 2-furyl | methyl |
| 3-furyl | methyl |
| 3-chloro-2-furyl | methyl |
| 4-chloro-2-furyl | methyl |
| 5-chloro-2-furyl | methyl |
| 2-chloro-3-furyl | methyl |
| 4-chloro-3-furyl | methyl |
| 5-chloro-3-furyl | methyl |
| 3-methyl-2-furyl | methyl |
| 5-methyl-2-furyl | methyl |
| 2-methyl-3-furyl | methyl |
| 5-methyl-3-furyl | methyl |
| 2-thienyl | methyl |
| 3-thienyl | methyl |
| 3-chloro-2-thienyl | methyl |
| 4-chloro-2-thienyl | methyl |
| 5-chloro-2-thienyl | methyl |
| 2-chloro-3-thienyl | methyl |
| 4-chloro-3-thienyl | methyl |
| 5-chloro-3-thienyl | methyl |
| 3-methyl-2-thienyl | methyl |

| Y | Z |
|---|---|
| 5-methyl-2-thienyl | methyl |
| 2-methyl-3-thienyl | methyl |
| 5-methyl-3-thienyl | methyl |
| 5-bromo-2-furyl | methyl |
| 5-bromo-2-thienyl | methyl |
| N-methyl-2-pyrrolyl | methyl |
| N-methyl-3-pyrrolyl | methyl |
| amino | methyl |
| methoxyamino | methyl |
| ethoxyamino | methyl |
| n-propyloxyamino | methyl |
| i-propyloxyamino | methyl |
| n-butoxyamino | methyl |
| i-butoxyamino | methyl |
| sec-butoxyamino | methyl |
| tert-butoxyamino | methyl |
| methyl(methoxy)amino | methyl |
| ethyl(methoxy)amino | methyl |
| methyl(ethoxy)amino | methyl |
| ethyl(ethoxy)amino | methyl |
| 2-pyridyloxy | methyl |
| 4-pyridyloxy | methyl |
| 3-fluoro-2-pyridyloxy | methyl |
| 3-chloro-2-pyridyloxy | methyl |
| 3-methyl-2-pyridyloxy | methyl |
| 3,5-dichloro-2-pyridyloxy | methyl |
| 3,5-difluoro-2-pyridyloxy | methyl |
| 5-trifluoromethyl-2-pyridyloxy | methyl |
| 3-fluoro-5-trifluoromethyl-2-pyridyloxy | methyl |
| hydroxy | methyl |

| Y | Z |
|---|---|
| methyl | vinyl |
| methyl | 1-propenyl |
| methyl | allyl |
| methyl | 1-chlorovinyl |
| methyl | 2-chlorovinyl |
| methyl | ethynyl |
| methyl | acetyl |
| methyl | chloroacetyl |
| methyl | acetamido |
| methyl | chloroacetamido |
| methyl | N-methylacetamido |
| methyl | acetoxy |
| methyl | chloroacetoxy |
| methyl | methoxymethyl |
| methyl | methylthiomethyl |
| methyl | methylaminomethyl |
| methyl | dimethylaminomethyl |
| methyl | acetoxymethyl |
| methyl | 2-furyl |
| methyl | 2-thienyl |
| methyl | amino |
| methyl | hydroxy |
| methyl | methoxyamino |
| methyl | 3-fluoro-5-trifluoro-methyl-2-pyridyloxy |
| chloro | vinyl |
| chloro | acetyl |
| chloro | ethynyl |
| chloro | acetamido |
| chloro | acetoxy |
| chloro | methoxymethyl |
| chloro | acetoxymethyl |

| Y | Z |
|---|---|
| chloro | 2-furyl |
| chloro | 2-thienyl |
| chloro | amino |
| chloro | hydroxy |
| chloro | methoxyamino |
| vinyl | chloro |
| 1-propenyl | chloro |
| isopropenyl | chloro |
| allyl | chloro |
| 1-chlorovinyl | chloro |
| 2-chlorovinyl | chloro |
| ethynyl | chloro |
| acetyl | chloro |
| chloroacetyl | chloro |
| acetamido | chloro |
| chloroacetamido | chloro |
| N-methylacetamido | chloro |
| acetoxy | chloro |
| chloroacetoxy | chloro |
| methoxymethyl | chloro |
| methylthiomethyl | chloro |
| methylaminomethyl | chloro |
| dimethylaminomethyl | chloro |
| acetoxymethyl | chloro |
| 2-furyl | chloro |
| 2-thienyl | chloro |
| 3-furyl | chloro |
| 3-thienyl | chloro |
| amino | chloro |
| hydroxy | chloro |
| methoxyamino | chloro |
| 3-fluoro-5-trifluoromethyl-2-pyridyloxy | chloro |

| Y | Z |
|---|---|
| vinyl | fluoro |
| 1-propenyl | fluoro |
| isopropenyl | fluoro |
| allyl | fluoro |
| 1-chlorovinyl | fluoro |
| 2-chlorovinyl | fluoro |
| ethynyl | fluoro |
| acetyl | fluoro |
| chloroacetyl | fluoro |
| acetamido | fluoro |
| chloroacetamido | fluoro |
| N-methylacetamido | fluoro |
| acetoxy | fluoro |
| chloroacetoxy | fluoro |
| methoxymethyl | fluoro |
| methylthiomethyl | fluoro |
| methylaminomethyl | fluoro |
| dimethylaminomethyl | fluoro |
| acetoxymethyl | fluoro |
| 2-furyl | fluoro |
| 2-thienyl | fluoro |
| 3-furyl | fluoro |
| 3-thienyl | fluoro |
| amino | fluoro |
| hydroxy | fluoro |
| methoxyamino | fluoro |
| 3-fluoro-5-trifluoromethyl-2-pyridyloxy | fluoro |

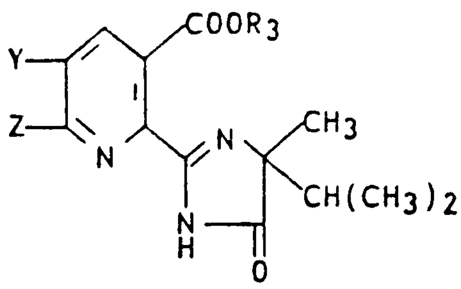

| Y | Z | $R_3$ |
|---|---|---|
| vinyl | hydrogen | methyl |
| ethynyl | hydrogen | ethyl |
| 2-furyl | hydrogen | 2-methoxyethyl |
| methylthiomethyl | hydrogen | furfuryl |
| acetoxymethyl | hydrogen | propargyl |
| hydrogen | methoxymethyl | methyl |
| hydrogen | N-methylacetamido | 2-methoxyethyl |
| hydrogen | chloroacetoxy | allyl |

The formula I compounds of this invention are effective herbicidal agents useful for the control of a Variety of monocotyledonous and dicotyledonous plants. These compounds are herbicidally effective for controlling weeds indigenous to both dry land and wet land areas. They are also useful as aquatic herbicides and are effective for controlling the above-said plants when applied to the foliage thereof or to the soil or water containing seeds or other propagating organs of said plants such as tubers, rhizomes or stolons, at rates of from about 0.016 to 8.0 kg/ha.

The formula I and formula II compounds of this invention can be formulated as wettable powders, flowable concentrates or granular formulations.

Wettable powders can be prepared by grinding together about 20% to 45% by weight of a finely divided carrier such as kaolin, bentonite, diatomaceous earth or attapulgite 45% to 80% by weight of the active compound, 2% to 5% by weight of a dispersing agent such as sodium lignosulfonate, and 2% to 5% by weight of a nonionic surfactant, such as octylphenoxy polyethoxy ethanol or nonylphenoxy polyethoxy ethanol.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

A typical emulsifiable concentrate can be prepared by dissolving about 5% to 25% by weight of the active ingredient in about 65% to 90% by weight of N-methylpyrrolidone, isophorone, butyl cellosolve or methylacetate and dispersing therein about 5% to 10% by weight of a nonionic surfactant such as an alkylphenoxy polyethoxy alcohol. This concentrate is dispersed in water for application as a liquid spray.

When the compounds of the invention are to be used as herbicides where soil treatments are involved, the compounds may be prepared and applied as granular products. Preparation of the granular product can be achieved by dissolving the active compound in a solvent such as methylene chloride or N-methylpyrrolidone and spraying the thus prepared solution on a granular carrier such as corncob grits, sand, attapulgite, kaolin or the like.

The granular product thus prepared generally comprises about 3% to 20% by weight of the active ingredient and about 97% to 80% by weight of the granular carrier.

In order to facilitate a further understanding of the invention, the following examples are presented primarily for the purpose of illustrating certain more specific details thereof.

EXAMPLE 1

Preparation of 6-(4-isopropyl-4-methyl-5-oxo-2-imidazolin-2-yl)pyridine-3-carboxaldehyde

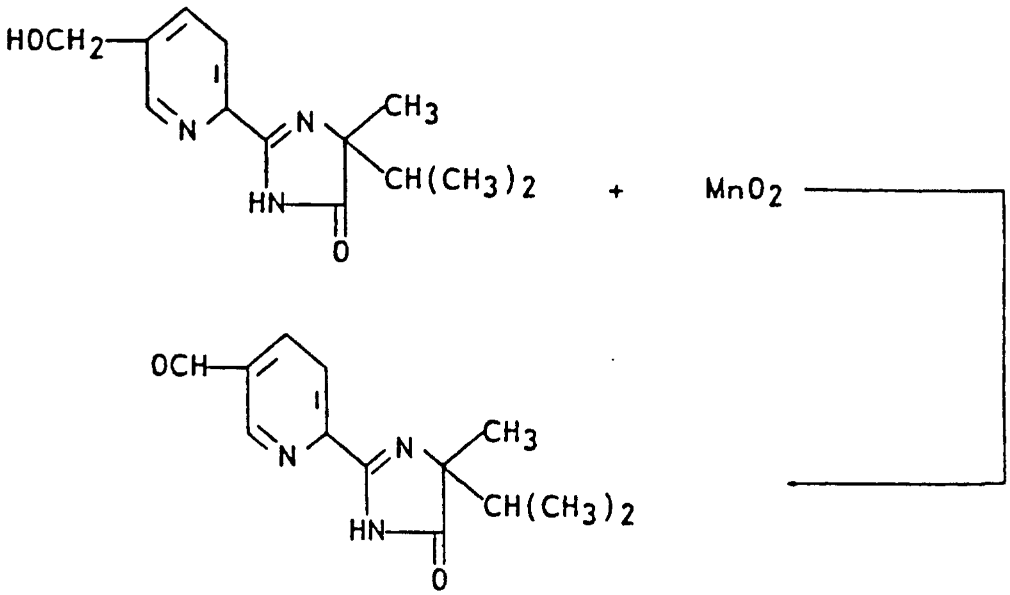

To a stirred solution containing 18 g hydroxymethyl pyridine in 180 mL dry methylene chloride under nitrogen is added 44.3 g of activated manganese dioxide. The mixture is heated under reflux for four hours. The mixture is filtered and the filtrate concentrated. Trituration of the residue with ether-hexane gave a crystalline solid. This is recrystallized from ether-hexane to give analytically pure 6-(4-isopropyl-4-methyl-5-oxo-2-imidazolin-2-yl)pyridine-3-carboxaldehyde, mp 105.5-108°C.

EXAMPLE 2

Preparation of 2-(5-Styryl-2-pyridyl)-4-isopropyl-4-methyl-2-imidazolin-5-one

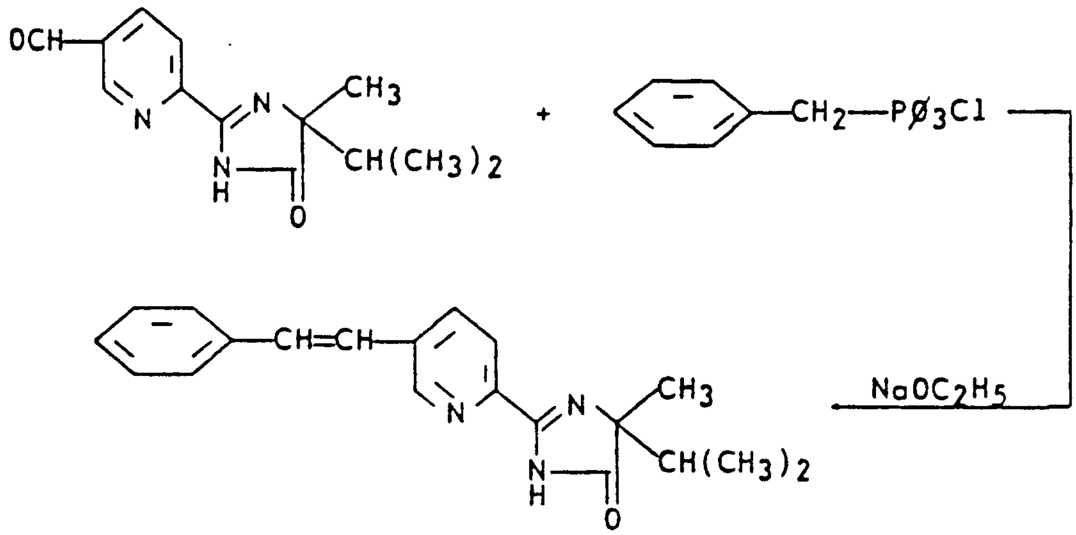


To a solution of 2.72g of sodium ethoxide (.04 mol) in 100 mL absolute ethanol, stirred at room temperature under nitrogen, is added 7.9g (.02 mol) (benzyl) triphenylphosphonium chloride. After 5 minutes, 5.0g 6-(4-isopropyl-4-methyl-5-oxo-2-imidazolin-2-yl)pyridine-3-carboxaldehyde (.02 mol) is added all at once, and the mixture is stirred at room temperature for 24 hours. The mixture is concentrated in vacuo, and the residue is chromatographed on silica gel using ether-hexane as eluant to give 0.4g of the desired product as the pure cis isomer (oil), 4.5g product as an isomeric mixture, and 1.25g of the trans isomer, mp 128-133°C.

EXAMPLE 3

Preparation of 2-(4-isopropyl-4-methyl-5-oxo-2-imidazolin-2-yl)-5-styrylnicotinic acid, (Z)

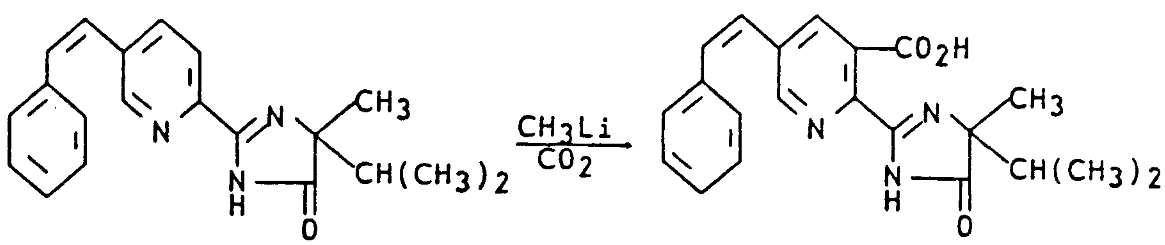


To a solution of 2.07g of 2-(5-styryl-2-pyridyl)-5-isopropyl-5-methyl-2-imidazolin-4-one in 100 mL dry tetrahydrofuran, stirred at -70° under nitrogen, is added slowly 9 mL of 1.6M methyllithium. After stirring the reaction mixture at -10° for 1 hour, it is slowly syringed into a saturated solution of carbon dioxide in tetrahydrofuran. After stirring the resulting mixture overnight, the solvent is removed in vacuo; the residue is dissolved in water, washed with methylene chloride, and the aqueous phase is acidified to pH 3 and extracted with methylene chloride. The combined extracts are dried and concentrated in vacuo to afford the product. Recrystallization from methylene chloride-hexanes affords a solid, mp 122.5-126°.

Utilizing the above procedure and substituting the appropriate 2-pyridyl-5-isopropy-5-methyl-2-imidazoline-4-one yields the compounds below:

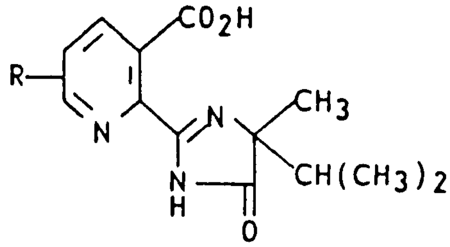

| R | mp· |
|---|---|
| 5-styryl(E) | 169-171° |
| 5- 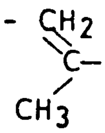 | 166.5°-168° |

EXAMPLE 4

Preparation of methyl-5-isopropenyl-2-(4-isopropyl-4-methyl-5-oxo-2-imidazolin-2-yl)nicotinate

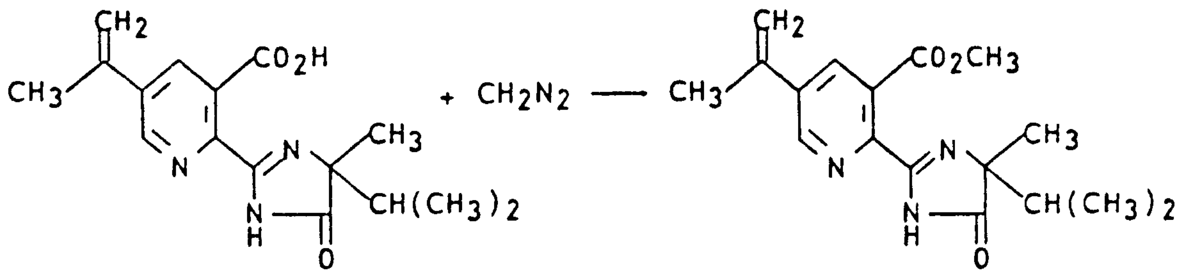


To a solution of 4.9g of 5-isopropenyl-2-(5-isopropyl-5-methyl-4-oxo-2-imidazolin-2-yl)nicotinic acid in 50 mL of methylene chloride, stirred at 0°, is added 60 mL of .25M ethereal diazomethane. After stirring for 15 minutes, the reaction is concentrated on a steam bath. The residue is chromatographed on silica gel using 2:1 ether-hexanes as eluant to afford the desired product. Recrystallization from ether-hexanes affords a solid, mp 90.5-92.5°.

EXAMPLE 5

Preparation of 5-allylpyridine-2,3-dicarboxylic acid

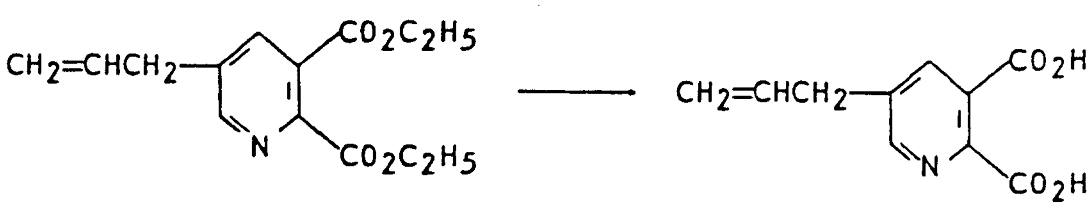

To a solution of 6.07g of diethyl 5-allylpyridine-2,3-dicarboxylate in 20 mL methanol, stirred under nitrogen, is added slowly a solution of 3.3g of sodium hydroxide in 13 mL water. After diluting the reaction mixture with 10 mL each of water and methanol, it is heated at reflux for 1 3/4 hours. The reaction is cooled in an ice bath, acidified to pH 3 with concentrated hydrochloric acid, and concentrated in vacuo to a damp solid. This solid is reevaporated from pyridine to afford the product as a brown solid, mp >250°(dec).

The following were prepared in similar fashion:

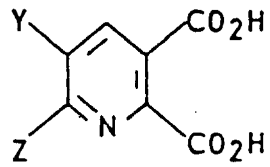

| Y | Z | mp |
|---|---|---|
| 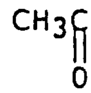 | $CH_3$ | solid |
| H | 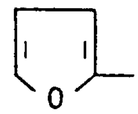 | solid |
| 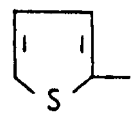 | H | solid |
| H | 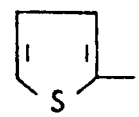 | solid |
| 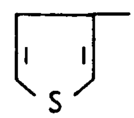 | H | solid |

## EXAMPLE 6

Preparation of 5-allylpyridine-2,3-dicarboxylic anhydride

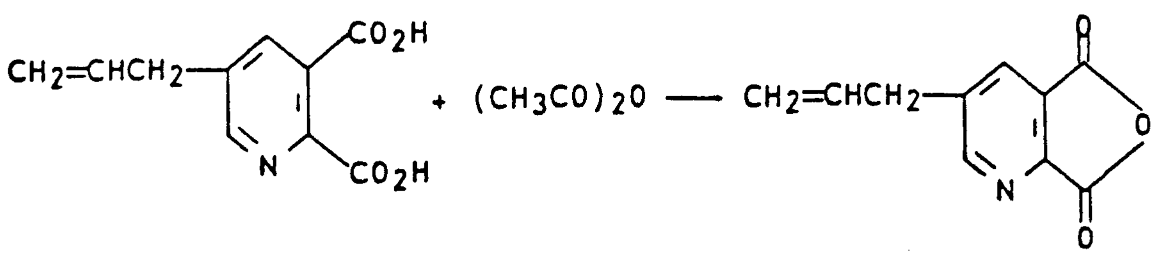


A mixture of 7 g of crude 5-allylpyridine-2,3-dicarboxylic acid, 9.8 mL acetic anhydride, and 3.3 mL pyridine in 100 mL dry 1,2-dimethoxyethane is stirred under nitrogen and heated at 65° for 1 hour. After adding an additional 1 mL of acetic anhydride, the reaction is heated for 2 hours at 65°, then at reflux overnight. The reaction mixture is filtered, and the solids are washed with ether and ethyl acetate. The combined filtrates are concentrated in vacuo and reevaporated from xylenes to afford the product as a solid.

Similarly were prepared the following:

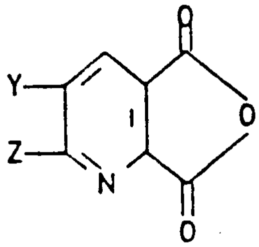

| Y | Z | mp |
|---|---|---|
| 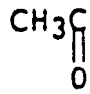 | $CH_3$ | solid |
| H | 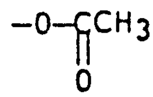 | solid |
| H | 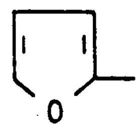 | solid |

| Y | Z | mp |
|---|---|---|
| 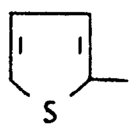 | H | solid |
| H | 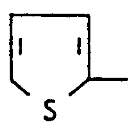 | solid |
| 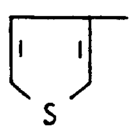 | H | solid |

EXAMPLE 7

Preparation of 2-(4-isopropyl-4-methyl-5-oxo-2-imidazolin-2-yl)-5-propenylnicotinic acid and its picolinic acid isomer

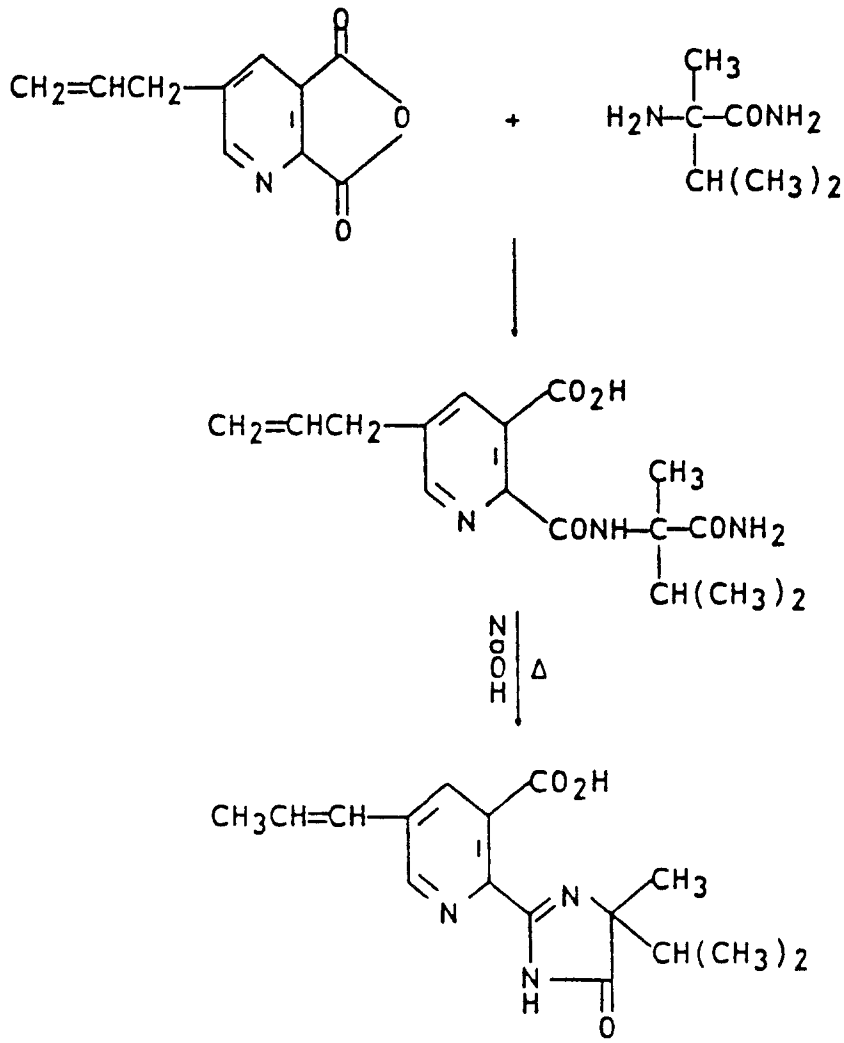


A mixture of 2.88g of 5-allylpyridine-2,3-dicarboxylic anhydride and 2.0g of 2-amino-2,3-dimethylbutyramide in 40 mL of dry tetrahydrofuran is stirred under nitrogen and heated at reflux for 2 hours. The reaction is cooled and concentrated in vacuo to afford a mixture if acid-diamides as a gum. The gum can be redigested in hot tetrahydrofuran, heated at reflux overnight, cooled, diluted with some ether, and filtered to afford the isomerically pure intermediate, 5-allyl-2-[(1-carbamoyl-1,2-dimethylpropyl)carbamoyl]nicotinic acid as a solid.

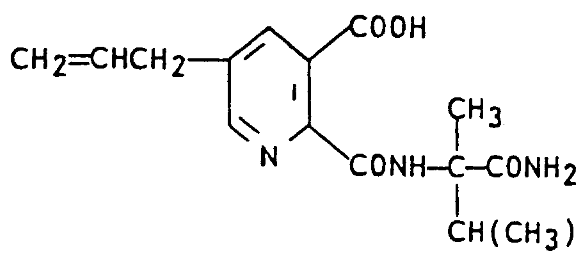

Using essentially the same procedure, but substituting 2-amino-2,3-dimethylthiobutyramide for 2-amino-2,3-dimethylbutyramide affords the corresponding 2-(4-isopropyl-4-methyl-5-thioxo-2-imidazolin-2-yl)-5-propenylnicotinic acid as a solid. This material is digested in 15.2 mL of 5N sodium hydroxide and heated at 80° for 1 hour. The reaction is cooled, acidified to pH 3 with 4N hydrochloric acid and extracted with methylene chloride and ethyl acetate. The combined extracts are dried and concentrated in vacuo to afford 2.68g crude product as a gummy isomeric mixture.

As above, followed by fractional crystallization, affords the following:

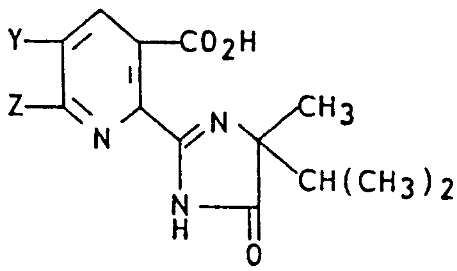

| Y | Z | mp |
|---|---|---|
| 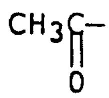 | $CH_3$ | 158-160° |

| Y | Z | mp |
|---|---|---|
| H | 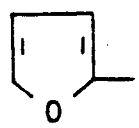 | 253-261°(dec) |
| 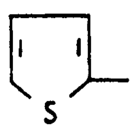 | H | 211.5-213° |
| H | 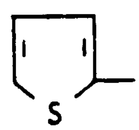 | 255-261°(dec) |
| 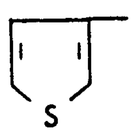 | H | 172-174° |

EXAMPLE 8

Preparation of methyl 2-(4-isopropyl-4-methyl-5-oxo-2-imidazolin-2-yl)-5-propenylnicotinate and its picolinate isomer

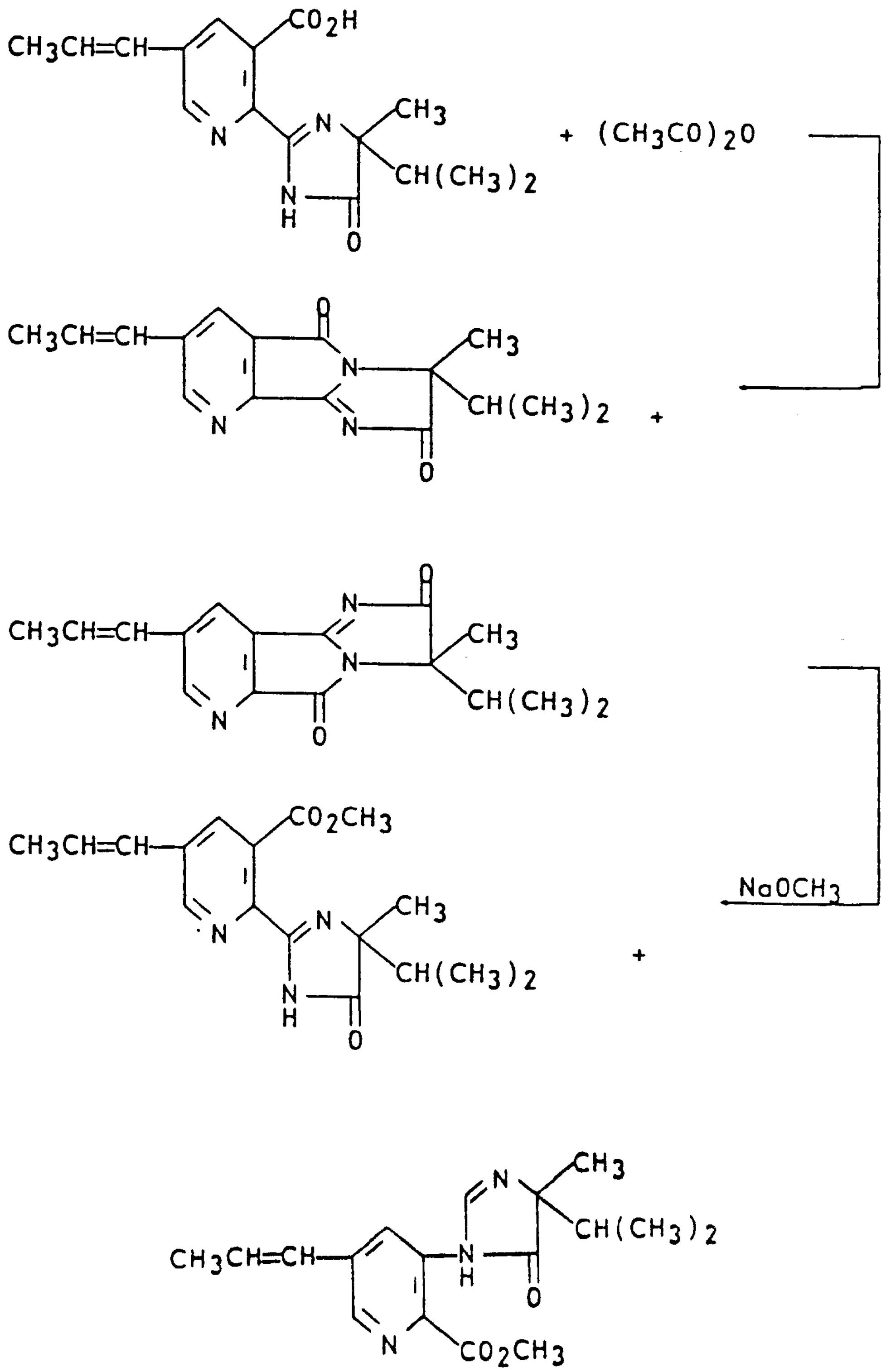


A mixture of 2.68g of 2-(4-isopropyl-4-methyl-5-oxo-2-imidazolin-2-yl)-5-propenylnicotinic acid and its picolinic acid isomer and 1.85 mL of acetic anhydride in 20 mL toluene is stirred under nitrogen and heated

at reflux for 2 1/2 hours. Solvent is removed in vacuo, and the residual gum is reevaporated from xylenes. The residue is dissolved in 20 mL methanol, .75g of sodium methoxide is added, and the mixture is stirred at room temperature for 3 hours. The reaction is then acidified with glacial acetic acid to pH 5, stirred overnight, and concentrated in vacuo. The residue is partitioned between methylene chloride and water, and the aqueous phase is further extracted with methylene chloride. The combined extracts are dried and concentrated in vacuo to an oil. Chromatography on silica gel using ether-hexane mixtures affords 1.85g of the nicotinate, and .56g of the picolinate. Using the same procedure on isomerically pure 2-(4-isopropyl-4-methyl-5-oxo-2-imidazolin-2-yl)-5-propenylnicotinic acid affords the isomerically pure intermediate, 3-isopropyl-3-methyl-7-propenyl-5H-imidazo[1:2:1,2]pyrrdo[3,4-b]pyridine-2(3H),5-dione, as a solid.

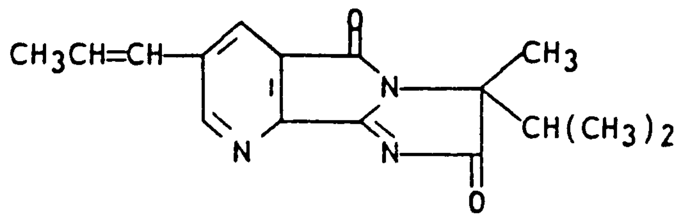

Recrystallization of the nicotinate from methylene chloride-hexanes gives 1.37g of a solid, mp 122.5-124.5.

By this method were also prepared:

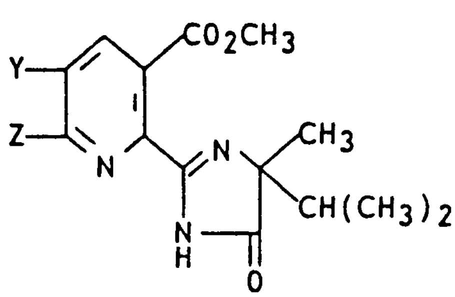

| Y | Z | mp |
| --- | --- | --- |
| H | 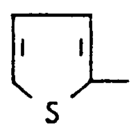 | 138-140.5° |
| H | 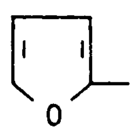 | 168-173.5° |

EXAMPLE 9

Preparation of 2-(4-isopropyl-4-methyl-5-oxo-2-imidazolin-2-yl)-5-propenylnicotinic acid

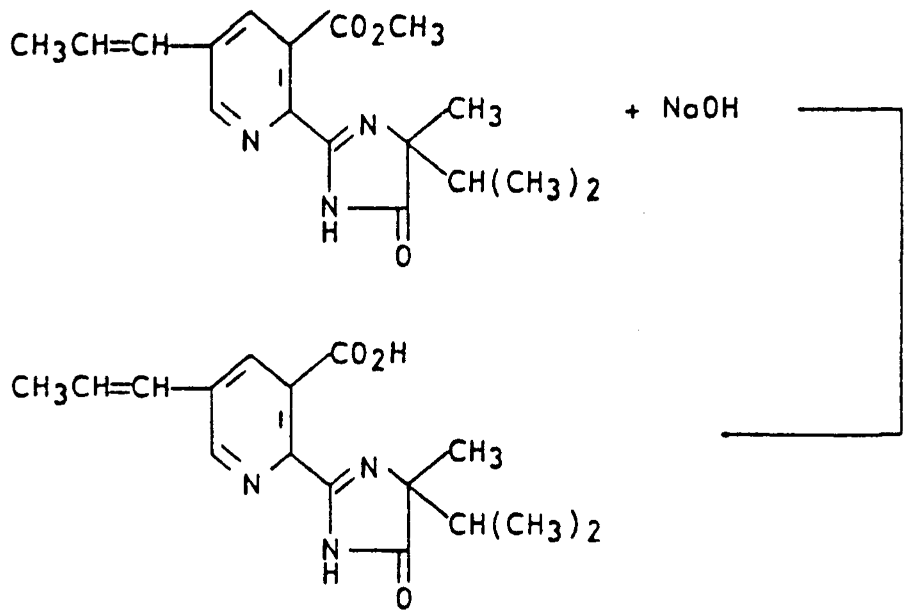


A mixture of 1.5g methyl 2-(4-isopropyl-4-methyl-5-oxo-2-imidazolin-2-yl)-5-propenylnicotinate and 3.2 mL of 2N sodium hydroxide in 15 mL methanol is stirred under nitrogen and heated at 45° for 3 1/2 hours. The reaction is cooled to 10°, acidified to pH 3 with 2N hydrochloric acid, and concentrated in vacuo. The residue is partitioned between methylene chloride and water, and the aqueous phase is further extracted with methylene chloride. The combined organic phases are dried and concentrated in vacuo to a solid. Recrystallization from methylene chloride-hexanes gives a solid, mp 175-176°.

Using essentially the same conditions, and substituting the appropriate nicotinate precursor, the following are obtained:

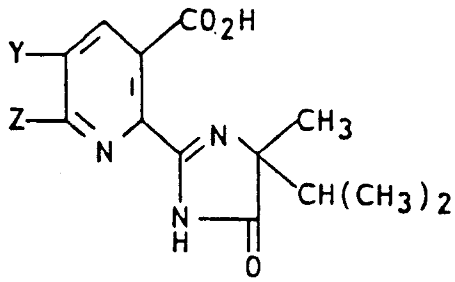

| Y | Z | mp |
|---|---|---|
| 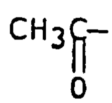 | H | 191.5-192.5° |
| $CH_3SCH_2$- | H | 162-165° |
| H | $CH_3SCH_2$- | 113-116° |
| $CH_3OCH_2$ | H | 160-162° |

EXAMPLE 10

Preparation of methyl 2-(1-benzoyl-4-isopropyl-4-methyl-5-oxo-2-imidazolin-2-yl)-5-(1-bromoethyl)nicotinate

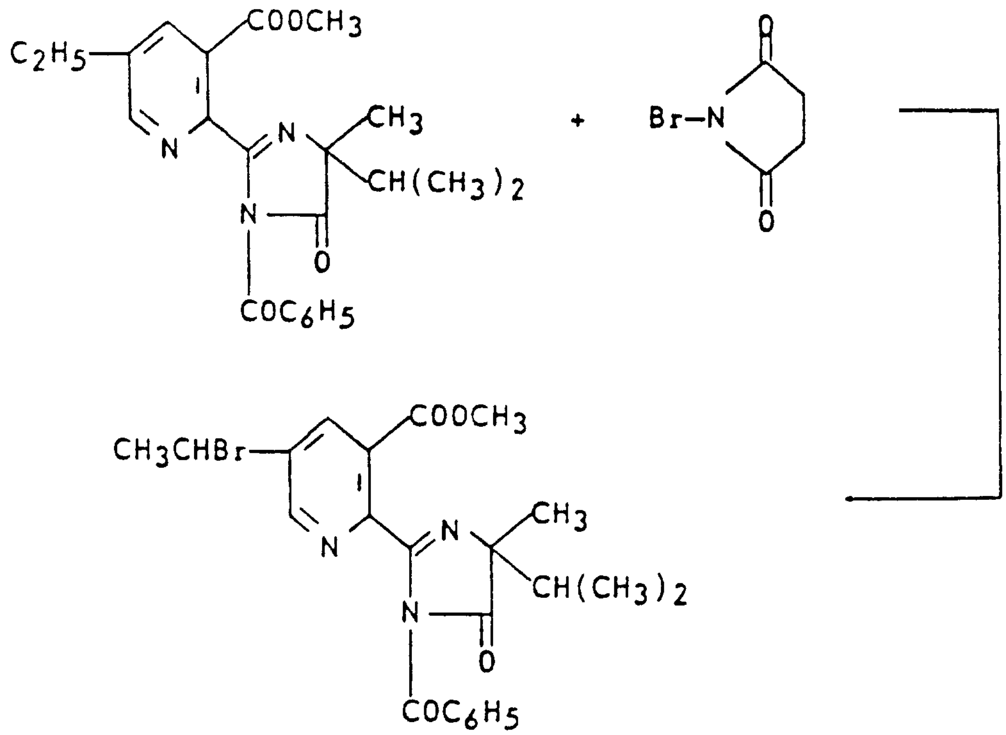

A mixture of 38.6g methyl 2-(1-benzoyl-4-isopropyl-4-methyl-5-oxo-2-imidazolin-2-yl)-5-ethylnicotinate, 16.9g N-bromosuccinimide and 2.3g benzoyl peroxide in 740 mL carbon tetrachloride is stirred under nitrogen and heated at reflux using a sunlamp for 1 1/2 hours. The reaction mixture is cooled, filtered through celite, and the filtrate is concentrated in vacuo to an orange oil. This oil is partitioned between 400 mL each of water and methylene chloride. The aqueous layer is further extracted with methylene chloride, and the combined organic phases are dried over sodium sulfate and concentrated in vacuo to give 49.8g of a cloudy orange glass; NMR shows the desired product to be the major component.

Using essentially the same conditions described above on the 5-methylnicotinate gives the corresponding 5-bromomethyl compound.

EXAMPLE 11

Preparation of methyl 2-(4-isopropyl-4-methyl-5-oxo-2-imidazolin-2-yl)-5-(1-bromoethyl)nicotinate

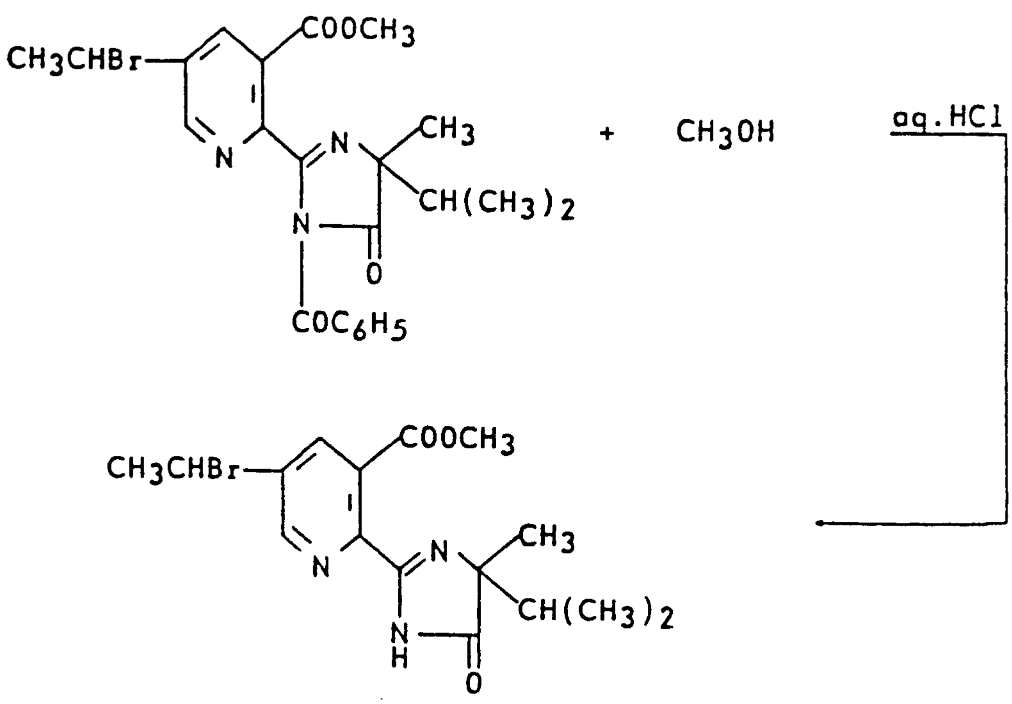


A solution of 49.6g of crude methyl 2-(1-benzoyl-4-isopropyl-4-methyl-5-oxo-2-imidazolin-2-yl)-5-(1-bromoethyl) nicotinate (See Exp. 10 ) in 300 mL of methanol is stirred at room temperature and 400 mL of 50% aqueous methanol (2N in hydrogen chloride) is added dropwise over 1 hour. After stirring the reaction for an additional 2 hours, it is concentrated in vacuo. The pH of the residue is adjusted to 6 with 2N aqueous sodium hydroxide, and the mixture is extracted with 4 x 200 mL methylene chloride. The combined extracts are dried and concentrated in vacuo to afford the crude desired product as a thick oil. A portion is chromatographed on silica gel using ether-hexane as eluant and recrystallized from methylene chloride-hexane to give the desired product as a light orange solid, mp 123-126°C.

EXAMPLE 12

Preparation of methyl 5-(1-acetoxyethyl)-2-(4-isopropyl-4-methyl-5-oxo-2-imidazolin-2-yl)nicotinate

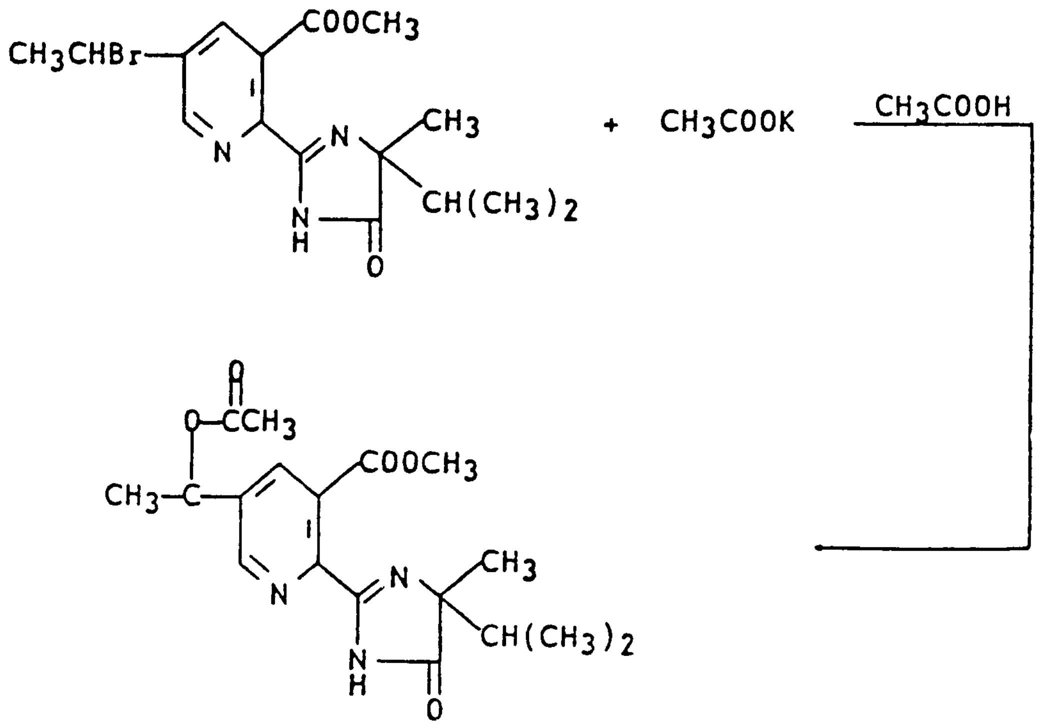


A mixture of 12g of methyl 2-(4-isopropyl-4-methyl-5-oxo-2-imidazolin-2-yl)-5-(1-bromoethyl)nicotinate, 3.1g potassium acetate and 30 mL acetic acid is stirred under nitrogen and heated at reflux for 8 hours. The reaction mixture is filtered, and the filtrate is concentrated in vacuo. The residue is triturated with ether, filtered, and concentrated in vacuo to give the desired product as an amber glass.

Using essentially the same conditions as described above, and using the appropriate 5-(1-bromoalkyl)-2-(4-isopropyl-4-methyl-5-oxo-2-imidazolin-2-yl)nicotinate and appropriate nucleophile, the following are prepared: the 5-methylthiomethyl and the 5-methoxymethyl nicotinates.

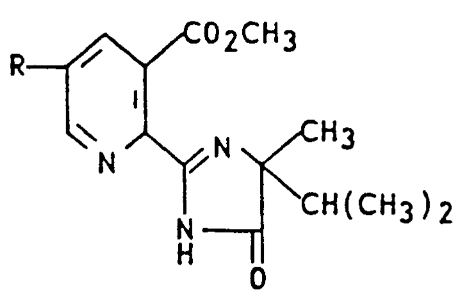

| R | mp |
|---|---|
| $CH_3SCH_2$ | oil |
| $CH_3OCH_2$ | oil |

EXAMPLE 13

Preparation of methyl 5-(1-hydroxyethyl)-2-(4-isopropyl-4-methyl-5-oxo-2-imidazolin-2-yl)nicotinate

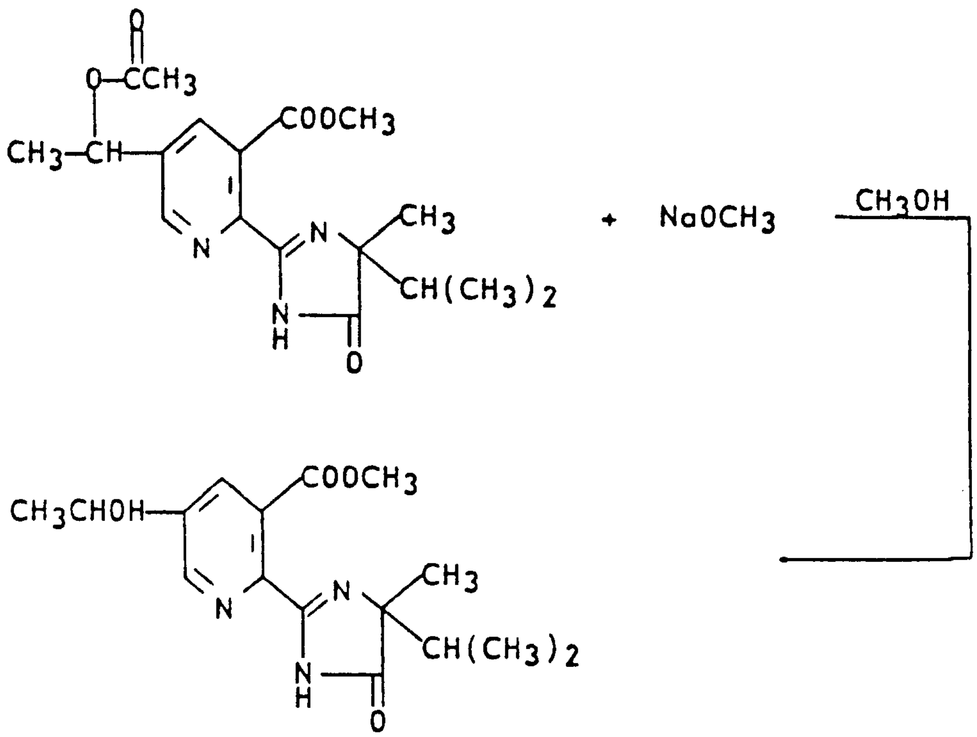


A solution of 16g of methyl 5-(1-acetoxyethyl)-2-(4-isopropyl-4-methyl-5-oxo-2-imidazolin-2-yl)nicotinate and 0.5g sodium methoxide in 150 mL methanol is stirred at room temperature under nitrogen for 5 days. The reaction is acidified to pH 5 with acetic acid and concentrated in vacuo. The residue is partitioned between methylene chloride and water, the aqueous layer is further extracted with methylene chloride, and the combined organic phases are dried over sodium sulfate and concentrated in vacuo to an orange oil. Column chromatography of this oil using ether-hexane as eluent affords the desired product as a white foam.

EXAMPLE 14

Preparation of methyl 5-acetyl-2-(4-isopropyl-4-methyl]-5-oxo-2-imidazolin-2-yl)nicotinate

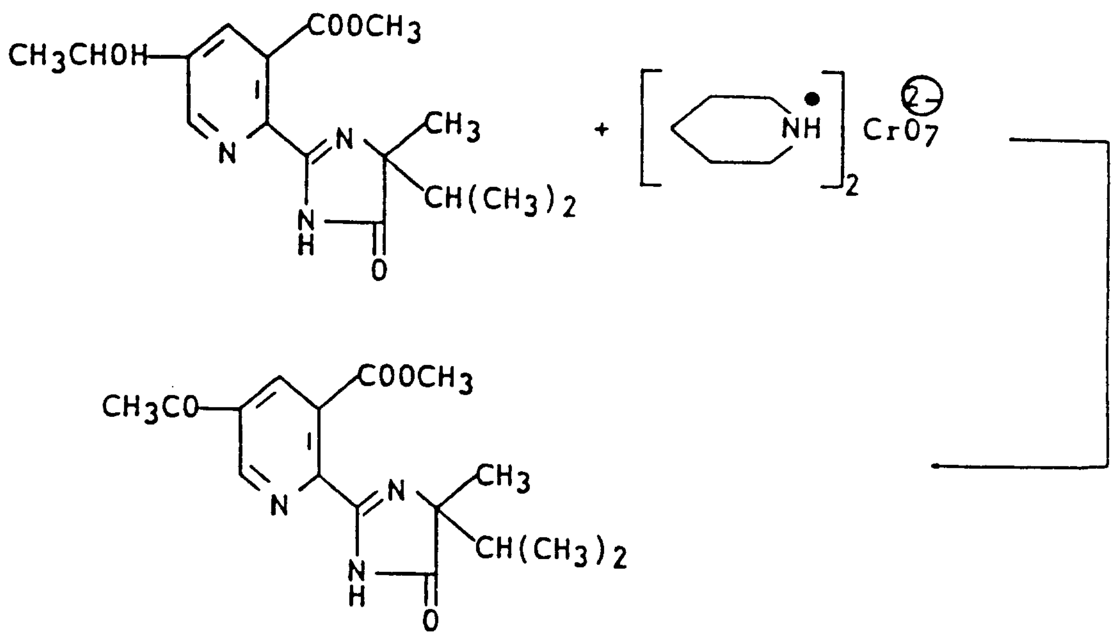

A mixture of 8.2g of methyl 5-(1-hydroxyethyl)-2-(4-isopropyl-4-methyl-5-oxo-2-imidazolin-2-yl)-nicotinate. 14.5g of pyridinum dichromate and 1.99g of pyridinium trifluoroacetate in 30 mL of methylene chloride is stirred at room temperature under nitrogen for 3 days. The reaction mixture is flash-chromatographed through florisil using methylene chloride as eluent, and the combined eluates are washed with water, dried, and concentrated in vacuo to a dark oil. Chromatography of this oil on silica gel using ether-hexane as eluant affords the desired product as a solid. Recrystallization of this solid from methylene chloride-hexane affords a white solid, mp 105-108°C.

EXAMPLE 15

Preparation of 5H-Imidazo[1',2':1,2]pyrrolo[3,4-b]pyridine-3(2H),5-dione, 7-acetyl-2,8-dimethyl-2-isopropyl

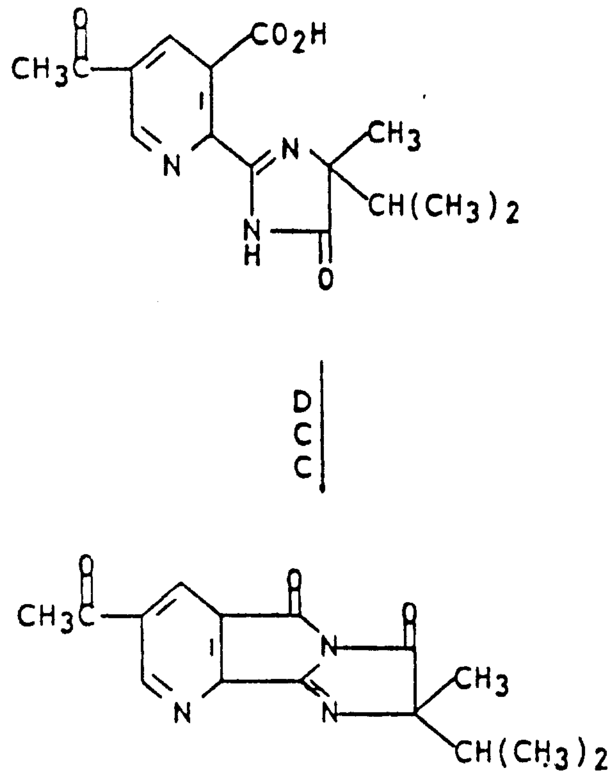


A solution of 2.6g 5-acetyl-2-(4-isopropyl-4-methyl-5-oxo-2-imidazolin-2-yl)nicotinic acid and 1.86g DCC in 100 mL methylene chloride is stirred at room temperature overnight. An additional 1.86g DCC is added and stirring continued over the weekend; a further 1.86g of DCC is added, and stirring continued for 1 more day. The reaction mixture is filtered, and the filtrate is stripped. Chromatography of the residue on silica gel using 5% ethyl acetate in methylene chloride affords 1.68g of the desired product, mp 158.160°.

EXAMPLE 16

Preparation of methyl 5-acetyl-2-(4-isopropyl-4-methyl-5-oxo-2-imidazolin-2-yl)-6-methylnicotinate

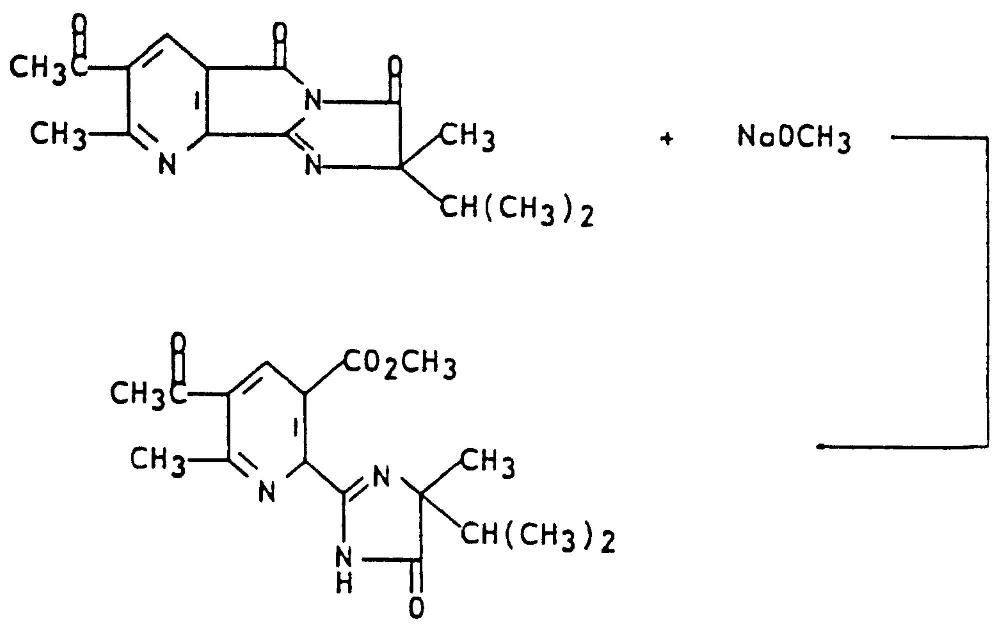

A solution of .68g of 7-acetyl-2,8-dimethyl-2-isopropyl-5H-imidazo[1',2':1,2]pyrrolo[3,4-b]pyridine-3(2H)-,5-dione in 50 mL methanol is stirred at 5° and .06g of sodium methoxide is added. The reaction is stirred and allowed to warm to room temperature over 3 hours, at which point it is acidified with acetic acid and concentrated in vacuo. The residue is dissolved in methylene chloride, washed with water, dried and concentrated in vacuo to afford the desired product. Recrystallization from methylene chloride-hexanes gives a solid, mp 118-120°.

EXAMPLE 17

Preparation of furfuryl 5-acetyl-2-(4-isopropyl-4-methyl-5-oxo-2-imidazolin-2-yl)-6-methylnicotinate

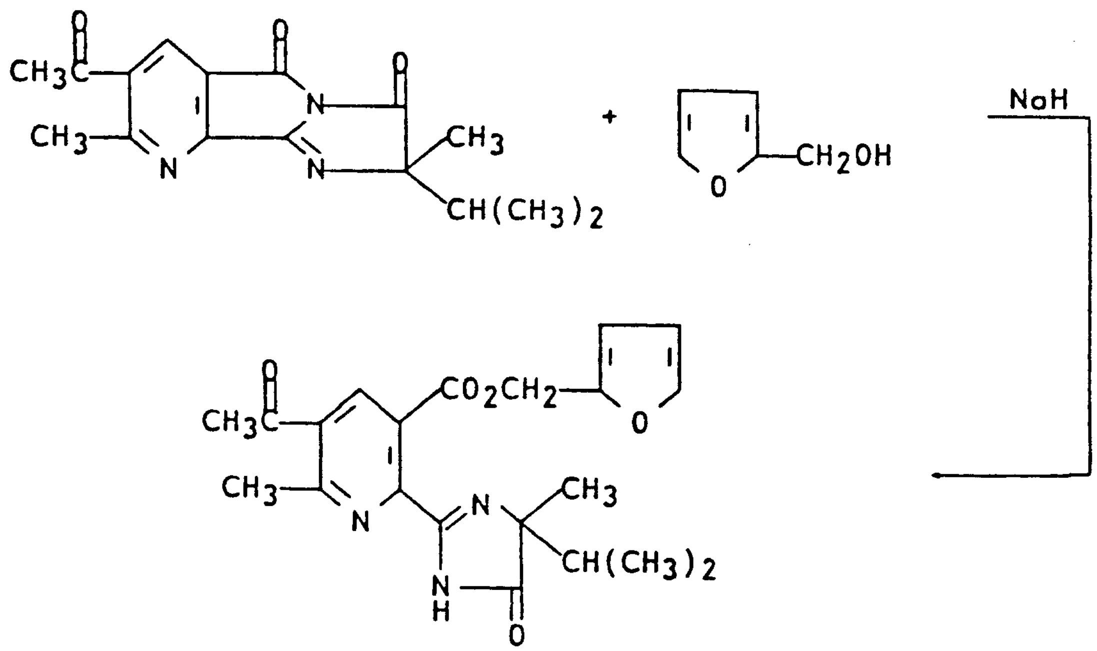


A solution of 1.0g of 7-acetyl-2,8-dimethyl-2-isopropyl-5H-imidazo[1',2':1,2]pyrrolo[3,4-b]pyridine-3(2H)-,5-dione in 50 mL tetrahydrofuran is added dropwise to a preformed mixture of .03g sodium hydride and .35 mL furfuryl alcohol in 50 mL tetrahydrofuran. The reaction mixture is stirred at room temperature overnight, acidified with acetic acid, and concentrated in vacuo. The residue is chromatographed on silica gel using 5% ethyl acetate in methylene chloride as eluant to afford the desired product. Recrystallization from methylene chloride-hexanes gives a solid, mp 133-136°.

EXAMPLE 18

Preparation of methyl 2-(1-acetyl-4-isopropyl-4-methyl-5-oxo-2-imidazolin-2-yl)-6-methylnicotinate

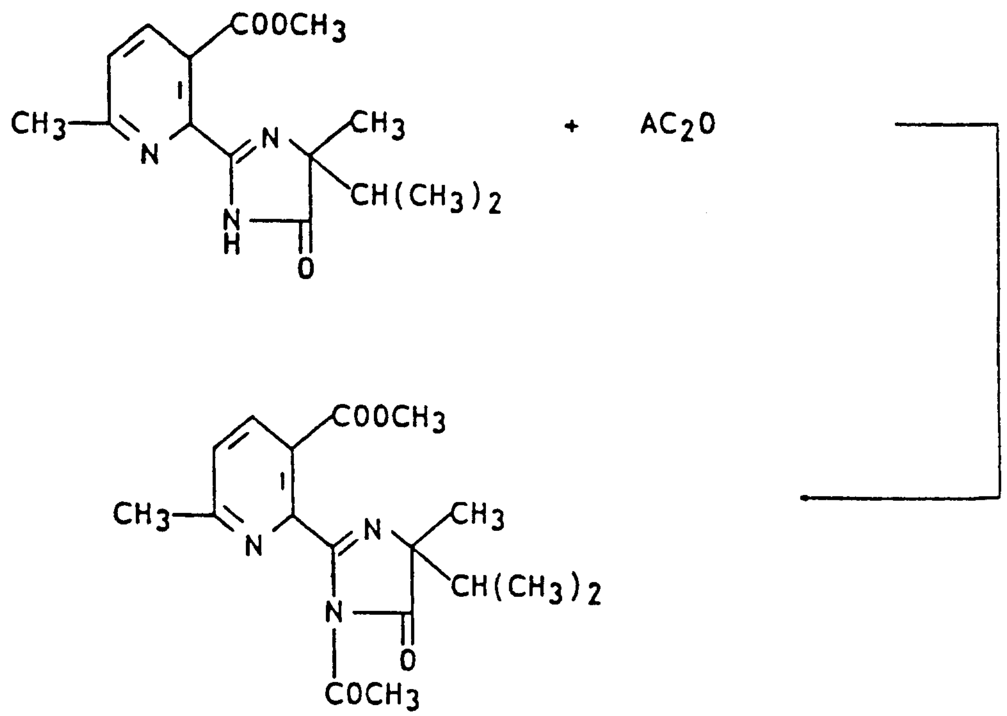

A solution of 33.2g of methyl 2-(4-isopropyl-4-methyl-5-oxo-2-imidazolin-2-yl)-6-methylnicotinate in 350 mL of acetic anhydride is stirred and heated at reflux for 4 hours. The solution is concentrated in vacuo, and the residue is triturated with 2:1 hexane-ether to give the desired product as a white solid.

EXAMPLE 19

Preparation of methyl 2-(1-acetyl-4-isopropyl-4-methyl-5-oxo-2-imidazolin-2-yl)-6-methylnicotinate-1-oxide

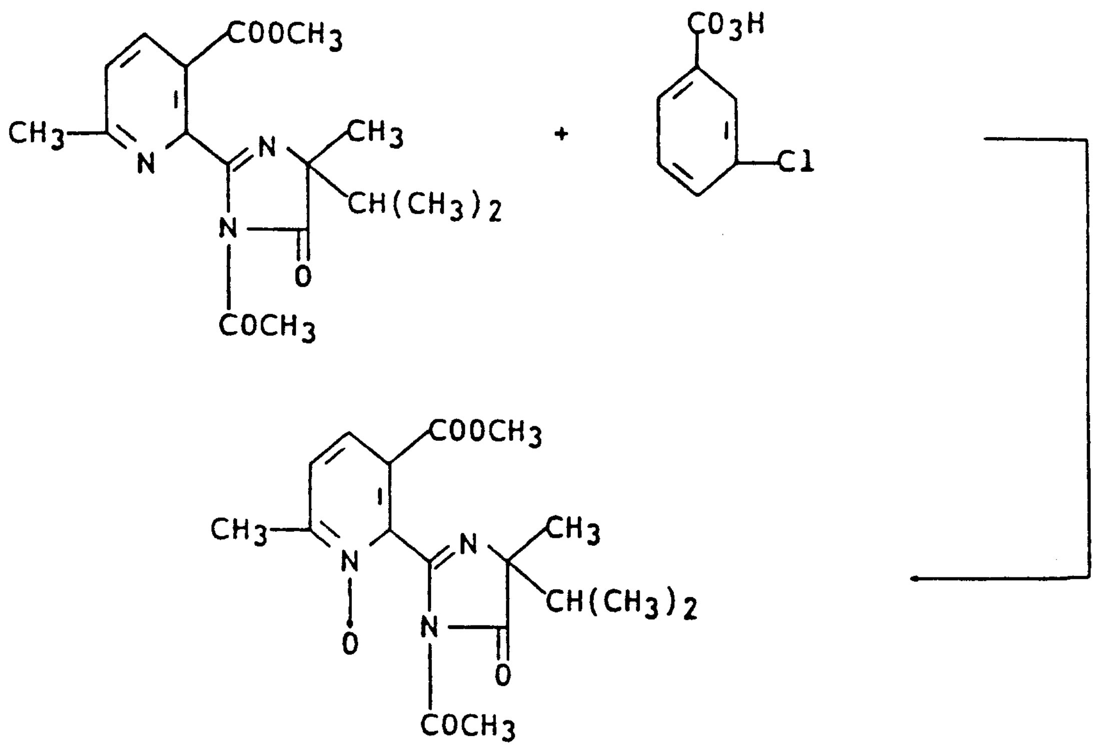

A solution of 29g of methyl 2-(1-acetyl-4-isopropyl-4-methyl-5-oxo-2-imidazolin-2-yl)-6-methylnicotinate and 28.7g of 80% M-chloroperbenzoic acid in 250 mL of methylene chloride is stirred and heated at reflux for 3 1/2 hours. The reaction mixture is filtered, and the solid is thoroughly washed with methylene chloride. The combined filtrates are cooled and treated cautiously with saturated aqueous sodium bisulfite until a negative starch-iodide test is obtained. The mixture is again filtered, and the organic layer is washed with saturated sodium bicarbonate solution, dried, and concentrated in vacuo to give an oil. Trituration of this oil with ether affords the desired product as a pale yellow solid, mp 135-138.5°C.

EXAMPLE 20

Preparation of methyl 6-acetoxymethyl-2-(1-acetyl-4-isopropyl-4-methyl-5-oxo-2-imidazolin-2-yl)nicotinate

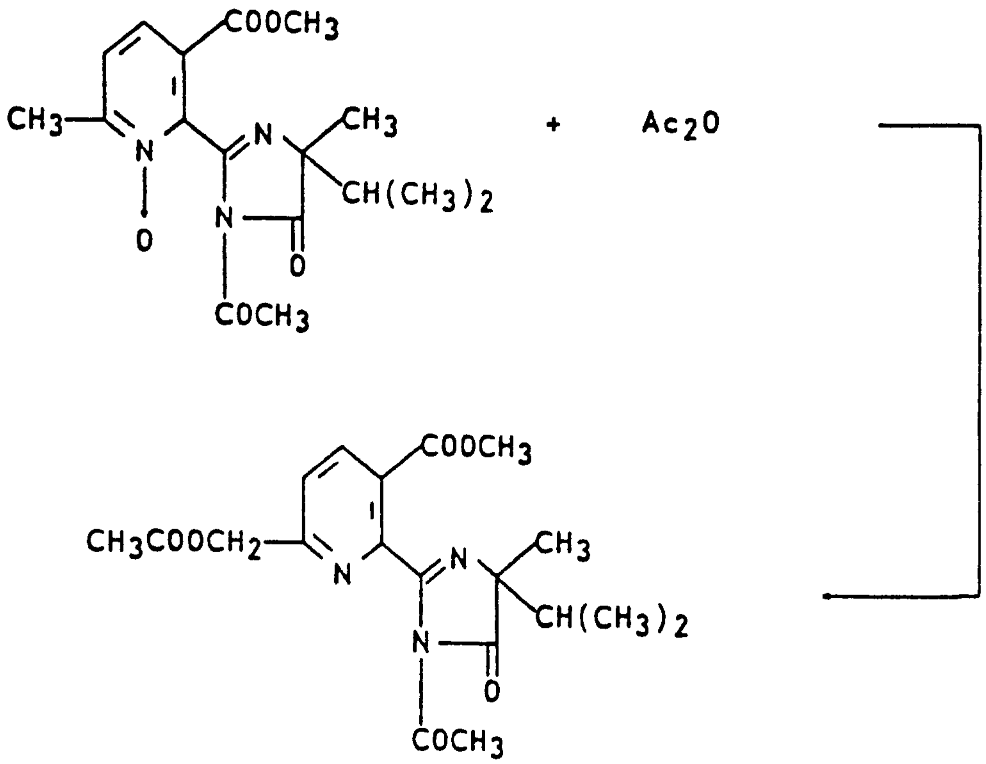

A solution of 37.6g of methyl 2-(1-acetyl-4-isopropyl-4-methyl-5-oxo-2-imidazolin-2-yl)-6-methylnicotinate-1-oxide in 350 mL of acetic anhydride is stirred and heated at reflux for 3 1/2 hours. The reaction is concentrated in vacuo, and the residue is chromatographed on silica gel using hexane-ethyl acetate as eluant to give the desired product as a solid. Recrystallization of the solid from methylene chloride-hexanes affords analytically pure material, mp 111-112°C.

EXAMPLE 21

Preparation of methyl 6-hydroxymethyl-2-(4-isopropyl-4-methyl-5-oxo-2-imidazolin-2-yl)nicotinate

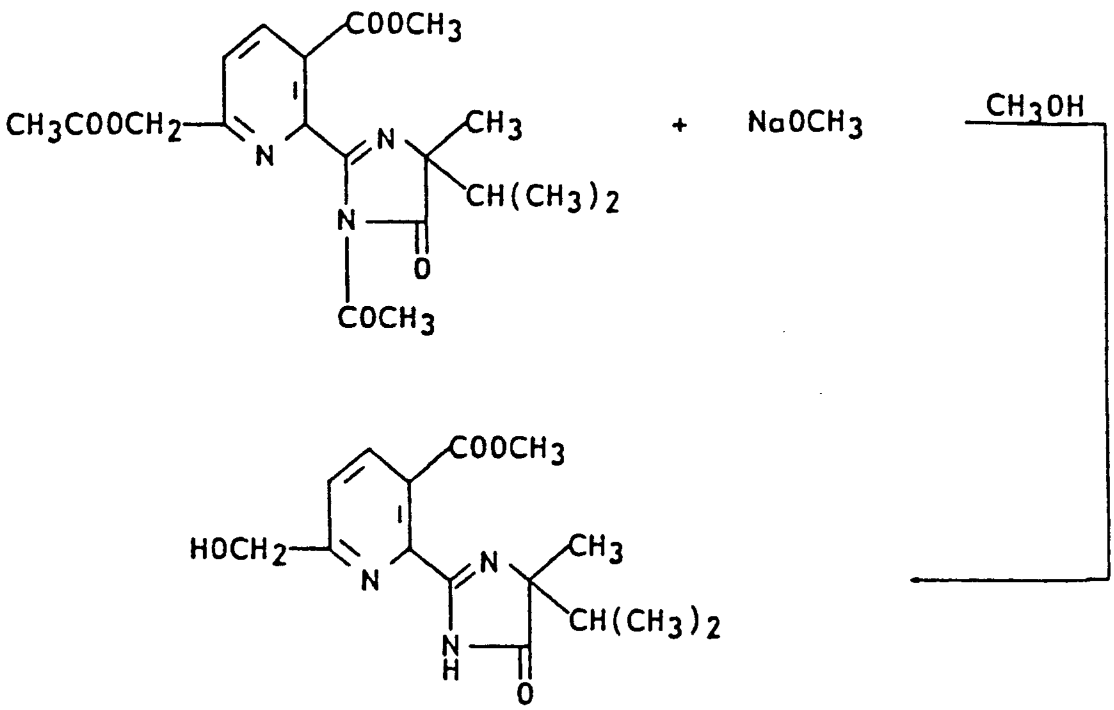

A solution of 16.7g of methyl 6-acetoxymethyl-2-(1-acetyl-4-isopropyl-4-methyl-5-oxo-2-imidazolin-2-yl) nicotinate in 125 mL methanol containing 0.7g of sodium methoxide is stirred at room temperature for 16 hours. The solution is adjusted to pH 5 with acetic acid and concentrated in vacuo. The residue is chromatographed on silica gel using ether-methanol as eluant to give the desired product as a solid. Recrystallization from methylene chloride-hexane gives analytically pure material, mp 115-118°C.

EXAMPLE 22

Preparation of methyl 6-chloromethyl-2-(4-isopropyl-4-methyl-5-oxo-2-imidazolin-2-yl)nicotinate

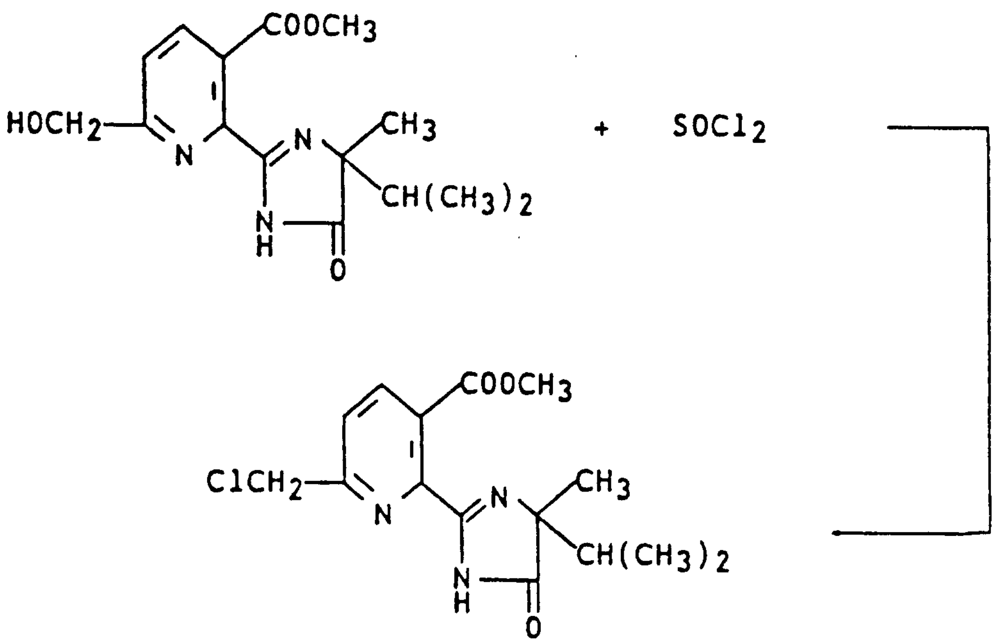

To a solution of 8.7g of methyl 6-hydroxymethyl-2-(4-isopropyl-4-methyl-5-oxo-2-imidazolin-2-yl)-nicotinate in 90 mL chloroform, stirred at room temperature, is added dropwise 4.2 mL of thionyl chloride. When the addition is complete, the solution is heated at reflux for 1 hour. The reaction is concentrated in vacuo, and the residue is dissolved in methylene chloride and washed with saturated sodium bicarbonate solution. The organic phase is dried and concentrated in vacuo to an oil. Trituration of this oil affords the desired product as a solid. Recrystallization from methylene chloride-hexane gives analytically pure material, mp 127-129°C.

EXAMPLE 23

Preparation of methyl 2-(4-isopropyl-4-methyl-5-oxo-2-imidazolin-2-yl)-6-methylthiomethylnicotinate

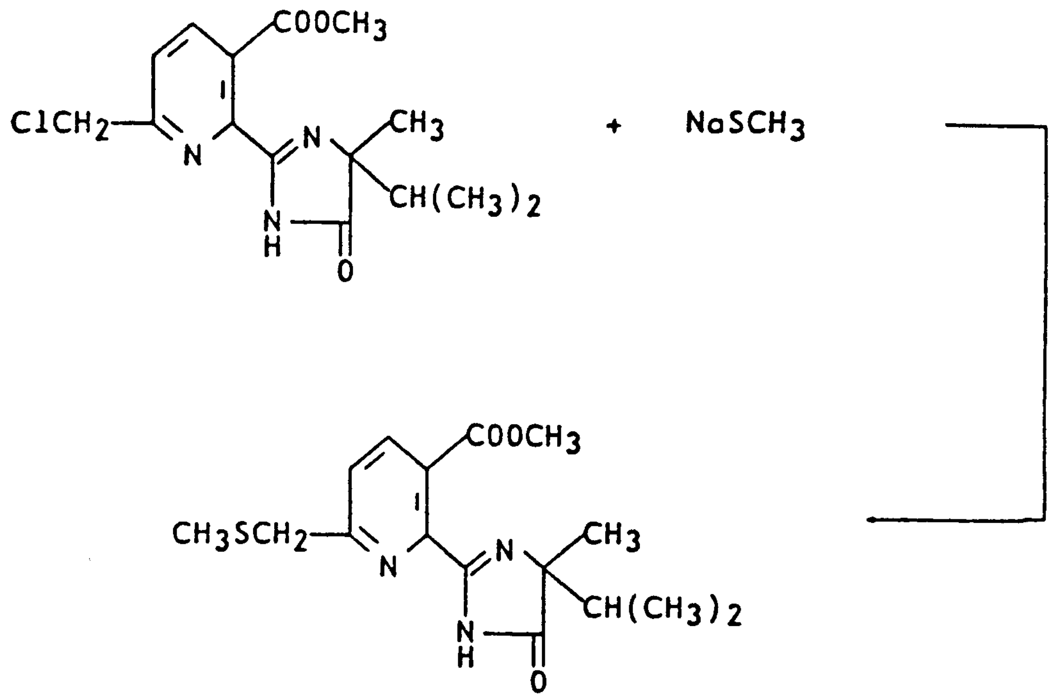


To 30 mL of absolute methanol, stirred at -10° under nitrogen, is added in portions 0.9g of 50% sodium hydride (oil dispersion). A stream of methyl mercaptan is then added to the solution until a pH of 10 is achieved. To this solution is added 3g of methyl 6-chloromethyl-2-(4-isopropyl-4-methyl-5-oxo-2-imidazolin-2-yl)nicotinate, and stirring is continued at -10° for 1 hour. The reaction is then allowed to warm to room temperature over 1 hour, and is then acidified with glacial acetic acid to pH 4. The mixture is concentrated in vacuo, and the residue is dissolved in methylene chloride, washed with saturated aqueous sodium bicarbonate, dried, and concentrated in vacuo. The residue is dissolved in absolute methanol, washed with hexanes, and concentrated in vacuo to afford the desired product. Recrystallization from methylene chloride-hexane affords a solid, mp 113.5-115°C.

EXAMPLE 24

Preparation of 1-dimethylamino-1-(2-furyl)ethylene

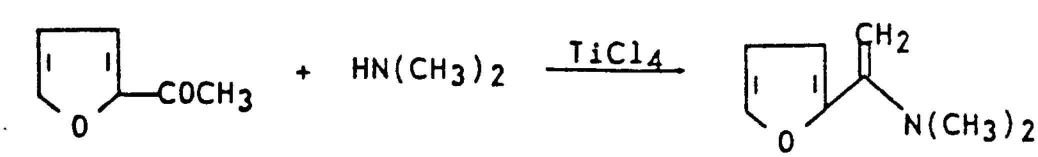


To a solution of 55g of 2-acetylfuran and 193g of dimethylamine in 1 liter of dry benzene, stirred at 3° under nitrogen, is added dropwise a solution of 31 mL of titanium tetrachloride in 100 mL benzene over 45 minutes. The mixture is stirred for a further 3 hours at 3°, then at ambient temperature overnight. The brown reaction mixture is filtered through a medium frit glass funnel under nitrogen, and the filtered solids are washed with dry benzene. The combined filtrates are concentrated in vacuo to give the desired enamine as an oil.

EXAMPLE 25

Preparation of 1-morpholino-2-(2-thienyl)ethylene

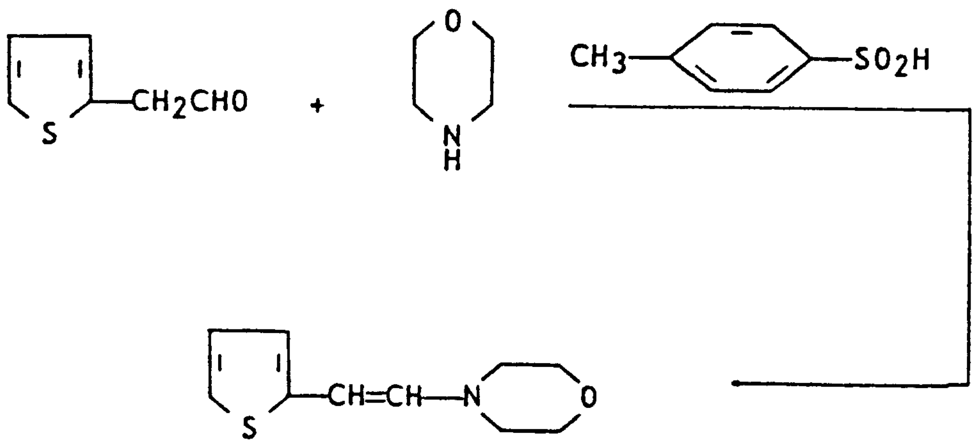

A mixture of 1.5g of 2-thienylacetaldehyde, 1.4 mL of morpholine, and .012g of p-toluenesulfonic acid hydrate in 7.5 mL of toluene is heated at reflux under a Dean-Stark Trap with azeotropic removal of water for 3 hours. The solution is cooled, neutralized by the addition of solid sodium methoxide, and concentrated in vacuo. The residue is taken up in toluene, filtered, and concentrated in vacuo to afford the desired enamine as an oil.

Using the same procedure, 1-morpholino-2-(3-thienyl)ethylene is prepared.

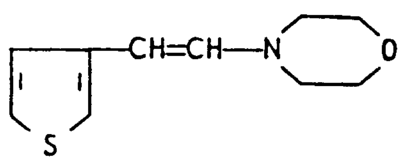

EXAMPLE 26

Preparation of diethyl 6-(2-furyl)pyridine-2,3-dicarboxylate

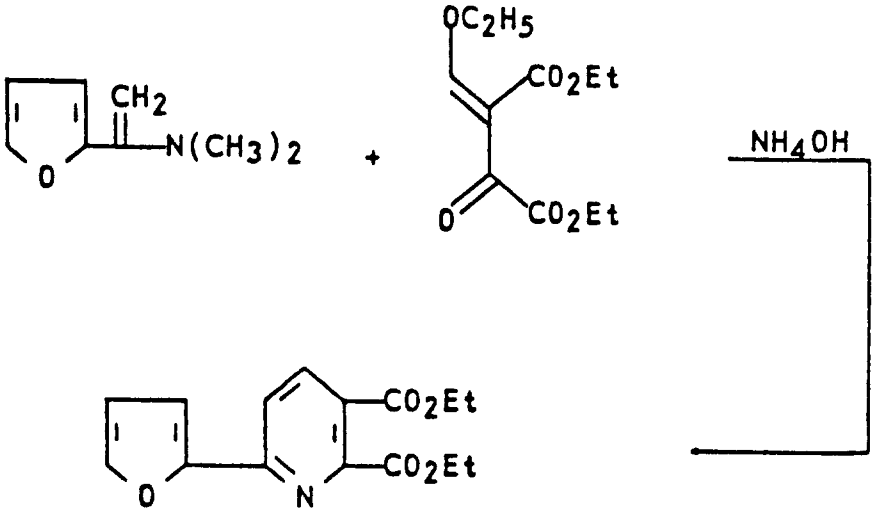


A solution of 147g of diethyl ethoxymethyleneoxalacetate in 600 mL of absolute ethanol is stirred at 5°C while a solution of 68.8g of 1-dimethylamino-1-(2-furyl)ethylene in 400 mL of absolute ethanol is added dropwise over 1 hour. After stirring for an additional 2 hours, the reaction mixture is treated with 250 mL of concentrated aqueous ammonium hydroxide. The resulting mixture is stirred at ambient temperature overnight and then concentrated in vacuo. Chromatography of the residue on silica gel using chloroform as eluant affords the desired product.

Using essentially the same procedure, and substituting the appropriate enamine, the following are prepared: the 5-(2-thienyl), the 6-(2-thienyl), and the 5-(3-thienyl) pyridine-2,3-dicarboxylates.

| | Y | Z | mp |
|---|---|---|---|
| 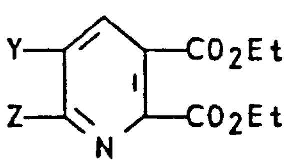 | 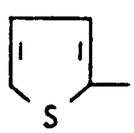 | H | Solid |
| | H | 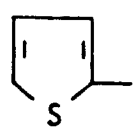 | 97-99° |
| | 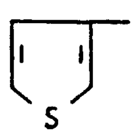 | H | 64-66° |

EXAMPLE 27

Preparation of 6-hydroxy-2-(4-isopropyl-4-methyl-5-oxo-2-imidazolin-2-yl)nicotinic acid

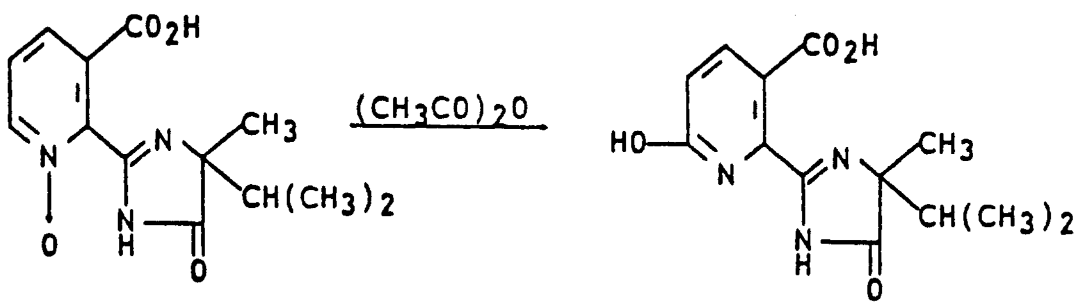


A mixture of 3g of 2-(4-isopropyl-4-methyl-5-oxo-2-imidazolin-2-yl)nicotinic acid -1-oxide, 2.24g of acetic anhydride, and 0.4g of pyridine in 80 mL of toluene is stirred and heated at reflux for 3 hours. The reaction mixture is cooled to 10°C and 75 mL of cold water is added with vigorous stirring. The resulting yellow solid is filtered, washed with toluene, and air dried. Recrystallization of this solid from methanol affords the desired product as a white solid, mp 201-203°C.

EXAMPLE 28

Preparation of diethyl pyridine-2,3-dicarboxylate-N-oxide

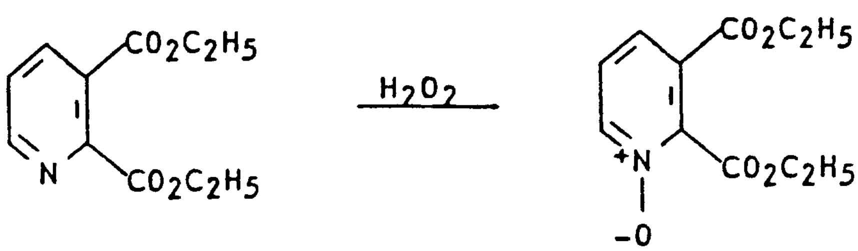


A mixture of 99g of diethyl pyridine-2,3-dicarboxylate, 7.2 mL of concentrated sulfuric acid, 85 mL of 30% hydrogen peroxide and 256 mL acetic acid is stirred and heated at 85° for 4 hours. The reaction is concentrated in vacuo, and the residue is dissolved in methylene chloride, and washed with water, then saturated sodium bicarbonate. After drying, the organic phase is concentrated in vacuo to afford 79g of the desired product, mp 80-85°. A portion recrystallized from carbon tetrachloridepentane has mp 87-88°.

EXAMPLE 29

Preparation of diethyl 6-chloropyrldine-2,3-dicarboxylate

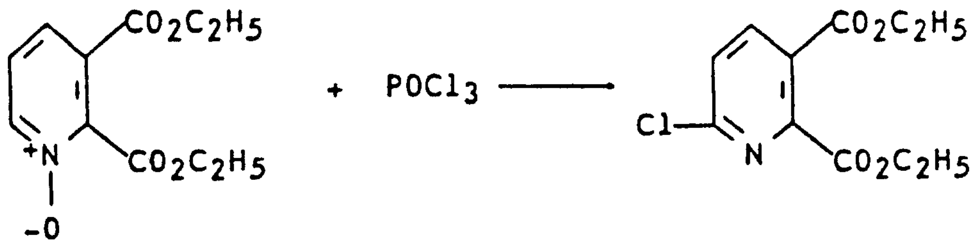

A mixture of 75.6g of diethyl pyridine-2,3-dicarboxylate-N-oxide and 150 mL of phosphorous oxychloride is warmed slowly in a large reaction vessel equipped with a reflux condenser and gas scrubber. Caution: exothermic reaction! At about 70°, an exothermic reaction ensues with vigorous liberation of hydrogen chloride gas. The reaction is heated at reflux after the exotherm subsides for an additional hour. The reaction is cooled and concentrated in vacuo to a syrup. This material is dissolved in methylene chloride, washed cautiously with saturated sodium bicarbonate, dried and concentrated in vacuo to give the desired product as an oil. An analytical sample is obtained by chromatography on silica gel using 2:1 petroleum ether-ether as eluant to give the product as a colorless oil.

EXAMPLE 30

Preparation of 6-hydroxypyridine-2,3-dicarboxylic acid

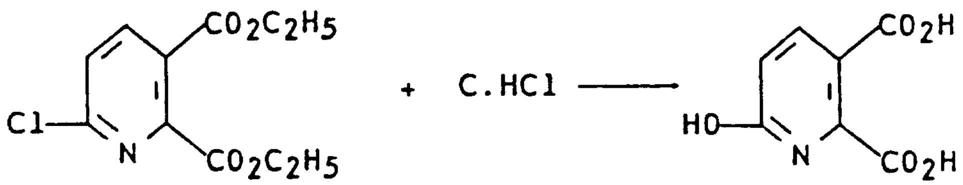

A mixture of 72g of diethyl 6-chloropyridine-2,3-dicarboxylate and 420 mL of concentrated hydrochloric acid is stirred and heated at reflux overnight. The reaction mixture is cooled, and the solid is filtered and washed with hot water to give the desired product as a solid.

EXAMPLE 31

Preparation of 6-H-pyrrolo[3,4-b]pyridine-6-acetonitrile, 5,7-dihydro-2-hydroxy-α-isopropyl-α-methyl-5,7-dioxo-, acetate (ester)

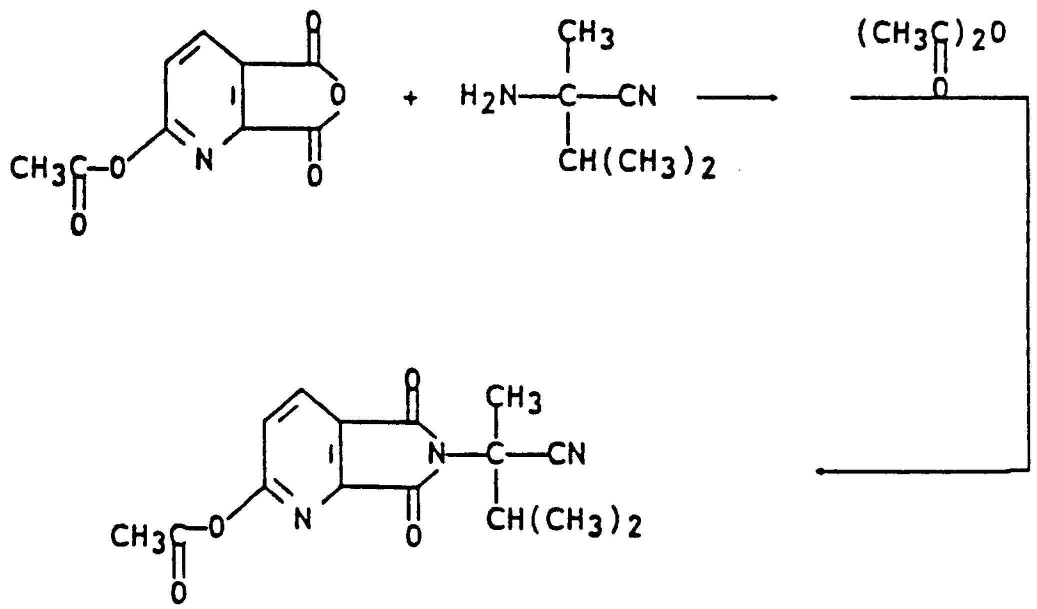

A mixture of 30g of crude 6-acetoxypyridine-2,3-dicarboxylic anhydride and 21g of 2-amino-2,3-dimethylbutyronitrile in 100 mL of methylene chloride is stirred at room temperature (exotherm) for 15 minutes, then at reflux for an additional 5 minutes. The solvent is removed in vacuo, and the residue is mixed with 15g sodium acetate and 300 mL of acetic anhydride and heated at reflux for 35 minutes. The reaction mixture is cooled and filter chromatographed through 200g of silica gel, using additional amounts of dilute methanol in ethyl acetate as eluant. The combined filtrates are concentrated in vacuo to give the desired product as an oil. A portion can be crystallized from carbon tetrachloride-pentane to give an analytically pure solid, mp 104.5-108°.

EXAMPLE 32

Preparation of 6H-pyrrolo[3,4-b]pyridine-6-acetamide, 5,7-dihydro-2-hydroxy-α-isopropyl-α-methyl-5,7-dioxo-

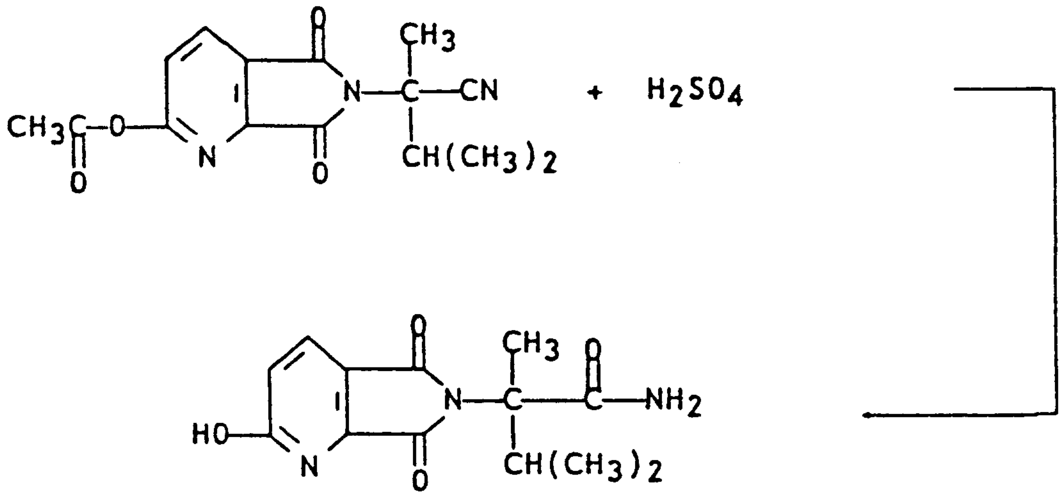

To a suspension of 34.5g of 5,7-dihydro-2-hydroxy-α-isopropyl-α-methyl-5,7-dioxo-6H-pyrrolo[3,4-b]-pyridine-6-acetonitrile, acetate(ester) in 150 mL ethylene dichloride, stirred in an ice bath, is added slowly 110g of concentrated sulfuric acid. The resulting mixture is heated at 60° for 3 hours, cooled to room temperature, mixed with 100g of sodium acetate, and poured onto ice-water with efficient stirring. Filtration and washing the filtered solid with water affords the desired product. A portion can be recrystallized from dimethylsulfoxide-water to give an analytically pure sample, mp 211-212°.

EXAMPLE 33

Preparation of methyl 6-hydroxy-2-(4-isopropyl-4-methyl--5-oxo-2-imidazolin-2-yl)nicotinate

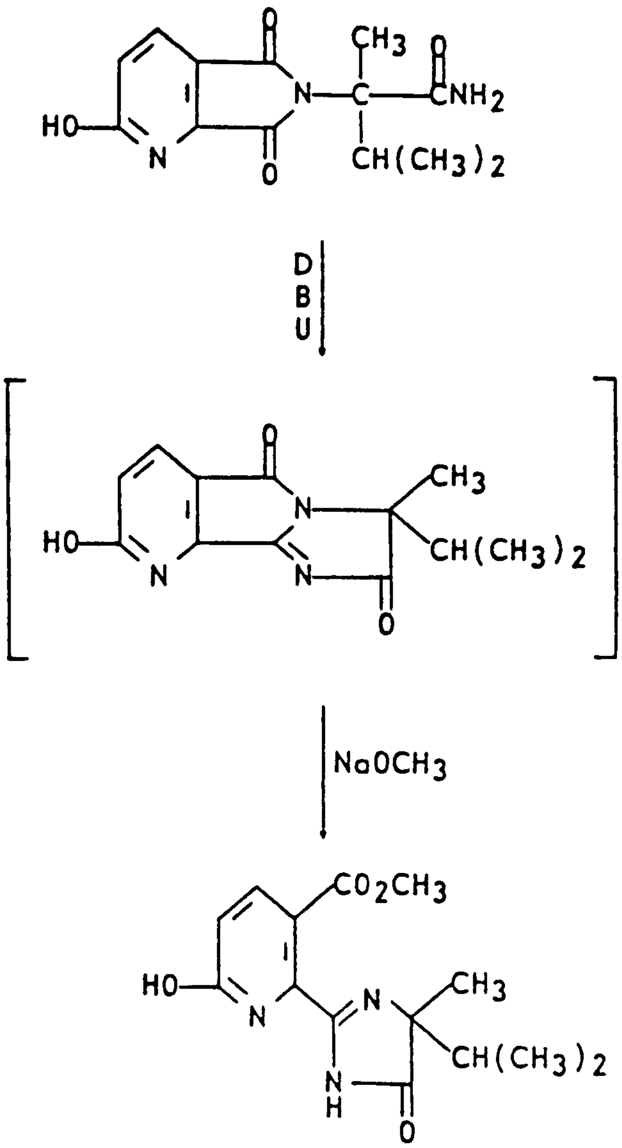


A mixture of 15g of 5,7-dihydro-2-hydroxy-α-isopropyl-α-methyl-5,7-dioxo-6H-pyrrolo[3,4-b]pyridine-6-acetamide and 600 mL xylene is heated at reflux under a water separator, and 2.46g of DBU is added in three portions over 20 minutes. After heating at reflux for an additional 45 minutes, the reaction is cooled to 100° and filtered through a sintered-glass funnel. The filtrate is treated with a solution of 6g sodium methoxide in 25 mL methanol and allowed to stand at room temperature overnight. The xylene layer is decanted off, and the residual yellow oil is dissolved in acetic acid and filtered through 50g of silica gel using additional 4:1 ethyl acetate-methanol as eluant. The combined filtrates are concentrated in vacuo and reevaporated from xylenes to give the desired product as a solid. A portion can be triturated with 50% aqueous methanol, filtered, washed with ether, and dried to give a solid, mp 186-191°.

EXAMPLE 34

Preparation of 2-(4-isopropyl-4-methyl-5-oxo-2-imidazolin-2-yl)-6-(2-thienyl)nicotinic acid, isopropylamine salt

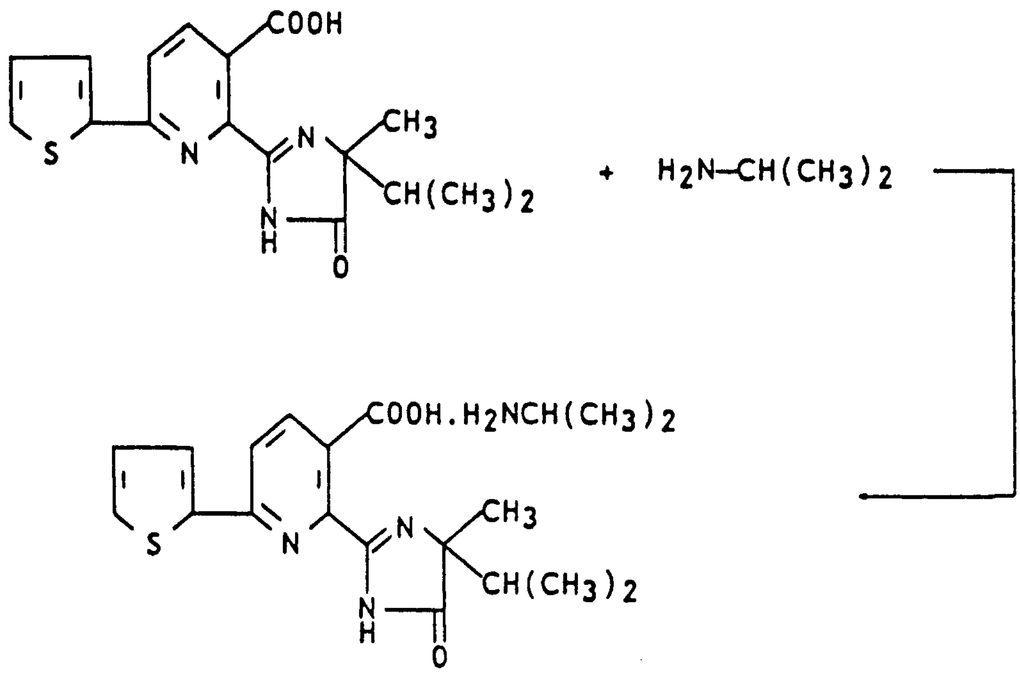

A solution of 2g of 2-(4-isopropyl-4-methyl-5-oxo-2-imidazolin-2-yl)-6-(2-thienyl)nicotinic acid and 0.34g isopropyl amine in 20 mL tetrahydrofuran is stirred at room temperature for 10 minutes. The reaction is diluted with 4 mL of hexane, filtered, and the filtrate is stirred overnight. The solid is filtered, washed with tetrahydrofuran, and dried in vacuo to give the desired product as a solid, mp 247-257°(dec).

Also prepared by this method is the isopropylamine salt of the 6-(furyl)nicotinic acid.

| | Y | Z | mp |
|---|---|---|---|
| 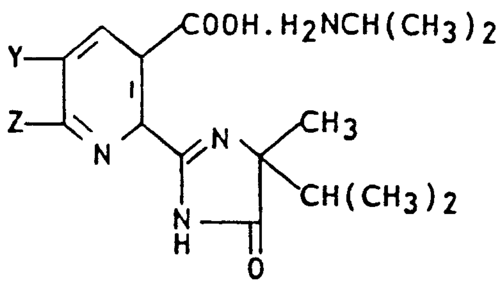 | H | 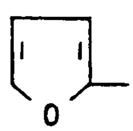 | 241-256°(dec) |

EXAMPLE 35

Preparation of methyl 2-(4-isopropyl-4-methyl-5-oxo-2-imidazolidinyi)-5-propenylnicotinate, cis and trans

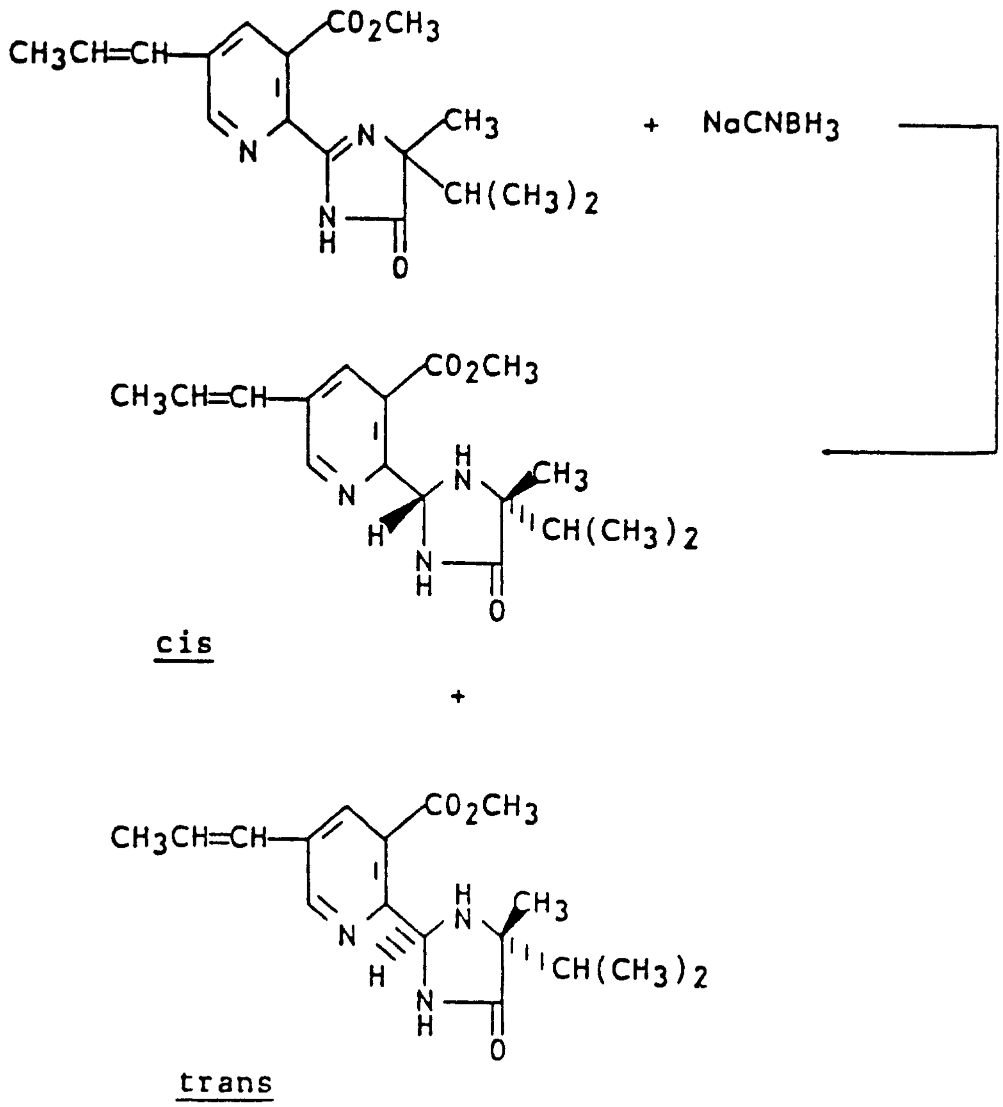


cis

trans

A solution of 2.0g of methyl 2-(4-isopropyl-4-methyl-5-oxo-2-imidazolin-2-yl)-5-propenylnicotinate in 50 mL of absolute methanol is cooled in an ice bath to 2° and the pH is adjusted to 3 with dilute methanolic hydrogen chloride. Sodium cyanoborohydride is added in small portions, with maintenance of the pH at 3 by concurrent addition of dilute methanolic hydrogen chloride, until the disappearance of the starting material is indicated by TLC. The reaction mixture is concentrated in vacuo, and the residue is chromatographed on silica gel using hexane-ethyl acetate mixtures as eluant to give the cis and trans products as solids.

EXAMPLE 36

Preemergence herbicidal evaluation of test compounds

The preemergence herbicidal activity of the compounds of the present invention is exemplified by the following tests in which the seeds of a variety of monocotyledonous and dicotyledonous plants are separately mixed with potting soil and planted on top of approximately one inch of soil in separate pint

cups. After planting, the cups are sprayed with the selected aqueous acetone solution containing test compound in sufficient quantity to provide the equivalent of about 0.016 to 8.0 kg per hectare of test compound per cup. The treated cups are then placed on greenhouse benches, watered and cared for in accordance with conventional greenhouse procedures. From four to five weeks after treatment, the tests are terminated and each cup is examined and rated according to the rating system set forth below. Data obtained are reported in Table I below. Where more than one test is involved for a given compound, the data are averaged.

Plant species employed in these evaluations are reported by header abbreviation, common name and scientific name.

Compounds employed in this preemergence herbicidal evaluation and in the post-emergence evaluation in the following example are given a compound number and identified by name. Data in Table I are reported by compound number.

***Herbicide Rating Scale

Results of herbicide evaluation are expressed on a rating scale (0-9). The scale is based upon a visual observation of plant stand, vigor, malformation, size, chlorosis and overall plant appearance as compared with a control.

| RATING | MEANING | % Control (COMPARED TO CHECK) |
|---|---|---|
| 9 | Complete Kill | 100 |
| 8 | Approaching Complete Kill | 91-99 |
| 7 | Good Herbicidal Effect | 80-90 |
| 6 | Herbicidal Effect | 65-79 |
| 5 | Definite Injury | 45-64 |
| 4 | Injury | 30-44 |
| 3 | Moderate Effect | 16-29 |
| 2 | Slight Effect | 6-15 |
| 1 | Trace Effect | 1-5 |
| 0 | No Effect | 0 |
| P | "PGR" Effect | --- |
| X | Unable to Read Sample | --- |

| PLANT SPECIES EMPLOYED IN HERBICIDAL EVALUATIONS | | |
|---|---|---|
| HEADER ABB | COMMON NAME | SCIENTIFIC NAME |
| BARNYARDGR | BARNYARDGRASS | ECHINOCHLOA CRUS-GALLI, (L)BEAU |
| LARGE CRAB | CRABGRASS, (HAIRY) LARGE | DIGITARIA SANGUINALIS, (L)SCOP |
| GREEN FOX | FOXTAIL, GREEN | SETARIA VIRIDIS, (L)BEAUV |
| P NUTSEDGE | NUTSEDGE, PURPLE | CYPERUS ROTUNDUS, L. |
| WILD OATS | OAT, WILD | AVENA FATUA, L. |
| QUACKGRASS | QUACKGRASS | AGROPYRON REPENS, (L)BEAUV. |
| FLD BINDWD | BINDWEED, FIELD (RHIZOME) | CONVOLVULUS ARVENSIS, L. |
| MATRICARIA | MATRICARIA | MATRICARIA CHAMOMILLA, L. |
| MRNGLRY SP | MORNINGGLORY SPP. | IPOMOEA SPP. |
| WILD MUSTD | MUSTARD, WILD | BRASSICA KABER, (DC)L.C. WHEELR |
| RAGWEED | RAGWEED, COMMON | AMBROSIA ARTEMISIIFOLIA, L. |
| PRIDY SIDA | SIDA, PRICKLY | SIDA SPINOSA, L. |
| VELVETLEAF | VELVETLEAF | ABUTILON THEOPHRASTI, MEDIC. |
| S BARLY LA | BARLEY,SPRING,LARKER | HORDEUM VULGARE, CV.LARKER |
| SUGARBEETS | SUGARBEETS | BETA VULGARIS, L. |
| CORN FIELD | CORN, FIELD | ZEA MAYS, L. |

## COMPOUNDS EVALUATED AS HERBICIDAL AGENTS

| Compound No. | |
|---|---|
| 1 | (E)-2-(4-isopropyl-4-methyl-5-oxo-2-imidazolin-2-yl)-5-styryl-nicotinic acid |
| 2 | (Z)-2-(4-isopropyl-4-methyl-5-oxo-2-imidazolin-2-yl)-5-styryl-nicotinic acid |
| 3 | methyl 5-isopropenyl-2-(4-isopropyl-4-methyl-5-oxo-2-imidazolin-2-yl)nicotinate |
| 4 | 5-isopropenyl-2-(4-isopropyl-4-methyl-5-oxo-2-imidazolin-2-yl)nicotinic acid |
| 5 | 1,6-dihydro-2-(4-isopropyl-4-methyl-5-oxo-2-imidazolin-2-yl)-6-oxonicotinic acid |
| 6 | methyl 5-(1-hydroxyethyl)-2-(4-isopropyl-4-methyl-5-oxo-2-imidazolin-2-yl)nicotinate acetate(ester) |
| 7 | 5-acetyl-2-(4-isopropyl-4-methyl-5-oxo-2-imidazolin-2-yl)-6-methyl-nicotinic acid |
| 8 | 2-(4-isopropyl-4-methyl-5-oxo-2-imidazolin-2-yl)-6-(2-thienyl)nicotinic acid |
| 9 | methyl 5-acetyl-2-(4-isopropyl-4-methyl-5-oxo-2-imidazolin-2-yl)-6-methylnicotinate |
| 10 | isopropylammonium 2-(4-isopropyl-4-methyl-5-oxo-2-imidazolin-2-yl)-6-(2-thienyl)-nicotinate |

| Compound No. | |
|---|---|
| 11 | furfuryl 5-acetyl-2-(4-isopropyl-4-methyl-5-oxo-2-imidazolin-2-yl)-6-methylnicotinate |
| 12 | methyl 2-(4-isopropyl-4-methyl-5-oxo-2-imidazolin-2-yl)-6-(2-thienyl)nicotinate |
| 13 | 6-(2-furyl)-2-(4-isopropyl-4-methyl-5-oxo-2-imidazolin-2-yl)nicotinic acid |
| 14 | isopropylammonium 6-(2-furyl)-2-(4-isopropyl-4-methyl-5-oxo-2-imidazolin-2-yl)nicotinate |
| 15 | methyl 6-(2-furyl)-2-(4-isopropyl-4-methyl-5-oxo-2-imidazolin-2-yl)nicotinate |
| 16 | methyl 5-acetyl-2-(4-isopropyl-4-methyl-5-oxo-2-imidazolin-2-yl)nicotinate |
| 17 | 5-acetyl-2-(4-isopropyl-4-methyl-5-oxo-2-imidazolin-2-yl)nicotinic acid |
| 18 | methyl 2-(4-isopropyl-4-methyl-5-oxo-2-imidazolin-2-yl)-5-propenylnicotinate |
| 19 | (E)-2-(4-isopropyl-4-methyl-5-oxo-2-imidazolin-2-yl)-5-propenylnicotinic acid |
| 20 | 2-(4-isopropyl-4-methyl-5-oxo-2-imidazolin-2-yl)-5-(2-thienyl)nicotinic acid |

| Compound No. | |
|---|---|
| 21 | 2-(4-isopropyl-4-methyl-5-oxo-2-imidazolin-2-yl)-5-[(methylthio)methyl]nicotinic acid |
| 22 | 2-(4-isopropyl-4-methyl-5-oxo-2-imidazolin-2-yl)-5-(3-thienyl)nicotinic acid |
| 23 | methyl 1,6-dihydro-2-(4-isopropyl-4-methyl-5-oxo-2-imidazolin-2-yl)-6-oxonicotinate |
| 24 | methyl 2-(4-isopropyl-4-methyl-5-oxo-2-imidazolin-2-yl)-6-[(methylthio)methyl]-nicotinate |
| 25 | 2-(4-isopropyl-4-methyl-5-oxo-2-imidazolin-2-yl)-6-[(methylthio)methyl]nicotinic acid |

TABLE I

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 1 | 4.000 | 2.0 | | 0.0 | 0.0 | 0.0 | 0.0 | 9.0 | 3.0 | | 0.0 | | 3.0 | 3.0 | | 7.0 | 7.0 |
| | 1.000 | 0.0 | | 0.0 | 0.0 | 0.0 | 0.0 | 7.0 | 0.0 | | 0.0 | | 0.0 | 3.0 | | 0.0 | 9.0 |
| | .500 | 0.0 | | 0.0 | 0.0 | 0.0 | 0.0 | 7.0 | 0.0 | | 0.0 | | 0.0 | 0.0 | | 0.0 | 4.0 |
| | .250 | 0.0 | | 0.0 | 0.0 | 0.0 | 0.0 | 2.0 | 0.0 | | 0.0 | | 0.0 | 0.0 | | 0.0 | 4.0 |
| | .125 | 0.0 | | 0.0 | 0.0 | 0.0 | 0.0 | 0.0 | 0.0 | | 0.0 | | 0.0 | 0.0 | | 0.0 | |
| | .063 | 0.0 | | 0.0 | 0.0 | 0.0 | | | 0.0 | | 0.0 | | 0.0 | 0.0 | | | |
| 2 | 4.000 | 2.0 | | 3.0 | 4.0 | 4.0 | 9.0 | 6.0 | 5.0 | | 2.0 | | 6.0 | 9.0 | | 8.0 | 2.0 |
| | 1.000 | 1.0 | | 1.0 | 3.0 | 1.0 | 7.0 | 0.0 | 0.0 | | 0.0 | | 5.0 | 0.0 | | 7.0 | 1.0 |
| | .500 | 0.0 | | 0.0 | 2.0 | 0.0 | 2.0 | 0.0 | 0.0 | | 0.0 | | 0.0 | 0.0 | | 6.0 | 1.0 |
| 3 | .500 | 8.0 | | 6.0 | 8.0 | 9.0 | 7.0 | 9.0 | 3.0 | | 3.0 | | 3.0 | 8.0 | | 8.0 | 9.0 |
| | .250 | 5.0 | | 5.0 | 4.0 | 7.0 | 7.0 | 9.0 | 1.0 | | 2.0 | | 3.0 | 3.0 | | 8.0 | 9.0 |
| | .125 | 1.0 | | 1.0 | 4.0 | 6.0 | 3.0 | 3.0 | 1.0 | | 1.0 | | 2.0 | 1.0 | | 7.0 | 5.0 |
| | .053 | 0.0 | | 0.0 | 1.0 | 4.0 | 2.0 | 1.0 | 0.0 | | 0.0 | | 2.0 | 0.0 | | 4.0 | 3.0 |
| | .032 | 0.0 | | 0.0 | 0.0 | 2.0 | 1.0 | 0.0 | 0.0 | | 0.0 | | 0.0 | 0.0 | | 3.0 | 2.0 |
| | .016 | 0.0 | | 0.0 | 0.0 | 1.0 | 0.0 | 0.0 | 0.0 | | 0.0 | | 0.0 | 0.0 | | 2.0 | 1.0 |
| 4 | 8.000 | 9.0 | 9.0 | 9.0 | 9.0 | 9.0 | 9.0 | 9.0 | 8.0 | 9.0 | 9.0 | 9.0 | 9.0 | | | | |
| | .500 | 9.0 | | | 9.0 | 9.0 | 9.0 | 9.0 | 9.0 | | 9.0 | | 7.0 | 9.0 | | 9.0 | 9.0 |
| | .250 | 8.0 | | | 8.0 | 8.0 | 9.0 | 9.0 | 7.0 | | 9.0 | | 7.0 | 9.0 | | 8.0 | 9.0 |
| | .125 | 6.0 | | | 2.0 | 8.0 | 6.0 | 9.0 | 6.0 | | 9.0 | | 6.0 | 7.0 | | 8.0 | 8.0 |
| | .063 | 6.0 | | | 2.0 | 6.0 | 6.0 | 9.0 | 4.0 | | 9.0 | | 4.0 | 7.0 | | 8.0 | 7.0 |
| | .032 | 4.0 | | | 7.0 | 7.0 | 4.0 | 7.0 | 3.0 | | 9.0 | | 3.0 | 7.0 | | 8.0 | 5.0 |
| | .016 | 3.0 | | | 2.0 | 5.0 | 4.0 | 6.0 | 1.0 | | 0.0 | | 2.0 | 4.0 | | 7.0 | 5.0 |

TABLE I (Cont.)

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 5 | .750 | 6.0 | 0.0 | 7.0 | 9.0 | | 9.0 | 9.0 | 7.0 | | 0.0 | 7.0 | 7.0 | | | | 7.0 |
| | .500 | 3.0* | 0.0 | 5.0 | 8.5 | 2.0 | 8.5 | 9.0 | 5.5 | | 0.0 | 6.0 | 7.5 | 0.0 | | 9.0 | 3.5 |
| | .375 | 3.0 | 0.0 | 4.0 | 8.0 | | 7.0 | 8.0 | 4.0 | | 0.0 | 6.0 | 7.0 | | | | 4.0 |
| | .250 | 0.0 | 0.0 | 0.0 | 4.5 | 1.0 | 4.0 | 4.0* | 0.0 | | 0.0 | 4.0 | 3.5 | 0.0 | | 8.0 | 0.5 |
| | .188 | 0.0 | 0.0 | 0.0 | 6.0 | | 3.0 | 8.0 | 0.0 | | 0.0 | 0.0 | 0.0 | | | | 0.0 |
| | .125 | 0.0 | 0.0 | 0.0 | 3.0 | 0.0 | 1.5 | 4.0* | 0.0 | | 0.0 | 0.0 | 0.5 | 0.0 | | 1.0 | 0.5 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| | .016 | 0.0 | | | 0.0 | 0.0 | 0.0 | 0.0 | 0.0 | | 0.0 | | 0.0 | 0.0 | | 0.0 | 0.0 |
| 6 | .500 | 0.0 | | | 8.0 | 0.0 | 4.0 | 8.0 | 8.0 | | 0.0 | | 3.0 | 0.0 | | 8.0 | 4.0 |
| | .250 | 0.0 | | | 3.0 | 0.0 | 2.0 | 7.0 | 7.0 | | 0.0 | | 3.0 | 0.0 | | 7.0 | 0.0 |
| | .125 | 0.0 | | | 0.0 | 0.0 | 0.0 | 7.0 | 1.0 | | 0.0 | | 0.0 | 0.0 | | 7.0 | 0.0 |
| | .063 | 0.0 | | | 0.0 | 0.0 | 0.0 | | 0.0 | | 0.0 | | 0.0 | 0.0 | | 7.0 | 0.0 |
| | .032 | 0.0 | | | 0.0 | 0.0 | 0.0 | 4.0 | 0.0 | | 0.0 | | 0.0 | 0.0 | | 2.0 | 0.0 |
| | .016 | 0.0 | | | 0.0 | 0.0 | 0.0 | 2.0 | 0.0 | | 0.0 | | 0.0 | 0.0 | | 0.0 | 0.0 |
| 7 | 8.000 | 9.0 | 9.0 | 9.0 | 9.0 | 9.0 | 9.0 | 9.0 | 8.0 | 9.0 | 9.0 | 9.0 | 9.0 | | | | |
| | .500 | 9.0 | | | 9.0 | 9.0 | 9.0 | 9.0 | 9.0 | | 9.0 | | 8.0 | 6.0 | | 9.0 | 9.0 |
| | .250 | 8.0 | | | 9.0 | 9.0 | 9.0 | 9.0 | 9.0 | | 8.0 | | 7.0 | 2.0 | | 9.0 | 9.0 |
| | .125 | 7.0 | | | 9.0 | 9.0 | 9.0 | 9.0 | 9.0 | | 8.0 | | 8.0 | 2.0 | | 9.0 | 9.0 |
| | .063 | 2.0 | | | 9.0 | 9.0 | | 9.0 | 8.0 | | 6.0 | | 7.0 | 0.0 | | 9.0 | 7.0 |
| | .032 | 0.0 | | | 9.0 | 9.0 | 9.0 | 3.0 | 6.0 | | 2.0 | | 5.0 | 0.0 | | 9.0 | 4.0 |
| | .016 | 0.0 | | | 7.0 | 5.0 | 4.0 | 3.0 | 4.0 | | 0.0 | | 2.0 | 0.0 | | 9.0 | 3.0 |
| 8 | .500 | 3.0 | | | 4.0 | 0.0 | 4.5 | 5.0 | 5.5 | | 4.0 | | 6.0 | 6.0 | | 9.0 | 0.0 |
| | .250 | 2.5 | | | 3.0 | 0.0 | 2.0 | 1.0 | 3.0 | | 2.0 | | 6.0 | 4.5* | | 9.0 | 0.0 |
| | .125 | 0.0 | | | 3.0 | 0.0 | 0.0 | 0.0 | 1.0 | | 0.0 | | 2.0 | 0.0 | | 5.5* | 0.0 |
| | .063 | 0.0 | | | 0.5 | 0.0 | 0.0 | 0.0 | 0.0 | | 0.0 | | 0.5 | 0.0 | | 4.5* | 0.0 |
| | .032 | 0.0 | | | 0.0 | 0.0 | 0.0 | 0.0 | 0.0 | | 0.0 | | 0.0 | 0.0 | | 2.0 | 0.0 |
| | .016 | 0.0 | | | 0.0 | 0.0 | 0.0 | 0.0 | 0.0 | | 0.0 | | 0.0 | 0.0 | | 1.0 | 0.0 |

TABLE I (Cont.)

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 9 | .500 | 7.0 | | | 8.0 | 9.0 | 9.0 | 9.0 | 6.0 | | 0.0 | | 7.0 | 0.0 | | 9.0 | 8.0 |
| | .250 | 2.0 | | | 7.0 | 9.0 | 3.0 | 4.0 | 0.0 | | 0.0 | | 4.0 | 0.0 | | 7.0 | 6.0 |
| | .125 | 0.0 | | | 6.0 | 7.0 | 1.0 | 2.0 | 0.0 | | 0.0 | | 1.0 | 0.0 | | | 3.0 |
| | .063 | 0.0 | | | 2.0 | 2.0 | 0.0 | 0.0 | 0.0 | | 0.0 | | 0.0 | 0.0 | | 5.0 | 1.0 |
| | .032 | 0.0 | | | 0.0 | 0.0 | 0.0 | 0.0 | 0.0 | | 0.0 | | 0.0 | 0.0 | | 0.0 | 0.0 |
| 10 | .500 | 9.0 | | | 6.0 | 0.0 | 5.0 | 9.0 | 6.0 | | 4.0 | | 7.0 | 4.0 | | 9.0 | 4.0 |
| | .250 | 2.0 | | | 4.0 | 0.0 | 5.0 | 4.0 | 4.0 | | 2.0 | | 6.0 | 2.0 | | 6.0 | 2.0 |
| | .125 | 0.0 | | | 4.0 | 0.0 | 3.0 | 4.0 | 2.0 | | 0.0 | | 5.0 | 1.0 | | 0.0 | 2.0 |
| | .063 | 0.0 | | | 1.0 | 0.0 | 1.0 | 1.0 | 0.0 | | 0.0 | | 3.0 | 0.0 | | 2.0 | 0.0 |
| | .032 | 0.0 | | | 1.0 | 0.0 | 0.0 | 0.0 | 0.0 | | 0.0 | | 1.0 | 0.0 | | | 0.0 |
| | .016 | 0.0 | | | 0.0 | 0.0 | 0.0 | 0.0 | 0.0 | | 0.0 | | 0.0 | 0.0 | | 2.0 | 0.0 |
| 11 | .500 | 9.0 | | | 9.0 | 9.0 | 9.0 | 9.0 | 9.0 | | 8.0 | | 8.0 | 9.0 | | 9.0 | 9.0 |
| | .250 | 9.0 | | | 9.0 | 9.0 | 9.0 | 9.0 | 9.0 | | 7.0 | | 8.0 | 9.0 | | 9.0 | 9.0 |
| | .125 | 2.0 | | | 9.0 | 9.0 | 9.0 | 9.0 | 8.0 | | 3.0 | | 7.0 | 4.0 | | 9.0 | 9.0 |
| | .063 | 1.0 | | | 9.0 | 9.0 | 9.0 | 9.0 | 7.0 | | 1.0 | | 3.0 | 1.0 | | 9.0 | 8.0 |
| | .032 | 0.0 | | | 7.0 | 7.0 | 8.0 | 3.0 | 3.0 | | 0.0 | | 3.0 | 0.0 | | 9.0 | 7.0 |
| | .016 | 0.0 | | | 4.0 | 2.0 | 2.0 | 0.0 | 0.0 | | 0.0 | | 0.0 | 0.0 | | 7.0 | 3.0 |
| 12 | .500 | 0.0 | | | 0.0 | 0.0 | 0.0 | 0.0 | 0.0 | | 0.0 | | 0.0 | 0.0 | | 7.0 | 0.0 |
| | .250 | 0.0 | | | 0.0 | 0.0 | 0.0 | 0.0 | 0.0 | | 0.0 | | 0.0 | 0.0 | | 7.0 | 0.0 |
| | .125 | 0.0 | | | 0.0 | 0.0 | 0.0 | 0.0 | 0.0 | | 0.0 | | 0.0 | 0.0 | | 7.0 | 0.0 |
| | .063 | 0.0 | | | 0.0 | 0.0 | 0.0 | 0.0 | 0.0 | | 0.0 | | 0.0 | 0.0 | | 7.0 | 0.0 |
| | .032 | 0.0 | | | 0.0 | 0.0 | 0.0 | 0.0 | 0.0 | | 0.0 | | 0.0 | 0.0 | | 7.0 | 0.0 |
| | .016 | 0.0 | | | 0.0 | 0.0 | 0.0 | 0.0 | 0.0 | | 0.0 | | 0.0 | 0.0 | | 0.0 | 0.0 |

TABLE I (Cont.)

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 13 | .500 | 3.0 | | | 7.0 | 0.0 | 6.0 | 6.0 | 7.0 | | 0.0 | | 8.0 | 0.0 | | 9.0 | 0.0 |
| | .250 | 2.0 | | | 6.0 | 0.0 | 4.0 | 2.0 | 2.0 | | 0.0 | | 7.0 | 0.0 | | 8.0 | 0.0 |
| | .125 | 0.0 | | | 7.0 | 0.0 | 4.0 | 4.0 | 0.0 | | 0.0 | | 7.0 | 0.0 | | 6.0 | 0.0 |
| | .063 | 0.0 | | | 2.0 | 0.0 | 2.0 | 2.0 | 0.0 | | 0.0 | | 5.0 | 0.0 | | 4.0 | 0.0 |
| | .032 | 0.0 | | | 2.0 | 0.0 | 0.0 | 0.0 | 0.0 | | 0.0 | | 4.0 | 0.0 | | 4.0 | 0.0 |
| | .016 | 0.0 | | | 0.0 | 0.0 | 0.0 | 0.0 | 0.0 | | 0.0 | | 2.0 | 0.0 | | 2.0 | 0.0 |
| 14 | .500 | 2.0 | | | 7.0 | 0.0 | 9.0 | 4.0 | 8.0 | | 6.0 | | 7.0 | 8.0 | | 9.0 | 4.0 |
| | .250 | 1.0 | | | 7.0 | 0.0 | 9.0 | 2.0 | 7.0 | | 4.0 | | 6.0 | 6.0 | | 9.0 | 1.0 |
| | .125 | 0.0 | | | 6.0 | 0.0 | 1.0 | 0.0 | 1.0 | | 0.0 | | 4.0 | | | 9.0 | 1.0 |
| | .063 | 0.0 | | | 1.0 | 0.0 | 0.0 | 0.0 | 0.0 | | 0.0 | | 1.0 | | | 7.0 | 0.0 |
| | .032 | 0.0 | | | 0.0 | 0.0 | 0.0 | 0.0 | 0.0 | | 0.0 | | 0.0 | | | 5.0 | 0.0 |
| | .016 | 0.0 | | | 0.0 | 0.0 | 0.0 | 0.0 | 0.0 | | 0.0 | | 0.0 | | | 5.0 | 0.0 |
| 15 | .500 | 8.0 | | | 2.0 | 2.0 | 4.0 | 0.0 | 0.0 | | 0.0 | | 2.0 | | | 9.0 | 7.0 |
| | .250 | 6.0 | | | 2.0 | 1.0 | 1.0 | 0.0 | 0.0 | | 0.0 | | 1.0 | | | 2.0 | 2.0 |
| | .125 | 4.0 | | | 1.0 | 0.0 | 0.0 | 0.0 | 0.0 | | 0.0 | | 0.0 | | | 2.0 | 0.0 |
| | .063 | 4.0 | | | 0.0 | 0.0 | 0.0 | 0.0 | 0.0 | | 0.0 | | 0.0 | | | 0.0 | 0.0 |
| | .032 | 1.0 | | | 0.0 | 0.0 | 0.0 | 0.0 | 0.0 | | 0.0 | | 0.0 | | | 0.0 | 0.0 |
| | .016 | 0.0 | | | 0.0 | 0.0 | 0.0 | 0.0 | 0.0 | | 0.0 | | 0.0 | | | 0.0 | 0.0 |
| 16 | .500 | 1.0 | | | 9.0 | 0.0 | 8.0 | 8.0 | 2.0 | | 0.0 | | 6.0 | | | 9.0 | 2.0 |
| | .250 | 0.0 | | | 6.0 | 0.0 | 9.0 | 4.0 | 1.0 | | 0.0 | | 4.0 | | | 9.0 | 1.0 |
| | .125 | 0.0 | | | 2.0 | 0.0 | 1.0 | 4.0 | 1.0 | | 0.0 | | 1.0 | | | 9.0 | 0.0 |
| | .063 | 0.0 | | | 0.0 | 0.0 | 0.0 | 0.0 | 0.0 | | 0.0 | | 0.0 | | | 7.0 | 0.0 |
| | .032 | 0.0 | | | 0.0 | 0.0 | 0.0 | 0.0 | 0.0 | | 0.0 | | 0.0 | | | 4.0 | 0.0 |
| | .016 | 0.0 | | | 0.0 | 0.0 | 0.0 | 0.0 | 0.0 | | 0.0 | | 0.0 | | | | 0.0 |

TABLE I (Cont.)

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 17 | .500 | 0.0 | | | 9.0 | 4.0 | 9.0 | 9.0 | 9.0 | | 4.0 | | 8.0 | | | 9.0 | 7.0 |
| | .250 | 0.0 | | | 9.0 | 3.0 | 9.0 | 9.0 | 9.0 | | 4.0 | | 8.0 | | | 9.0 | 7.0 |
| | .125 | 0.0 | | | 9.0 | 0.0 | 9.0 | 9.0 | 8.0 | | 1.0 | | 7.0 | | | 9.0 | 3.0 |
| | .063 | 0.0 | | | 7.0 | 0.0 | 1.0 | 8.0 | 2.0 | | 1.0 | | 4.0 | 0.0 | | 8.0 | 1.0 |
| | .032 | 0.0 | | | 5.0 | 0.0 | 1.0 | 0.0 | 2.0 | | 0.0 | | 0.0 | 0.0 | | | 0.0 |
| | .016 | 0.0 | | | 2.0 | 0.0 | 0.0 | 0.0 | 0.0 | | 0.0 | | 0.0 | 0.0 | | 4.0 | 0.0 |
| 18 | .500 | 9.0 | | | 9.0 | 9.0 | 9.0 | 9.0 | 5.0 | | 8.0 | | 9.0 | 8.0 | | 9.0 | 9.0 |
| | .250 | 4.0 | | | 4.0 | 9.0 | 9.0 | 7.0 | 2.0 | | 7.0 | | 9.0 | 4.0 | | 9.0 | 8.0 |
| | .125 | 1.0 | | | 3.0 | 8.0 | 9.0 | 2.0 | 2.0 | | | | 8.0 | 4.0 | | 8.0 | 8.0 |
| | .063 | 0.0 | | | 2.0 | 5.0 | 7.0 | 0.0 | 0.0 | | 0.0 | | 6.0 | 0.0 | | 6.0 | 3.0 |
| | .032 | 0.0 | | | 0.0 | 0.0 | 6.0 | 0.0 | 0.0 | | 0.0 | | 0.0 | 0.0 | | 6.0 | 3.0 |
| | .016 | 0.0 | | | 0.0 | 0.0 | 4.0 | 0.0 | 0.0 | | 0.0 | | 0.0 | 0.0 | | | 2.0 |
| 19 | .500 | 8.0 | | | 9.0 | 9.0 | 9.0 | 9.0 | 8.0 | | 7.0 | | 9.0 | 5.0 | | 9.0 | 9.0 |
| | .250 | 8.0 | | | 8.0 | 9.0 | 9.0 | 9.0 | 8.0 | | 7.0 | | 9.0 | 5.0 | | 9.0 | 9.0 |
| | .125 | 8.0 | | | 8.0 | 9.0 | 9.0 | 8.0 | 6.0 | | 6.0 | | 8.0 | 3.0 | | 9.0 | 9.0 |
| | .063 | 6.0 | | | 6.0 | 9.0 | 9.0 | 5.0 | 2.0 | | 5.0 | | 8.0 | 3.0 | | 9.0 | 7.0 |
| | .032 | 4.0 | | | 2.0 | 9.0 | 5.0 | 0.0 | 0.0 | | 3.0 | | 4.0 | 1.0 | | 9.0 | 8.0 |
| | .016 | 2.0 | | | 0.0 | 6.0 | 3.0 | 0.0 | 0.0 | | 1.0 | | 3.0 | 0.0 | | 7.0 | 6.0 |
| 20 | .500 | 9.0 | | | 2.0 | 9.0 | 9.0 | 9.0 | 8.0 | | 8.0 | | 8.0 | 8.0 | | 9.0 | 9.0 |
| | .250 | 9.0 | | | 2.0 | 9.0 | 9.0 | 3.0 | 7.0 | | 7.0 | | 9.0 | 7.0 | | 9.0 | 8.0 |
| | 125 | 3.0 | | | 1.0 | 8.0 | 9.0 | 7.0 | 3.0 | | 7.0 | | 6.0 | 4.0 | | 9.0 | 8.0 |
| | .063 | 1.0 | | | 0.0 | 7.0 | 6.0 | 1.0 | 1.0 | | 4.0 | | 5.0 | 2.0 | | 8.0 | 7.0 |
| | .032 | 0.0 | | | 0.0 | 5.0 | 2.0 | 0.0 | 0.0 | | 2.0 | | 0.0 | 1.0 | | 8.0 | 7.0 |
| | .016 | 0.0 | | | 0.0 | 0.0 | 0.0 | 0.0 | 0.0 | | 0.0 | | 0.0 | 0.0 | | 8.0 | 3.0 |

TABLE I (Cont.)

PRE-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 21 | .500 | 0.0 | | | 8.0 | 0.0 | 4.0 | 9.0 | 7.0 | | 0.0 | | 6.0 | 2.0 | | 9.0 | 0.0 |
| | .250 | 0.0 | | | 4.0 | 0.0 | 3.0 | 6.0 | 4.0 | | 0.0 | | 4.0 | 0.0 | | 8.0 | 0.0 |
| | .125 | 0.0 | | | 2.0 | 0.0 | 1.0 | 4.0 | 2.0 | | 0.0 | | 4.0 | 0.0 | | 8.0 | 0.0 |
| | .063 | 0.0 | | | 0.0 | 0.0 | 0.0 | 2.0 | 0.0 | | 0.0 | | 0.0 | 0.0 | | 4.0 | 0.0 |
| | .032 | 0.0 | | | 0.0 | 0.0 | 0.0 | 0.0 | 0.0 | | 0.0 | | 0.0 | 0.0 | | 2.0 | 0.0 |
| | .016 | 0.0 | | | 0.0 | 0.0 | 0.0 | 0.0 | 0.0 | | 0.0 | | 0.0 | 0.0 | | 2.0 | 0.0 |
| 22 | .500 | 9.0 | | | 4.0 | 9.0 | 9.0 | 9.0 | 8.0 | | 7.0 | | 8.0 | 9.0 | | 9.0 | 8.0 |
| | .250 | 9.0 | | | 1.0 | 9.0 | 9.0 | 9.0 | 7.0 | | 6.0 | | 8.0 | 7.0 | | 9.0 | 7.0 |
| | .125 | 6.0 | | | 1.0 | 7.0 | 9.0 | 4.0 | 7.0 | | 4.0 | | 6.0 | 7.0 | | 8.0 | 6.0 |
| | .063 | 2.0 | | | 0.0 | 4.0 | 8.0 | 4.0 | 2.0 | | 0.0 | | 4.0 | 2.0 | | 7.0 | 3.0 |
| | .032 | 0.0 | | | 0.0 | 2.0 | 8.0 | 0.0 | 0.0 | | 0.0 | | 2.0 | 2.0 | | 6.0 | 2.0 |
| | .016 | 0.0 | | | 0.0 | 0.0 | 2.0 | 0.0 | 0.0 | | 0.0 | | 2.0 | 0.0 | | 0.0 | 1.0 |
| 23 | 8.000 | 8.0 | 8.0 | 9.0 | 7.0 | 9.0 | 9.0 | 9.0 | 8.0 | 9.0 | 5.0 | 8.0 | 8.0 | | | | |
| | .500 | 0.0 | | 0.0 | 0.0 | 0.0 | 0.0 | 0.0 | 0.0 | | 0.0 | | 0.0 | 0.0 | 0.0 | 0.0 | 0.0 |
| | .250 | 0.0 | | 0.0 | 0.0 | 0.0 | 0.0 | 0.0 | 0.0 | | 0.0 | | 0.0 | 0.0 | 0.0 | 0.0 | 0.0 |
| | .125 | 0.0 | | 0.0 | 0.0 | 0.0 | 0.0 | 0.0 | 0.0 | | 0.0 | | 0.0 | 0.0 | 0.0 | 0.0 | 0.0 |
| | .063 | 0.0 | | 0.0 | 0.0 | 0.0 | 0.0 | 0.0 | 0.0 | | 0.0 | | 0.0 | 0.0 | 0.0 | 0.0 | 0.0 |
| | .032 | 0.0 | | 0.0 | 0.0 | 0.0 | 0.0 | 0.0 | 0.0 | | 0.0 | | 0.0 | 0.0 | 0.0 | 0.0 | 0.0 |
| | .016 | 0.0 | | 0.0 | 0.0 | 0.0 | 0.0 | 0.0 | 0.0 | | 0.0 | | 0.0 | 0.0 | 0.0 | 0.0 | 0.0 |

EXAMPLE 37

Postemergence herbicidal evaluation of test compounds

The postemergence herbicidal activity of the compounds of the present invention is determined by the following tests, wherein a variety of monocotyledonous and dicotyledonous plants are treated with test compounds dispersed in aqueous acetone mixtures. In the tests, seedling plants are grown in jiffy flats for about two weeks. The test compounds are dispersed in 50/50 acetone/water mixtures containing 0.5% TWEEN® 20, a polyoxyethylene sorbitan monolaurate surfactant of Atlas Chemical Industries, in sufficient quantities to provide the equivalent of about 0.16 kg to 8.0 kg per hectare of active compound when applied to the plants through a spray nozzle operating at 40 psig for a predetermined time. After spraying, the plants are placed on greenhouse benches and are cared for in the usual manner, commensurate with conventional greenhouse practices. From four to five weeks after treatment, the seedling plants, are examined and rated according to the rating system provided in Example 36 above. The data obtained are recorded in Table II below. The compounds evaluated are reported by compound number given in Example 36.

## TABLE II

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 1 | 1.000 | 0.0 | | 4.0 | 0.0 | 0.0 | 0.0 | 8.0 | 1.0 | | | | 2.0 | 0.0 | | 7.0 | 4.0 |
| | .500 | 0.0 | | 4.0 | 0.0 | 0.0 | 0.0 | 8.0 | 1.0 | | 0.0 | | 2.0 | 0.0 | | 4.0 | 3.0 |
| | .250 | 0.0 | | 3.0 | 0.0 | 0.0 | 0.0 | 6.0 | 0.0 | | 0.0 | | 0.0 | 0.0 | | 4.0 | 3.0 |
| | .125 | 0.0 | | 4.0 | 0.0 | 0.0 | 0.0 | 5.0 | 0.0 | | 0.0 | | 0.0 | 0.0 | | 4.0 | |
| 3 | 1.000 | 4.0 | | | 3.0 | 7.0 | 7.0 | 6.0 | 5.0 | | 6.0 | | 3.0 | 9.0 | | 8.0 | 4.0 |
| | .500 | 3.0 | | | 2.0 | 6.0 | 7.0 | 4.0 | 2.0 | | 5.0 | | 1.0 | 9.0 | | 3.0 | 3.0 |
| | .250 | 2.0 | | | 2.0 | 4.0 | 3.0 | 2.0 | 0.0 | | 0.0 | | 0.0 | 0.0 | | 1.0 | 2.0 |
| | .125 | 0.0 | | | 0.0 | 2.0 | 1.0 | 0.0 | 0.0 | | 0.0 | | 0.0 | 0.0 | | 0.0 | 0.0 |
| | .063 | 0.0 | | | 0.0 | 2.0 | 0.0 | 0.0 | 0.0 | | 0.0 | | 0.0 | 0.0 | | 0.0 | 0.0 |
| | .032 | 0.0 | | | 0.0 | 1.0 | 0.0 | 0.0 | 0.0 | | 0.0 | | 0.0 | 0.0 | | 0.0 | 0.0 |
| 4 | 8.000 | 9.0 | 9.0 | 9.0 | 9.0 | 9.0 | 9.0 | 9.0 | 9.0 | 9.0 | 9.0 | 9.0 | 9.0 | | | | |
| | 1.000 | 9.0 | | | 4.5 | 9.0 | 9.0 | 9.0 | 8.5 | | 9.0 | | 7.5 | 9.0 | | 9.0 | 9.0 |
| | .500 | 9.0 | | | 3.5 | 9.0 | 9.0 | 9.0 | 5.5 | | 7.5 | | 6.5 | 9.0 | | 9.0 | 9.0 |
| | .250 | 9.0 | | | 1.5 | 9.0 | 8.0 | 9.0 | 5.0 | | 5.5 | | 5.5• | 8.0 | | 9.0 | 9.0 |
| | .125 | 8.0 | | | 0.5 | 9.0 | 8.0 | 6.5 | 4.5 | | 3.0 | | 3.5 | 1.5 | | 6.0• | 9.0 |
| | .063 | 5.0 | | | 0.0 | 7.5 | 3.5 | 4.0 | 3.5 | | 1.0 | | 2.5 | 1.5 | | 6.0• | 8.5 |
| | .032 | 3.5 | | | 0.0 | 3.0 | 2.0 | 1.0 | 3.0 | | 0.5 | | 1.5 | 0.5 | | 9.0 | 7.5 |
| 5 | 1.000 | 9.0 | | | 9.0 | 9.0 | 8.0 | 9.0 | 9.0 | | 7.0 | | 8.0 | 7.0 | | 9.0 | 9.0 |
| | .750 | 9.0 | 6.0 | 9.0 | 7.0 | | 7.0 | 9.0 | 8.0 | | 7.0 | 7.0 | 4.0 | | | | 8.0 |
| | .500 | 9.0 | 6.0 | 8.0 | 8.0 | 9.0 | 8.0 | 8.5 | 8.0 | | 5.5 | 6.0 | 6.5 | 7.0 | | 9.0 | 8.5 |
| | .375 | 7.0 | 5.0 | 8.0 | 6.0 | | 6.0 | 8.0 | 7.0 | | 6.0 | 5.0 | 4.0 | | | | 8.0 |
| | .250 | 8.0 | 4.0 | 8.0 | 5.5 | 9.0 | 7.5 | 8.0 | 8.0 | | 5.0 | 4.0 | 5.5 | 5.0 | | 9.0 | 8.5 |
| | .188 | 6.0 | 4.0 | 8.0 | 3.0 | | 5.0 | 5.0 | 4.0 | | 4.0 | 3.0 | 3.0 | | | | 7.0 |
| | .125 | 7.5 | 4.0 | 8.0 | 3.5• | 8.0 | 5.5 | 4.0• | 5.0• | | 3.0 | 0.0 | 5 0 | 3.0 | | 9.0 | 8.0 |
| | .063 | 8.0 | | | 5.0 | 8.0 | 7.0 | 7.0 | 8.0 | | 3.0 | | 6.0 | 3.0 | | 3.0 | 8.0 |
| | .032 | 7.0 | | | 2.0 | 6.0 | 4.0 | 6.0 | 5.0 | | 3.0 | | 2.0 | 0.0 | | 8.0 | 5.0 |

TABLE II (Cont.)

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD [illegible] | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 6 | 1.000 | 0.0 | | | 0.0 | 0.0 | 0.0 | 0.0 | 2.0 | | 0.0 | | 0.0 | 0.0 | | 2.0 | 0.0 |
| | .500 | 0.0 | | | 0.0 | 0.0 | 0.0 | 0.0 | 1.0 | | 0.0 | | 0.0 | 0.0 | | 2.0 | 0.0 |
| | .250 | 0.0 | | | 0.0 | 0.0 | 0.0 | 0.0 | 0.0 | | 0.0 | | 0.0 | 0.0 | | 1.0 | 0.0 |
| | .125 | 0.0 | | | 0.0 | 0.0 | 0.0 | 0.0 | 0.0 | | 0.0 | | 0.0 | 0.0 | | 0.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| 7 | 8.000 | 9.0 | 9.0 | 9.0 | 9.0 | 9.0 | 9.0 | 9.0 | 8.0 | 9.0 | 9.0 | 9.0 | 9.0 | | | | |
| | 1.000 | 9.0 | | | 9.0 | 9.0 | 8.0 | 9.0 | 9.0 | | 7.0 | | 9.0 | 8.0 | | 9.0 | 9.0 |
| | .500 | 9.0 | | | 8.0 | 9.0 | 8.0 | 9.0 | 9.0 | | 6.0 | | 9.0 | 7.0 | | 9.0 | 9.0 |
| | .250 | 9.0 | | | 8.0 | 9.0 | 7.0 | 9.0 | 9.0 | | 6.0 | | 9.0 | 7.0 | | 9.0 | 9.0 |
| | .125 | 9.0 | | | 6.0 | 9.0 | 7.0 | 9.0 | 8.0 | | 4.0 | | 4.0 | 7.0 | | 9.0 | 9.0 |
| | .063 | 9.0 | | | 6.0 | 9.0 | 7.0 | 9.0 | 7.0 | | 0.0 | | 4.0 | 2.0 | | 8.0 | 9.0 |
| | .032 | 9.0 | | | 4.0 | 8.0 | 2.0 | 8.0 | 6.0 | | 0.0 | | 4.0 | 0.0 | | 6.0 | 9.0 |
| 8 | 1.000 | 0.0 | | | 0.0 | 0.0 | 0.0 | 0.0 | 0.0 | | 0.0 | | 2.0 | 0.0 | | 0.0 | 0.0 |
| | .500 | 0.0 | | | 0.0 | 0.0 | 0.0 | 0.0 | 0.0 | | 0.0 | | 1.0 | 0.0 | | 0.0 | 0.0 |
| | .250 | 0.0 | | | 0.0 | 0.0 | 0.0 | 0.0 | 0.0 | | 0.0 | | 0.0 | 0.0 | | 0.0 | 0.0 |
| | .125 | 0.0 | | | 0.0 | 0.0 | 0.0 | 0.0 | 0.0 | | 0.0 | | 0.0 | 0.0 | | 0.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| 9 | 1.000 | 6.0 | | | 4.0 | 9.0 | 2.0 | 7.0 | 6.0 | | 0.0 | | 2.0 | 0.0 | | 4.0 | 6.0 |
| | .500 | 3.0 | | | 2.0 | 9.0 | 1.0 | 6.0 | 4.0 | | 0.0 | | 2.0 | 0.0 | | 4.0 | 2.0 |
| | .250 | 1.0 | | | 0.0 | 6.0 | 0.0 | 6.0 | 2.0 | | 0.0 | | 0.0 | 0.0 | | 4.0 | 1.0 |
| | .125 | 0.0 | | | 0.0 | 2.0 | 0.0 | 2.0 | 0.0 | | 0.0 | | 0.0 | 0.0 | | 2.0 | 1.0 |
| | .063 | 0.0 | | | 0.0 | 2.0 | 0.0 | 0.0 | 0.0 | | 0.0 | | 0.0 | 0.0 | | 1.0 | 1.0 |
| | .032 | 0.0 | | | 0.0 | 1.0 | 0.0 | 0.0 | 0.0 | | 0.0 | | 0.0 | 0.0 | | 0.0 | 0.0 |

TABLE II (Cont.)

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | WRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 11 | 1.000 | 9.0 | | | 7.0 | 9.0 | 9.0 | 9.0 | 9.0 | | 6.0 | | 8.0 | 8.0 | | 9.0 | 9.0 |
| | .500 | 9.0 | | | 7.0 | 9.0 | 9.0 | 9.0 | 9.0 | | 3.0 | | 7.0 | 6.0 | | 9.0 | 9.0 |
| | .250 | 9.0 | | | 7.0 | 9.0 | 9.0 | 9.0 | 9.0 | | 1.0 | | 7.0 | 4.0 | | 9.0 | 9.0 |
| | .125 | 7.0 | | | 5.0 | 9.0 | 2.0 | 9.0 | 7.0 | | 0.0 | | 4.0 | 0.0 | | 9.0 | 9.0 |
| | .063 | 7.0 | | | 4.0 | 8.0 | 0.0 | 8.0 | 6.0 | | 0.0 | | 2.0 | 0.0 | | 9.0 | 7.0 |
| | .032 | 4.0 | | | 4.0 | 2.0 | 0.0 | 4.0 | 0.0 | | 0.0 | | 2.0 | 0.0 | | 4.0 | 4.0 |
| 12 | 1.000 | 0.0 | | | 0.0 | 0.0 | 0.0 | 0.0 | 0.0 | | 0.0 | | 0.0 | 0.0 | | 0.0 | 0.0 |
| | .500 | 0.0 | | | 0.0 | 0.0 | 0.0 | 0.0 | 0.0 | | 0.0 | | 0.0 | 0.0 | | 0.0 | 0.0 |
| | .250 | 0.0 | | | 0.0 | 0.0 | 0.0 | 0.0 | 0.0 | | 0.0 | | 0.0 | 0.0 | | 0.0 | 0.0 |
| | .125 | 0.0 | | | 0.0 | 0.0 | 0.0 | 0.0 | 0.0 | | 0.0 | | 0.0 | 0.0 | | 0.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| 13 | 1.000 | 4.0 | | | 6.0 | 2.0 | 2.0 | 8.0 | 4.0 | | 0.0 | | 3.0 | | | 9.0 | 6.0 |
| | .500 | 2.0 | | | 6.0 | 0.0 | 0.0 | 3.0 | 1.0 | | 0.0 | | 2.0 | | | 4.0 | 3.0 |
| | .250 | 0.0 | | | 0.0 | 0.0 | 0.0 | 1.0 | 0.0 | | 0.0 | | 0.0 | | | 4.0 | 2.0 |
| | .125 | 0.0 | | | 0.0 | 0.0 | 0.0 | 0.0 | 0.0 | | 0.0 | | 0.0 | | | 2.0 | 2.0 |
| | .063 | 0.0 | | | 0.0 | 0.0 | 0.0 | 0.0 | 0.0 | | 0.0 | | 0.0 | | | 2.0 | 0.0 |
| | .032 | 0.0 | | | 0.0 | 0.0 | 0.0 | 0.0 | 0.0 | | 0.0 | | 0.0 | | | 0.0 | 0.0 |
| 14 | 1.000 | 7.0 | | | 6.0 | 8.0 | 7.0 | 8.0 | 7.0 | | 3.0 | | 6.0 | 6.0 | | 9.0 | 8.0 |
| | .500 | 6.0 | | | 5.0 | 5.0 | 5.0 | 9.0 | 7.0 | | 3.0 | | 7.0 | 7.0 | | 9.0 | 7.0 |
| | .250 | 4.0 | | | 0.0 | 2.0 | 3.0 | 3.0 | 5.0 | | 2.0 | | 3.0 | 4.0 | | 7.0 | 3.0 |
| | .125 | 3.0 | | | 0.0 | 0.0 | 0.0 | 0.0 | 0.0 | | 0.0 | | 0.0 | 0.0 | | 7.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |

TABLE II (Cont.)

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 15 | 1.000 | 5.0 | | | 0.0 | 0.0 | 0.0 | 3.0 | 0.0 | | 0.0 | | 3.0 | 0.0 | | 0.0 | 0.0 |
| | .500 | 0.0 | | | 0.0 | 0.0 | 0.0 | 0.0 | 0.0 | | 0.0 | | 2.0 | 0.0 | | 0.0 | 0.0 |
| | .250 | 0.0 | | | 0.0 | 0.0 | 0.0 | 0.0 | 0.0 | | 0.0 | | 0.0 | 0.0 | | 0.0 | 0.0 |
| | .125 | 0.0 | | | 0.0 | 0.0 | 0.0 | 0.0 | 0.0 | | 0.0 | | 0.0 | 0.0 | | 0.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| 16 | 1.000 | 0.0 | | | 0.0 | 0.0 | 0.0 | 0.0 | 0.0 | | 0.0 | | 0.0 | 0.0 | | 0.0 | 0.0 |
| | .500 | 0.0 | | | 0.0 | 0.0 | 0.0 | 0.0 | 0.0 | | 0.0 | | 0.0 | 0.0 | | 0.0 | 0.0 |
| | .250 | 0.0 | | | 0.0 | 0.0 | 0.0 | 0.0 | 0.0 | | 0.0 | | 0.0 | 0.0 | | 0.0 | 0.0 |
| | .125 | 0.0 | | | 0.0 | 0.0 | 0.0 | 0.0 | 0.0 | | 0.0 | | 0.0 | 0.0 | | 0.0 | 0.0 |
| | .063 | 0.0 | | | 0.0 | 0.0 | 0.0 | 0.0 | 0.0 | | 0.0 | | 0.0 | 0.0 | | 0.0 | 0.0 |
| | .032 | 0.0 | | | 0.0 | 0.0 | 0.0 | 0.0 | 0.0 | | 0.0 | | 0.0 | 0.0 | | 0.0 | 0.0 |
| 17 | 1.000 | 9.0 | | | 9.0 | 8.0 | 7.0 | 9.0 | 8.0 | | 3.0 | | 9.0 | 4.0 | | 9.0 | 7.0 |
| | .500 | 7.0 | | | 8.0 | 6.0 | 3.0 | 9.0 | 8.0 | | 2.0 | | 8.0 | 4.0 | | 9.0 | 6.0 |
| | .250 | 6.0 | | | 8.0 | 3.0 | 0.0 | 8.0 | 7.0 | | 0.0 | | 8.0 | 4.0 | | 9.0 | 5.0 |
| | .125 | 3.0 | | | 6.0 | 0.0 | 0.0 | 7.0 | 8.0 | | 0.0 | | 7.0 | 3.0 | | 8.0 | 5.0 |
| | .063 | 0.0 | | | 3.0 | 0.0 | 0.0 | 5.0 | 8.0 | | 0.0 | | 3.0 | 0.0 | | 4.0 | 3.0 |
| | .032 | 0.0 | | | 0.0 | 0.0 | 0.0 | 5.0 | 5.0 | | 0.0 | | 0.0 | 0.0 | | 0.0 | 0.0 |
| 18 | 1.000 | 2.0 | | | 5.0 | 4.0 | 7.0 | 6.0 | 0.0 | | 2.0 | | 7.0 | | | 9.0 | 0.0 |

TABLE II (Cont.)

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 18 | .500 | 1.0 | | | 5.0 | 3.0 | 7.0 | 6.0 | 0.0 | | 1.0 | | 7.0 | | | 9.0 | 0.0 |
| | .250 | 0.0 | | | 2.0 | 1.0 | 3.0 | 2.0 | 0.0 | | 0.0 | | 3.0 | 0.0 | | 8.0 | 0.0 |
| | .125 | 0.0 | | | 1.0 | 0.0 | 2.0 | 0.0 | 0.0 | | 0.0 | | 1.0 | 0.0 | | 8.0 | 0.0 |
| | .063 | 0.0 | | | 0.0 | 0.0 | 0.0 | 0.0 | 0.0 | | 0.0 | | 0.0 | 0.0 | | 6.0 | 0.0 |
| | .032 | 0.0 | | | 0.0 | 0.0 | 0.0 | 0.0 | 0.0 | | 0.0 | | 0.0 | 0.0 | | 5.0 | 0.0 |
| 19 | 1.000 | 9.0 | | | 8.0 | 9.0 | 9.0 | 9.0 | 9.0 | | 9.0 | | 9.0 | 8.0 | | 9.0 | 9.0 |
| | .500 | 9.0 | | | 7.0 | 9.0 | 9.0 | 9.0 | 8.0 | | 7.0 | | 9.0 | 7.0 | | 9.0 | 9.0 |
| | .250 | 9.0 | | | 6.0 | 9.0 | 9.0 | 9.0 | 8.0 | | 7.0 | | 9.0 | 7.0 | | 9.0 | 9.0 |
| | .125 | 9.0 | | | 5.0 | 9.0 | 8.0 | 9.0 | 6.0 | | 5.0 | | 6.0 | 5.0 | | 9.0 | 9.0 |
| | .063 | 9.0 | | | 2.0 | 9.0 | 8.0 | 9.0 | 2.0 | | 1.0 | | 2.0 | 3.0 | | 9.0 | 9.0 |
| | .032 | 5.0 | | | 0.0 | 9.0 | 7.0 | | 0.0 | | 0.0 | | 0.0 | 1.0 | | 9.0 | 9.0 |
| 20 | 1.000 | 9.0 | | | 0.0 | 9.0 | 9.0 | 9.0 | 5.0 | | 7.0 | | 9.0 | 9.0 | | 9.0 | 9.0 |
| | .500 | 9.0 | | | 0.0 | 9.0 | 9.0 | 5.0 | 4.0 | | 3.0 | | 9.0 | 8.0 | | 9.0 | 9.0 |
| | .250 | 9.0 | | | 0.0 | 9.0 | 9.0 | | 1.0 | | 1.0 | | 8.0 | 6.0 | | 8.0 | 9.0 |
| | .125 | 8.0 | | | 0.0 | 8.0 | 7.0 | 0.0 | 0.0 | | 1.0 | | 3.0 | 5.0 | | 8.0 | 9.0 |
| | .063 | 2.0 | | | 0.0 | 7.0 | 7.0 | 0.0 | 0.0 | | 0.0 | | 0.0 | 1.0 | | 7.0 | 9.0 |
| | .032 | 0.0 | | | 0.0 | 3.0 | 2.0 | 0.0 | 0.0 | | 0.0 | | 0.0 | 0.0 | | 7.0 | 7.0 |
| 21 | 1.000 | 9.0 | | | 9.0 | 9.0 | 7.0 | 9.0 | 9.0 | | 7.0 | | 9.0 | 6.0 | | 9.0 | 8.0 |
| | .500 | 9.0 | | | 7.0 | 9.0 | 6.0 | 8.0 | 9.0 | | 7.0 | | 9.0 | 3.0 | | 9.0 | 6.0 |
| | .250 | 9.0 | | | 7.0 | 8.0 | 7.0 | 7.0 | 7.0 | | 6.0 | | 7.0 | 1.0 | | 8.0 | 3.0 |
| | .125 | 5.0 | | | 2.0 | 8.0 | 3.0 | 7.0 | 4.0 | | 2.0 | | 6.0 | 0.0 | | 5.0 | 1.0 |
| | .063 | 2.0 | | | 1.0 | 4.0 | 1.0 | 6.0 | 0.0 | | 1.0 | | 2.0 | 0.0 | | 2.0 | 0.0 |
| | .032 | 0.0 | | | 0.0 | 2.0 | 0.0 | 2.0 | 0.0 | | 0.0 | | 1.0 | 0.0 | | 2.0 | 0.0 |

TABLE II (Cont.)

POST-EMERGENCE TESTS -- RATES IN KG/HA

| Compound No. | RATE | BARNY ARDGR | LARGE CRAB | GREEN FOX | P NUT SEDGE | WILD OATS | QUACK GRASS | FLD B INDWD | MRNGL RY SP | WILD MUSTD | RAGWE ED | PRIKY SIDA | VELVE TLEAF | MATRI CARIA | S BAR LY LA | SUGAR BEETS | CORN FIELD |
|---|---|---|---|---|---|---|---|---|---|---|---|---|---|---|---|---|---|
| 23 | 8.000 | 0.0 | 0.0 | 2.0 | 0.0 | 6.0 | 0.0 | 0.0 | 3.0 | 0.0 | 0.0 | 0.0 | 0.0 | | | | |
| | 1.000 | 0.0 | | 0.0 | 0.0 | 0.0 | 0.0 | 0.0 | 0.0 | | 0.0 | | 0.0 | 0.0 | 0.0 | 0.0 | 0.0 |
| | .500 | 0.0 | | 0.0 | 0.0 | 0.0 | 0.0 | 0.0 | 0.0 | | 0.0 | | 0.0 | 0.0 | 0.0 | 0.0 | 0.0 |
| | .250 | 0.0 | | 0.0 | 0.0 | 0.0 | 0.0 | 0.0 | 0.0 | | 0.0 | | 0.0 | 0.0 | 0.0 | 0.0 | 0.0 |
| | .125 | 0.0 | | 0.0 | 0.0 | 0.0 | 0.0 | 0.0 | 0.0 | | 0.0 | | 0.0 | 0.0 | 0.0 | 0.0 | 0.0 |
| | .063 | 0.0 | | 0.0 | 0.0 | 0.0 | 0.0 | 0.0 | 0.0 | | 0.0 | | 0.0 | 0.0 | 0.0 | 0.0 | 0.0 |
| | .032 | 0.0 | | 0.0 | 0.0 | 0.0 | 0.0 | 0.0 | 0.0 | | 0.0 | | 0.0 | 0.0 | 0.0 | 0.0 | 0.0 |
| 24 | 1.000 | 0.0 | | | 0.0 | 0.0 | 0.0 | 7.0 | 4.0 | | 0.0 | | 0.0 | 0.0 | | 0.0 | 2.0 |
| | .500 | 0.0 | | | 0.0 | 0.0 | 0.0 | 6.0 | 2.0 | | 0.0 | | 0.0 | 0.0 | | 0.0 | 2.0 |
| | .250 | 0.0 | | | 0.0 | 0.0 | 0.0 | 2.0 | 1.0 | | 0.0 | | 0.0 | 0.0 | | 0.0 | 2.0 |
| | .125 | 0.0 | | | 0.0 | 0.0 | 0.0 | 0.0 | 0.0 | | 0.0 | | 0.0 | 0.0 | | 0.0 | 1.0 |
| | .063 | 0.0 | | | 0.0 | 0.0 | 0.0 | 0.0 | 0.0 | | 0.0 | | 0.0 | 0.0 | | 0.0 | 1.0 |
| | .032 | 0.0 | | | 0.0 | 0.0 | 0.0 | 0.0 | 0.0 | | 0.0 | | 0.0 | 0.0 | | 0.0 | 0.0 |
| 25 | 1.000 | 4.0 | | | 7.0 | 8.0 | 9.0 | 9.0 | 2.0 | | 2.0 | | 7.0 | 2.0 | | 6.0 | 8.0 |
| | .500 | 2.0 | | | 6.0 | 4.0 | 7.0 | 9.0 | 1.0 | | 1.0 | | 7.0 | 1.0 | | 6.0 | 8.0 |
| | .250 | 0.0 | | | 4.0 | 3.0 | 3.0 | 7.0 | 0.0 | | 0.0 | | 3.0 | 0.0 | | 4.0 | 4.0 |
| | .125 | 0.0 | | | 2.0 | 1.0 | 2.0 | 6.0 | 0.0 | | 0.0 | | 2.0 | 0.0 | | 2.0 | 3.0 |

## Claims

**Claims for the following Contracting States : BE, CH, LI, DE, FR, GB, GR, IT, LU, NL, SE**

**1.** A compound having the structure:

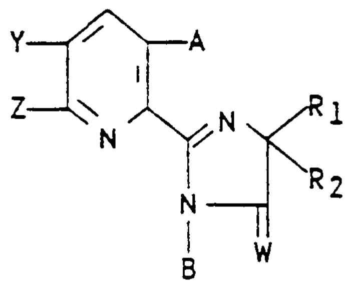

( I )

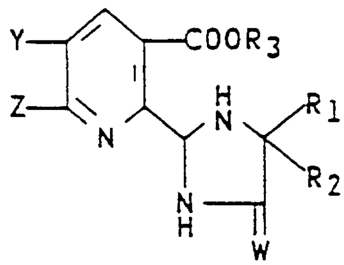

( II )

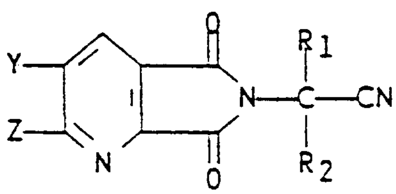

( III )

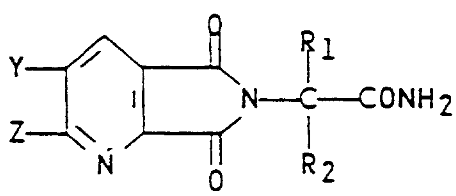

( IV )

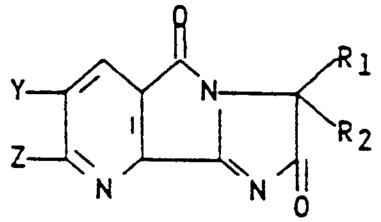

(V)

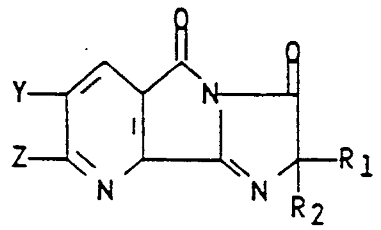

(VI)

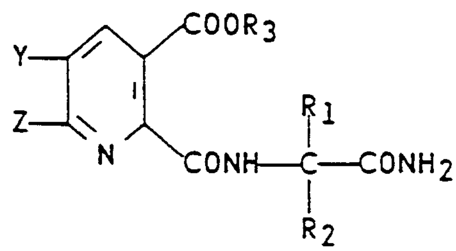

(VII)

wherein

- $R_1$ is $C_1$-$C_4$ alkyl;
- $R_2$ is $C_1$-$C_4$ alkyl;
- A is $COOR_3$, $CONHR_6$, CHO, $CH_2OH$, $COCH_3$, $COC_6H_5$, CN, $CH_3$, CH = NOH, $CH_2COOH$, CONHOH, $CH_2CH_2COOH$, $CHR_8OH$,

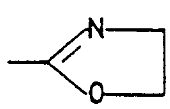

or

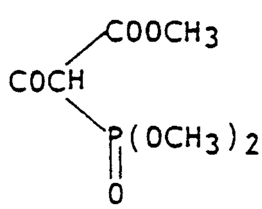

;

- $R_3$ is hydrogen,

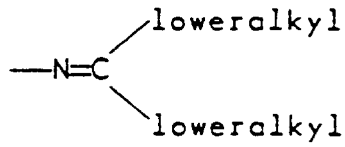

$C_1$-$C_{12}$ alkyl optionally substituted with one of the following groups: $C_1$-$C_3$ alkoxy, $C_1$-$C_3$ alkylthio, halogen, hydroxyl, $C_3$-$C_6$ cycloalkyl, benzyloxy, fury], phenyl, halophenyl, loweralkylphenyl, loweralkoxyphenyl, nitrophenyl, carboxyl, loweralkoxycarbonyl, cyano or triloweralkylammonium halide;

$C_3$-$C_{12}$ alkenyl optionally substituted with one of the following groups: $C_1$-$C_3$ alkoxy, phenyl, halogen or loweralkoxycarbonyl or with two $C_1$-$C_3$ alkoxy groups or two halogen groups;

$C_3$-$C_6$ cycloalkyl optionally substituted with one or two $C_1$-$C_3$ alkyl groups;

$C_3$-$C_{16}$ alkynyl optionally substituted with one or two $C_1$-$C_3$ alkyl groups; or a cation;

$R_6$ is hydrogen, hydroxyl, $C_3$-alkenyl, $C_3$-alkynyl or $C_1$-$C_4$ alkyl optionally substituted with one hydroxyl or one chlorine group;

B is H, $COR_4$ or $SO_2R_5$, provided that when B is $COR_4$ or $SO_2R_5$; A is $COOR_3$ in which $R_3$ is other than H, or a cation, $CH_3$ or CN; W is O; and Y and Z are not alkylamino, hydroxyl, or hydroxyloweralkyl;

$R_4$ is $C_1$-$C_{11}$ alkyl, chloromethyl or phenyl optionally substituted with one chloro, one nitro or one methoxy group;

$R_5$ is $C_1$-$C_4$ alkyl or phenyl optionally substituted with one methyl group;

W is O;

$R_8$ is $C_1$-$C_4$ alkyl or phenyl;

Y and Z are each hydrogen, halogen, $C_1$-$C_6$ alkyl, $C_1$-$C_4$ hydroxyloweralkyl, $C_1$-$C_6$ alkoxy, $C_1$-$C_4$ alkylthio, phenoxy, $C_1$-$C_4$ haloalkyl, nitro, cyano, $C_1$-$C_4$ alkylamino, formyl, di-$C_1$-$C_4$ loweralkylamino, $C_1$-$C_4$ alkylsulfonyl, difluoromethoxy, trifluoromethoxy, 1,1,2,2-tetrafluoroethoxy, phenyl optionally substituted with one $C_1$-$C_4$ alkyl, $C_1$-$C_4$ alkoxy or halogen;

$C_3$-$C_8$ straight or branched alkenyloxy optionally substituted with one to three halogens;

$C_3$-$C_8$ straight or branched alkynyloxy optionally substituted with one to three halogens;

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkylcarbonyloxy optionally substituted with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-fury] optionally substituted with halogen or $C_1$-$C_3$ alkyl;

2(or 3)-thieny] optionally substituted with halogen or $C_1$-$C_3$ alkyl;

N-methyl-2(or 3)-pyrrolyl;

hydroxy;

amino optionally substituted with $C_1$-$C_4$ alkoxy or $C_1$-$C_2$ alkyl and $C_1$-$C_4$ alkoxy;

2(or 4)-pyridyloxy optionally substituted with $C_1$-$C_4$ alkyl, trifluoromethyl or 1 or 2 halogens;

$C_1$-$C_4$ mono or dialkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

Provided that at least one of Y and Z is:

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-fury] optionally substituted with halogen or $C_1$-$C_3$ alkyl;

2(or 3)-thieny] optionally substituted with halogen or $C_1$-$C_3$ alkyl;

N-methy]-2-(or 3)-pyrrolyl;

hydroxy;

amino optionally substituted with $C_1$-$C_4$ alkoxy, or $C_1$-$C_2$ alkyl and $C_1$-$C_4$ alkoxy; or

2(or 4)-pyridyloxy optionally substituted by $C_1$-$C_4$ alkyl, trifluoromethyl or 1 to 2 halogens; and

$C_1$-$C_4$ mono or dialkylaminocarbony]amino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

Provided also that when Y is hydroxy, Z cannot be hydrogen;

the N-oxides thereof, provided that $R_3$ cannot be unsaturated alkyl and Y and Z cannot be alkylthioalkyl, alkylaminoalkyl, dialkylaminoalkyl, trialkylammoniumalkyl, amino, 2(or 4)-pyridyloxy, alkoxyamino or unsaturated alkyl;

the optical isomers thereof when $R_1$ and $R_2$ are not the same;

the tautomers and geometric isomers thereof; and

the acid addition salts thereof except when $R_3$ is a salt forming cation.

**2.** A compound according to claim 1 having the structure:

(I)

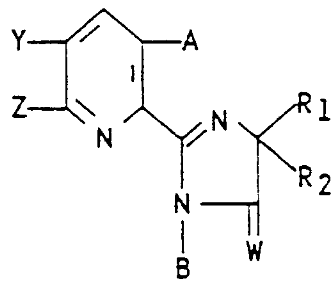

wherein A, B, W, $R_1$, $R_2$, Y and Z are as described in said claim 1.

**3.** A compound according to claim 2 having the structure:

(I)

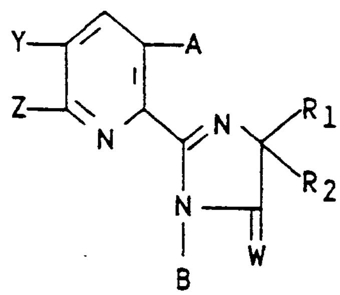

wherein A, B, W, $R_1$ and $R_2$ are as described in said claim 1, one of Y and Z represents hydrogen or methyl and the other of Y and Z represents

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-fury] optionally substituted with halogen or $C_1$-$C_3$ alkyl;

2(or 3)-thienyl optionally substituted with halogen or $C_1$-$C_3$ alkyl;

N-methyl-2(or 3)-pyrrolyl;

hydroxy;

amino optionally substituted with $C_1$-$C_4$ alkoxy or $C_1$-$C_2$ alkyl and $C_1$-$C_4$ alkoxy;

2(or 4)-pyridyloxy optionally substituted with $C_1$-$C_4$ alkyl, trifluoromethyl or 1 or 2 halogens;

$C_1$-$C_4$ mono or dialkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

the N-oxides thereof, provided that $R_3$ cannot be unsaturated alkyl;

the optical isomers thereof when $R_1$ and $R_2$ are not the same;

the tautomers and geometric isomers thereof; and

the acid addition salts thereof except when $R_3$ is a salt forming cations.

4. A compound according to Claim 2, 5-isopropenyl-2-(4-isopropyl-4-methyl-5-oxo-2-imidazolin-2-yl) nicotinic acid and the salts and esters thereof.

5. The compound according to Claim 2, namely 2-(4-isopropyl-4-methyl-5-oxo-2- imidazolin-2-yl)-5-(methoxymethyl)nicotinic acid or the esters or salts thereof.

6. A compound according to Claim 2, 2-(4-isopropyl-4-methyl-5-oxo-2-imidazolin-2-yl)-5-styrylnicotinic acid, 2-(4-isopropyl-4-methyl-5-oxo-2-imidazolin-2-yl)-5-propenylnicotinic acid, 5-acetyl-2-(4-isopropyl-4-methyl-5-oxo-2-imidazolin-2-yl)nicotinic acid, 5-acetyl-2-(4-isopropyl-4-methyl-5-oxo-2-imidazolin-2-yl)-6-methyl nicotinic acid, 2-(4-isopropyl-4-methyl-5-oxo-2-imidazolin-2-yl)-5-[(methylthio)methyl]nicotinic acid, 6-(2-furyl)-2-(4-isopropyl-4-methyl-5-oxo-2-imidazolin-2-yl)nicotinic acid, 2-(4-isopropyl-4-methyl-5-oxo-2-imidazolin-2-yl)-5-(2-thienyl)nicotinic acid, 2-(4-isopropyl-4-methyl-5-oxo-2-imidazolin-2-yl)-6-(2-thienyl)nicotinic acid, 2-(4-isopropyl-4-methyl-5-oxo-2-imidazolin-2-yl)-5-(3-thienyl)nicotinic acid, 1,6-dihydro-2-(4-isopropyl-4-methyl-5-oxo-2-imidazolin-2-yl)6-oxynicotinic acid and 2-(4-isopropyl-4-methyl-5-oxo-2-imidazolin-2-yl)-6[(methylthio)methyl]nicotinic acid and the salts and esters thereof.

7. A compound according to Claim 2, methyl 2-(1-acetyl-4-isopropyl-4-methyl-5-oxo-2-imidazolin-2-yl)-6-(hydroxymethyl)nicotinate acetate(ester), and isopropylammonium 6-(2-furyl)-2-(4-isopropyl-4-methyl-5-oxo-2-imidazolin-2-yl)nicotinate.

8. A compound according to Claim 1 having the structure:

(III)

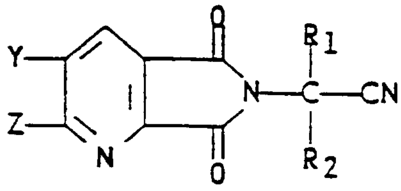

wherein $R_1$, $R_2$, Y and Z are as described in said Claim 1.

**9.** A compound according to Claim 1 having the structure:

(IV) 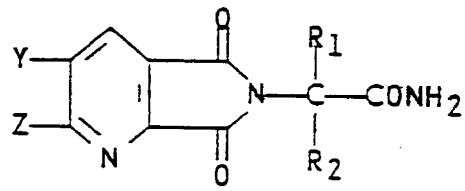

wherein $R_1$, $R_2$, Y and Z are as described in said Claim 1.

**10.** A compound according to Claim 1 having the structure:

(V) 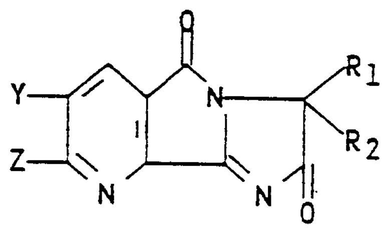

wherein $R_1$, $R_2$, Y and Z are as described in said Claim 1.

**11.** A compound according to Claim 1 having the structure:

(VI) 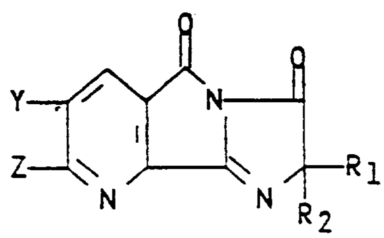

wherein $R_1$, $R_2$, Y and Z are as described in said Claim 1.

**12.** A compound according to Claim 1 having the structure:

(VII) 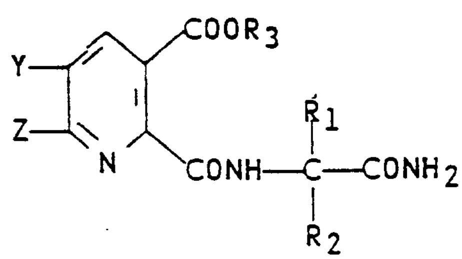

wherein $R_1$, $R_2$, $R_3$, Y and Z are as described in said Claim 1.

**13.** A method for the control of undesirable plant species comprising: applying to the foliage of said plants or to soil or water containing seeds or other propagating organs thereof, a herbicidally effective amount of a compound having the structure:

I. 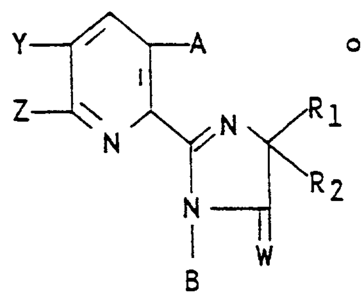 or II. 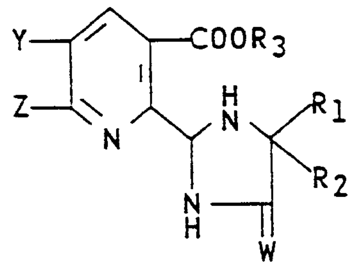

wherein

R1 is $C_1$-$C_4$ alkyl;

R2 is $C_1$-$C_4$ alkyl,

A is $COOR_3$, $CONHR_6$, CHO, $CH_2OH$, $COCH_3$, $COC_6H_5$, CN, $CH_3$, CH = NOH, $CH_2COOH$, CONHOH, $CH_2CH_2COOH$, $CHR_8OH$,

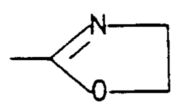 or 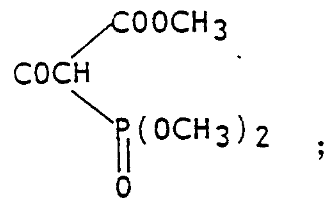 ;

R3 is hydrogen,

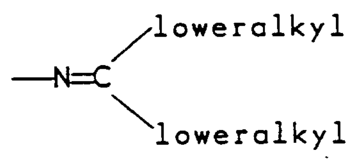

$C_1$-$C_{12}$ alkyl optionally substituted with one of the following groups: $C_1$-$C_3$ alkoxy, $C_1$-$C_3$ alkylthio, halogen, hydroxyl, $C_3$-$C_6$ cycloalkyl, benzyloxy, fury], phenyl, halophenyl, loweralkylphenyl, loweralkoxyphenyl, nitrophenyl, carboxyl, loweralkoxycarbonyl, cyano or triloweralkylammonium halide;

$C_3$-$C_{12}$ alkenyl optionally substituted with one of the following groups: $C_1$-$C_3$ alkoxy, phenyl, halogen or loweralkoxycarbonyl or with two $C_1$-$C_3$ alkoxy groups or two halogen groups;

$C_3$-$C_6$ cycloalkyl optionally substituted with one or two $C_1$-$C_3$ alkyl groups;

$C_3$-$C_{16}$ alkynyl optionally substituted with one or two $C_1$-$C_3$ alkyl groups; or a cation;

R6 is hydrogen, hydroxyl, $C_3$-alkenyl, $C_3$-alkynyl or $C_1$-$C_4$ alkyl optionally substituted with one hydroxyl or one chlorine group;

B is H, $COR_4$ or $SO_2R_5$, provided that when B is $COR_4$ or $SO_2R_5$; A is $COOR_3$ in which $R_3$ is other than H, or a cation, $CH_3$ or CN; W is O; and Y and Z are not alkylamino, hydroxyl, or hydroxyloweralkyl;

R4 is $C_1$-$C_{11}$ alkyl, chloromethyl or phenyl optionally substituted with one chloro, one nitro or one methoxy group;

R5 is $C_1$-$C_4$ alkyl or phenyl optionally substituted with one methyl group;

W is O;

R8 is $C_1$-$C_4$-alkyl or phenyl;

Y and Z are each hydrogen, halogen, $C_1$-$C_6$ alkyl, $C_1$-$C_4$ hydroxyloweralkyl, $C_1$-$C_6$ alkoxy, $C_1$-$C_4$ alkylthio, formyl, phenoxy, $C_1$-$C_4$ haloalkyl, nitro, cyano, $C_1$-$C_4$ alkylamino, di-$C_1$-$C_4$-

loweralkylamino, $C_1$-$C_4$ alkylsulfonyl, difluoromethoxy, trifluoromethoxy, 1,1,2,2-tetrafluoroethoxy, phenyl optionally substituted with one $C_1$-$C_4$ alkyl, $C_1$-$C_4$ alkoxy or halogen;

$C_3$-$C_8$ straight or branched alkenyloxy optionally substituted with one to three halogens;

$C_3$-$C_8$ straight or branched alkynyloxy optionally substituted with one to three halogens;

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen iwth $C_1$-$C_2$ alkyl or $C_1$-$C_4$ alkoxy and on carbon with $C_1$-$C_4$ alkoxy or 1 to 3 halogens;

$C_1$-$C_4$ alkylcarbonyloxy optionally substituted with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-furyl optionally substituted with halogen or $C_1$-$C_3$ alkyl;

2(or 3)-thienyl optionally substituted with halogen or $C_1$-$C_3$ alkyl;

N-methyl-2(or 3)-pyrrolyl;

hydroxy;

amino optionally substituted with $C_1$-$C_4$ alkoxy or $C_1$-$C_2$ alkyl and $C_1$-$C_4$ alkoxy;

2(or 4)-pyridyloxy optionally substituted with $C_1$-$C_4$ alkyl, trifluoromethyl or 1 or 2 halogens;

$C_1$-$C_4$ mono or dialkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

Provided that at least one of Y and Z is:

$C_2$-$C_6$ straight or branched alkenyl optionally substituted with phenyl, $C_1$-$C_4$ alkoxy or 1 to 3 halogens;

$C_1$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-furyl optionally substituted with halogen or $C_1$-$C_3$ alkyl;

2(or 3)-thienyl optionally substituted with halogen or $C_1$-$C_3$ alkyl;

N-methyl-2-(or 3)-pyrrolyl;

hydroxy;

amino optionally substituted with $C_1$-$C_4$ alkoxy, or $C_1$-$C_2$ alkyl and $C_1$-$C_4$ alkoxy; or

2(or 4)-pyridyloxy optionally substituted by $C_1$-$C_4$ alkyl, trifluoromethyl or 1 to 2 halogens; and

$C_1$-$C_4$ mono or dialkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

Provided also that when Y is hydroxy, Z cannot be hydrogen;

the N-oxides thereof, provided that $R_3$ cannot be unsaturated alkyl and Y and Z cannot be alkylthioalkyl, alkylaminoalkyl, dialkylaminoalkyl, trialkylammoniumalkyl, amino, 2(or 4)-pyridyloxy, alkoxyamino or unsaturated alkyl;

the optical isomers thereof when $R_1$ and $R_2$ are not the same;

the tautomers and geometeric isomers thereof; and

the acid addition salts thereof except when $R_3$ is a salt forming cation.

14. A method according to Claim 13 for controlling undesirable plant species comprising: applying to the foliage of said plants or to soil or water containing seeds or other propagating organs thereof, a herbicidally effective amount of a compound having the structure:

(I) 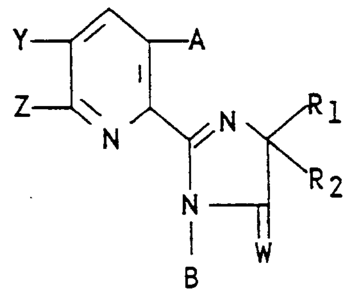

wherein A, B, W, $R_1$ and $R_2$ are as described in said claim 1, one of Y and Z represents hydrogen or methyl and the other of Y and Z represents;

$C_2$-$C_6$ straight or branched alkenyl optionally substituted with phenyl, $C_1$-$C_4$ alkoxy or 1 to 3 halogens;

$C_2$-$C_6$ straight or branched alkynyl optionally substituted with phenyl, $C_1$-$C_4$ alkoxy or 1 to 3 halogens;

$C_3$-$C_6$ cycloalkyl optionally substituted with methyl, halogen or $C_1$-$C_4$ alkoxy;

$C_1$-$C_4$ alkylcarbonyl optionally substituted with $C_1$-$C_4$ alkoxy or 1 to 3 halogens;

$C_1$-$C_4$ alkylcarbonylamino optionally substituted on nitrogen with $C_1$-$C_2$ alkyl or $C_1$-$C_4$ alkoxy and on carbon with $C_1$-$C_4$ alkoxy or 1 to 3 halogens;

$C_1$-$C_4$ alkyl substituted with $C_1$-$C_4$ alkoxy, $C_1$-$C_4$ alkylthio, $C_1$-$C_4$ alkylamino, $C_1$-$C_4$ dialkylamino, $C_1$-$C_4$ trialkylammonium or $C_1$-$C_4$ alkylcarbonyloxy;

2(or 3)-furyl optionally substituted with halogen or $C_1$-$C_3$ alkyl;

2(or 3)-thienyl optionally substituted with halogen or $C_1$-$C_3$ alkyl;

N-methyl-2(or 3)-pyrrolyl;

hydroxy;

amino optionally substituted with $C_1$-$C_4$ alkoxy or $C_1$-$C_2$ alkyl and $C_1$-$C_4$ alkoxy;

2(or 4)-pyridyloxy optionally substituted with $C_1$-$C_4$ alkyl, trifluoromethyl or 1 or 2 halogens;

$C_1$-$C_4$ mono or dlalkylaminocarbonylamino, optionally substituted on the nitrogen attached to the ring with $C_1$-$C_2$ alkyl or $C_1$-$C_4$ alkoxy;

$C_1$-$C_4$ alkoxycarbonylamino, optionally substituted on nitrogen with $C_1$-$C_2$ alkyl or $C_1$-$C_4$ alkoxy;

$C_1$-$C_4$ mono or dialkylaminocarbonyloxy;

the N-oxides thereof, provided that $R_3$ cannot be unsaturated alkyl;

the optical isomers thereof when $R_1$ and $R_2$ are not the same;

the tautomers thereof, and

the acid addition salts thereof except when $R_3$ is a cation.

**Claims for the following Contracting States : AT, ES**

1. A process for the preparation of a compound having the structural formula

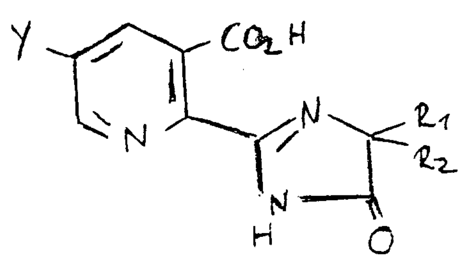

wherein $R_1$ and $R_2$ are each independently $C_1$-$C_4$ alkyl; and Y is $C_2$-$C_6$ straight or branched alkenyl optionally substituted with phenyl, $C_1$-$C_4$ alkoxy or 1 to 3 halogens, which comprises oxidizing a hydroxymethyl compound having the structural formula

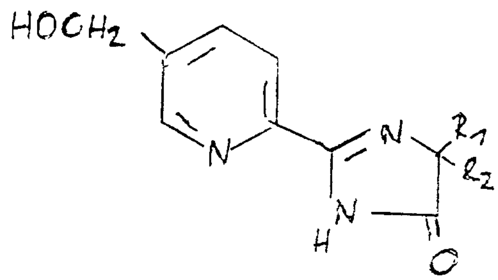

wherein $R_1$ and $R_2$ are as described above with manganese dioxide at an elevated temperature in the presence of an inert organic solvent to form a carboxaldehyde compound having the structural formula

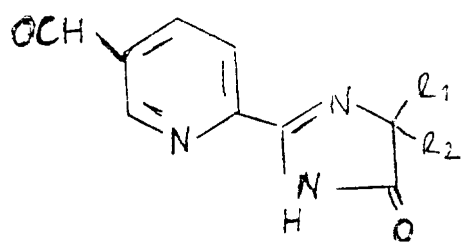

wherein $R_1$ and $R_2$ are as described above, reacting the carboxaldehyde compound with an appropriate alkyl triphenylphosphonium halide compound in the presence of an alkali metal hydride, mineral oil and dimethylsulfoxide to form a 5-alkenyl compound having the structural formula

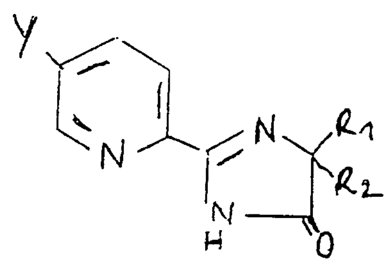

wherein Y is as described above, and reacting the 5-alkenyl compound with methyl lithium and carbon dioxide in the presence of tetrahydrofuran.

**2.** A process for the preparation of 2-(4-isopropyl-4-methyl-5-oxo-2-imidazolin-2-yl)-5-(methoxymethyl)-nicotinic acid which comprises reacting methyl 5-(bromomethyl)-2-(4-isopropyl-4-methyl-5-oxo-2-imidazolin-2-yl)nicotinate with potassium methoxide to form methyl 2-(4-isopropyl-4-methyl-5-oxo-2-imidazolin-2-yl)-5-(methoxymethyl)nicotinate, reacting the 5-(methoxymethyl)nicotinate with sodium hydroxide in the presence of methanol to form a 5-(methoxymethyl) nicotinic acid salt, and reacting the acid salt with hydrochloric acid.

## Patentansprüche

## Patentansprüche für folgende Vertragsstaaten : BE, CH, LI, DE, FR, GB, GR, IT, LU, NL, SE

**1.** Verbindung der Struktur

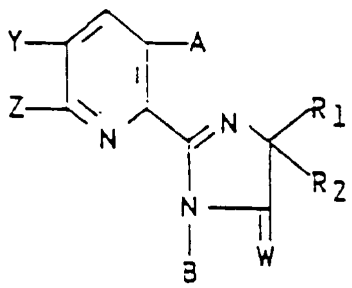

(I)

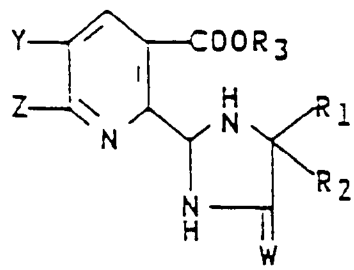

(II)

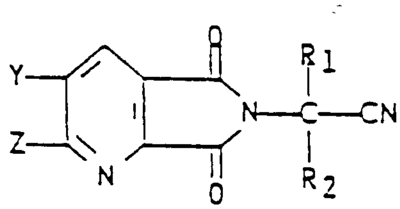

(III)

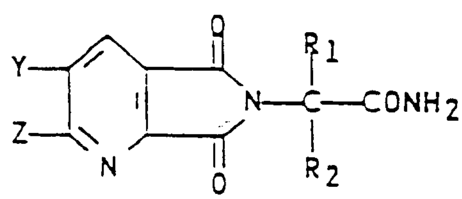

(IV)

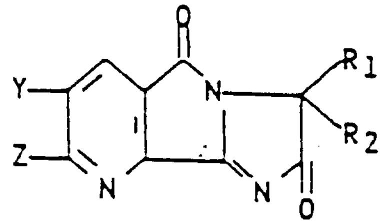

(V)

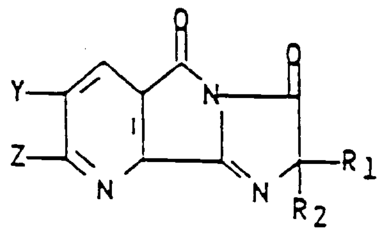

(VI)

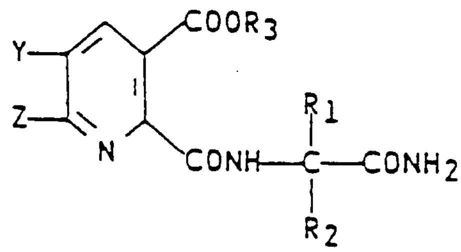

(VII)

worin

R1 $C_1$-$C_4$-Alkyl bedeutet,

R2 $C_1$-$C_4$-Alkyl bedeutet,

A $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

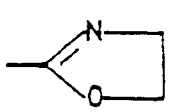

oder

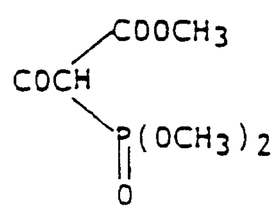

;

bedeutet,

R3 Wasserstoff,

```
            Niederalkyl
           /
    -N=C
           \
            Niederalkyl,
```

$C_1$-$C_{12}$ -Alkyl,gegebenenfalls substituiert mit einer der nachstehenden Gruppen: $C_1$-$C_3$-Alkoxy, $C_1$-$C_3$-Alkylthio, Halogen, Hydroxyl, $C_3$-$C_6$-Cycloalkyl, Benzyloxy, Furyl, Phenyl, Halogenphenyl, Niederalkylphenyl, Niederalkoxyphenyl, Nitrophenyl, Carboxyl, Niederalkoxycarbonyl, Cyano oder Triniederalkylammoniumhalogenid;

$C_3$-$C_{12}$ -Alkenyl, gegebenenfalls substituiert mit einer der nachstehenden Gruppen: $C_1$-$C_3$-Alkoxy, Phenyl, Halogen oder Niederalkoxycarbonyl oder mit zwei $C_1$-$C_3$-Alkoxygruppen oder zwei Halogengruppen,

$C_3$-$C_6$ -Cycloalkyl, gegebenenfalls substituiert mit einer oder zwei $C_1$-$C_3$-Alkylgruppen;

$C_3$-$C_{16}$ -Alkinyl, gegebenenfalls substituiert mit einer oder zwei $C_1$-$C_3$-Alkylgruppen oder ein Kation bedeutet;

$R_6$ Wasserstoff, Hydroxyl, $C_3$-Alkenyl, $C_3$-Alkinyl oder $C_1$-$C_4$-Alkyl, gegebenenfalls substituiert mit einer Hydroxyl- oder einer Chlorgruppe bedeutet;

B H, $COR_4$ oder $SO_2R_5$ bedeutet, mit der Maßgabe, daß, wenn B $COR_4$ oder $SO_2R_5$ ist; A $COOR_3$ bedeutet, wobei $R_3$ nicht H ist oder ein Kation, $CH_3$ oder CN; W O ist; und Y und Z nicht Alkylamino, Hydroxyl oder Hydroxyniederalkyl bedeuten;

$R_4$ $C_1$-$C_{11}$-Alkyl, Chlormethyl oder Phenyl, gegebenenfalls substituiert mit einer Chlor-, einer Nitro-oder einer Methoxygruppe, bedeutet;

$R_5$ $C_1$-$C_4$-Alkyl oder Phenyl, gegebenenfalls substituiert mit einer Methylgruppe, darstellt,

W O ist,

$R_8$ $C_1$-$C_4$-Alkyl oder Phenyl bedeutet,

Y und Z jeweils Wasserstoff, Halogen, $C_1$-$C_6$-Alkyl, $C_1$-$C_4$-Hydroxyniederalkyl, $C_1$-$C_6$-Alkoxy, $C_1$-$C_4$-Alkylthio, Phenoxy, $C_1$-$C_4$-Halogenalkyl, Nitro, Cyano, $C_1$-$C_4$-Alkylamino, Formyl, Di-$C_1$-$C_4$-niederalkylamino, $C_1$-$C_4$-Alkylsulfonyl, Difluormethoxy, Trifluormethoxy, 1,1,2,2-Tetrafluorethoxy, Phenyl, gegebenenfalls substituiert mit einer $C_1$-$C_4$-Alkyl-, $C_1$-$C_4$-Alkoxy- oder Halogengruppe;

$C_3$-$C_8$ gerad- oder verzweigtkettige Alkenyloxygruppe, gegebenenfalls substituiert mit ein bis drei Halogenatomen;

$C_3$-$C_8$ gerad- oder verzweigtkettige Alkinyloxygruppe, gegebenenfalls substituiert mit ein bis drei Halogenatomen;

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_2$-$C_6$ gerad- oder verzweigtkettige Alkinylgruppe, gegebenenfalls substituiert mit Phenyl, $C_1$-$C_4$-Alkoxy mit 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkylcarbonyloxy, gegebenenfalls substituiert mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Dialkylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy;

2(oder 4)-Pyridyloxy, gegebenenfalls substituiert mit $C_1$-$C_4$-Alkyl, Trifluormethyl

oder 1 oder 2 Halogenatomen;

$C_1$-$C_4$ -Mono- oder Dialkylaminocarbonylamino, gegebenenfalls substituiert am Stickstoffatom, das an den Ring gebunden ist mit $C_1$-$C_2$-Alkyl oder $C_1$-$C_4$-Alkoxy;

$C_1$-$C_4$ -Alkoxycarbonylamino, gegebenenfalls substituiert am Stickstoffatom mit $C_1$-$C_2$-Alkyl oder $C_1$-$C_4$-Alkoxy;

$C_1$-$C_4$ -Mono- oder Dialklaminocarbonyloxy; sind,

mit der Maßgabe, daß mindestens einer der Reste Y und Z ist:

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_2$-$C_6$ gerad- oder verzweigtkettige Alkinylgruppe, gegebenenfalls substituiert mit Phenyl, $C_1$-$C_4$-Alkoxy, oder mit 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Dialkylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy; oder

2(oder 4)-Pyridyloxy, gegebenenfalls substituiert mit $C_1$-$C_4$-Alkyl, Trifluormethyl oder 1 bis 2 Halogenatomen; und

$C_1$-$C_4$ -Mono- oder Dialkylaminocarbonylamino, gegebenenfalls substituiert am Stickstoffatom, das an dem Ring gebunden ist mit $C_1$-$C_2$-Alkyl oder $C_1$-$C_4$-Alkoxy;

$C_1$-$C_4$ -Alkoxycarbonylamino, gegebenenfalls substituiert am Stickstoffatom, mit $C_1$-$C_2$-Alkyl oder $C_1$-$C_4$-Alkoxy;

$C_1$-$C_4$ -Mono- oder Dialkylaminocarbonyloxy;

mit der weiteren Maßgabe, daß wenn Y Hydroxy ist Z nicht Wasserstoff sein kann;

die N-Oxide davon, mit der Maßgabe, daß $R_3$ nicht ungesättigtes Alkyl bedeutet und Y und Z nicht Alkylthioalkyl, Alkylaminoalkyl, Dialkylaminoalkyl, Trialkylammoniumalkyl, Amino, 2(oder 4)-Pyridyloxy, Alkoxyamino oder eine ungesättigte Alkylgruppe bedeuten können und die optischen Isomeren davon, wenn $R_1$ und $R_2$ nicht gleich sind;

die tautomeren und geometrischen Isomeren davon und die Säureadditionssalze davon, mit der Ausnahme, wenn $R_3$ ein salzbildendes Kation ist.

**2.** Verbindung nach Anspruch 1 der Struktur:

(I)

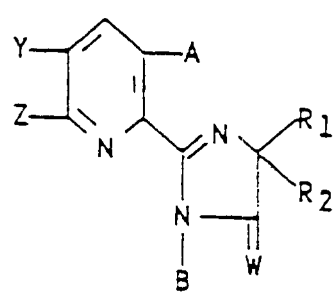

worin A, B, W, $R_1$, $R_2$, Y und Z wie in Anspruch 1 beschrieben sind.

**3.** Verbindung nach Anspruch 2 der Struktur:

(I)

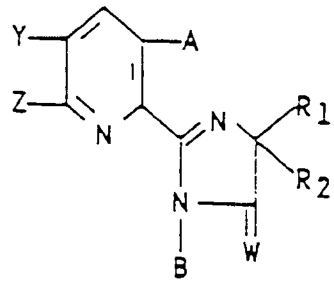

worin A, B, W, $R_1$ und $R_2$ wie in Anspruch 1 beschrieben sind, einer der Reste Y und Z Wasserstoff oder Methyl bedeutet und die anderen Reste Y und Z wiedergeben:

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_2$-$C_6$ gerad- oder verzweigtkettige Alkinylgruppe, gegebenenfalls substituiert mit Phenyl, $C_1$-$C_4$-Alkoxy oder 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Dialkylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy;

2(oder 4)-Pyridyloxy, gegebenenfalls substituiert mit $C_1$-$C_4$-Alkyl, Trifluormethyl oder 1 oder 2 Halogenatomen;

$C_1$-$C_4$ -Mono- oder Dialkylaminocarbonylamino, gegebenenfalls substituiert am Stickstoffatom, das an den Ring gebunden ist mit $C_1$-$C_2$-Alkyl oder $C_1$-$C_4$-Alkoxy;

$C_1$-$C_4$ -Alkoxycarbonylamino, gegebenenfalls substituiert am Stickstoffatom mit $C_1$-$C_2$-Akyl oder $C_1$-$C_4$-Alkoxy;

$C_1$-$C_4$ -Mono- oder Dialkylaminocarbonyloxy;

die N-Oxide davon, mit der Maßgabe, daß $R_3$ keine ungesättigte Alkylgruppe bedeutet, die optischen Isomeren davon,wenn $R_1$ und $R_2$ nicht dieselben sind;

die tautomeren und geometrischen Isomeren davon und die Säureadditionssalze davon, mit der Ausnahme, wenn $R_3$ ein salzbildendes Kation ist.

**4.** Verbindung nach Anspruch 2, nämlich 5-Isopropenyl-2-(4-isopropyl-4-methyl-5-oxo-2-imdazolin-2-yl)-nicotinsäure und die Salze und Ester davon.

**5.** Verbindung nach Anspruch 2, nämlich 2-(4-Isopropyl-4-methyl-5-oxo-2-imidazolin-2-yl)-5-(methoxymethyl)-nicotinsäure oder die Ester oder Salze davon.

**6.** Verbindung nach Anspruch 2, nämlich 2-(4-Isopropyl-4-methyl-5-oxo-2-imdazolin-2-yl)-5-styrylnicotinsäure, 2-(4-Isopropyl-4-methyl-5-oxo-2-imdazolin-2-yl)-5-propenylnicotinsäure, 5-Acetyl-2-(4-isopropyl-4-methyl-5-oxo-2-imidazolin-2-yl)nicotinsäure, 5-Acetyl-2-(4-isopropyl-4-methyl-5-oxo-2-imdazolin-2-yl)-6-methylnicotinsäure, 2-(4-Isopropyl-4-methyl-5-oxo-2-imdazolin-2-yl)-5-[(methylthio)methyl]-nicotinsäure, 6-(2-Furyl)-2-(4-isopropyl-4-methyl-5-oxo-2-imdazolin-2-yl)nicotinsäure, 2-(4-Isopropyl-4-methyl-5-oxo-2-imidazolin-2-yl)-5-(2-thienyl)nicotinsäure, 2-(4-Isopropyl-4-methyl-5-oxo-2-imidazolin-2-yl)-6-(2-thienyl)-nicotinsaure, 2-(4-Isopropyl-4-methyl-5-oxo-2-imdazolin-2-yl)-5-(3-thienyl)nicotinsäure, 1,6-Dihydro-2-(4-isopropyl-4-methyl-5-oxo-2-imdazolin-2-yl)-6-oxynicotinsäure und 2-(4-Isopropyl-4-methyl-5-oxo-2-imidazolin-2-yl)-6-[(methylthio)methyl]nicotinsäure und die Salze und Ester davon.

7. Verbindung nach Anspruch 2, nämlich 2-(1-Acetyl-4-isopropyl-4-methyl-5-oxo-2-imidazolin-2-yl)-6-(hydroxymethyl)nicotinsäureessigmethylester und Isopropylammonium-6-(2-furyl)-2-(4-isopropyl-4-methyl-5-oxo-2-imidazolin-2-yl)nicotinat.

8. Verbindung nach Anspruch 1 der Struktur:

(III) 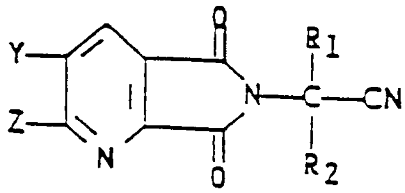

worin $R_1$, $R_2$, Y und Z wie in Anspruch 1 beschrieben sind.

9. Verbindung nach Anspruch 1 der Struktur:

(IV) 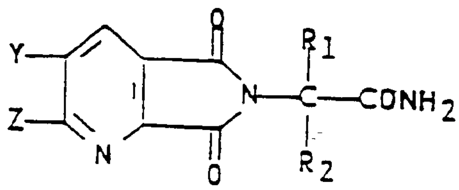

worin $R_1$, $R_2$, Y und Z wie in Anspruch 1 beschrieben sind.

10. Verbindung nach Anspruch 1 der Struktur:

(V) 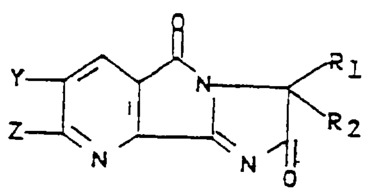

worin $R_1$, $R_2$, Y und Z wie in Anspruch 1 beschrieben sind.

11. Verbindung nach Anspruch 1 der Struktur:

(VI) 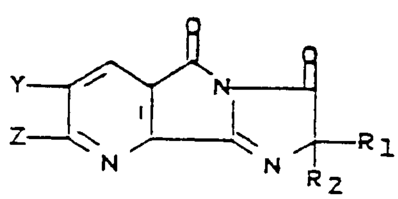

worin $R_1$, $R_2$, Y und Z wie in Anspruch 1 beschrieben sind.

**12.** Verbindung nach Anspruch 1 der Struktur:

(VII) 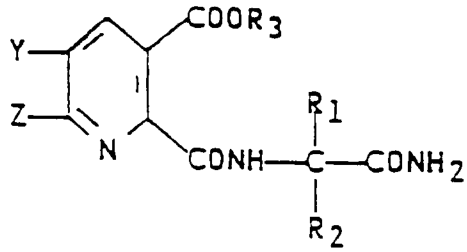

worin $R_1$, $R_2$, $R_3$, Y und Z wie in Anspruch 1 beschrieben sind.

**13.** Verfahren zur Bekämpfung unerwünschter Pflanzenarten, umfassend: Anwenden auf das Blattwerk der Pflanzen oder auf den Boden oder das Wasser, enthaltend Samen oder andere Fortpflanzungsorgane davon, einer herbizid wirksamen Menge einer Verbindung der Struktur:

I. 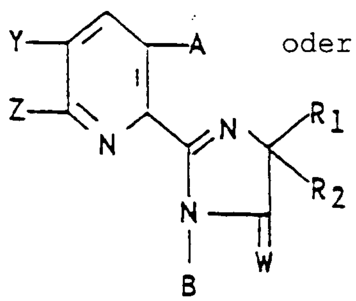 oder II. 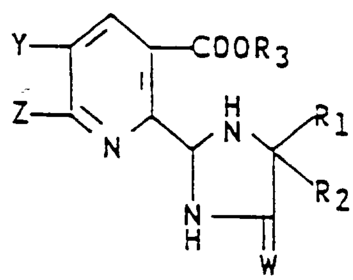

worin

$R_1$ $C_1$-$C_4$-Alkyl bedeutet,

$R_2$ $C_1$-$C_4$-Alkyl bedeutet,

A $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

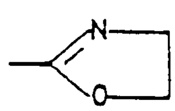 oder 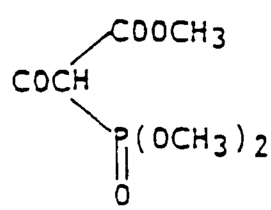 ;

bedeutet,

$R_3$ Wasserstoff,

```
       Niederalkyl
      /
-N=C
      \
       Niederalkyl
```

$C_1$-$C_{12}$ -Alkyl, gegebenenfalls substituiert mit einer der nachstehenden Gruppen: $C_1$-$C_3$-Alkoxy, $C_1$-$C_3$-Alkylthio, Halogen, Hydroxyl, $C_3$-$C_6$-Cycloalkyl, Benzyloxy, Furyl, Phenyl, Halogenphenyl, Niederalkylphenyl, Niederalkoxyphenyl, Nitrophenyl, Carboxyl,

Niederalkoxycarbonyl, Cyano oder Triniederalkylammoniunhalogenid;

$C_3$-$C_{12}$ -Alkenyl, gegebenenfalls substituiert mit einer der nachstehenden Gruppen: $C_1$-$C_3$-Alkoxy, Phenyl, Halogen oder Niederalkoxycarbonyl oder mit zwei $C_1$-$C_3$-Alkoxygruppen oder zwei Halogengruppen,

$C_3$-$C_6$ -Cycloalkyl, gegebenenfalls substituiert mit einer oder zwei $C_1$-$C_3$-Alkylgruppen;

$C_3$-$C_{16}$ -Alkinyl, gegebenenfalls substituiert mit einer oder zwei $C_1$-$C_3$-Alkylgruppen oder ein Kation bedeutet;

$R_6$ Wasserstoff, Hydroxyl, $C_3$-Alkenyl, $C_3$-Alkinyl oder $C_1$-$C_4$-Alkyl, gegebenenfalls substituiert mit einer Hydroxyl- oder einer Chlorgruppe, bedeutet;

B H, $COR_4$ oder $SO_2R_5$ bedeutet, mit der Maßgabe, daß, wenn B $COR_4$ oder $SO_2R_5$ ist; A $COOR_3$ bedeutet, wobei $R_3$ nicht H ist oder ein Kation, $CH_3$ oder CN; W O ist; und Y und Z nicht Alkylamino, Hydroxyl oder Hydroxyniederalkyl bedeuten;

$R_4$ $C_1$-$C_{11}$-Alkyl, Chlormethyl oder Phenyl, gegebenenfalls substituiert mit einer Chlor-, einer Nitro- oder einer Methoxygruppe bedeutet;

$R_5$ $C_1$-$C_4$-Alkyl oder Phenyl, gegebenenfalls substituiert mit einer Methylgruppe darstellt,

W O ist,

$R_8$ $C_1$-$C_4$-Alkyl oder Phenyl bedeutet,

Y und Z jeweils Wasserstoff, Halogen, $C_1$-$C_6$-Alkyl, $C_1$-$C_4$-Hydroxyniederalkyl, $C_1$-$C_6$-Alkoxy, $C_1$-$C_4$-Alkylthio, Formyl, Phenoxy, $C_1$-$C_4$-Halogenalkyl, Nitro, Cyano, $C_1$-$C_4$-Alkylamino, Di-$C_1$-$C_4$-niederalkylamino, $C_1$-$C_4$-Alkylsulfonyl, Difluormethoxy, Trifluormethoxy, 1,1,2,2-Tetrafluorethoxy, Phenyl, gegebenenfalls substituiert mit einer $C_1$-$C_4$-Alkyl-, $C_1$-$C_4$-Alkoxy- oder Halogengruppe;

$C_3$-$C_8$ gerad- oder verzweigtkettige Alkenyloxygruppe, gegebenenfalls substituiert mit ein bis drei Halogenatomen;

$C_3$-$C_8$ gerad- oder verzweigtkettige Alkinyloxygruppe, gegebenenfalls substituiert mit ein bis drei Halogenatomen;

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_2$-$C_6$ gerad- oder verzweigtkettige Alkinylgruppe, gegebenenfalls substituiert mit Phenyl, $C_1$-$C_4$-Alkoxy oder 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkylcarbonyloxy, gegebenenfalls substituiert mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Dialkylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy;

2(oder 4)-Pyridyloxy, gegebenenfalls substituiert mit $C_1$-$C_4$-Alkyl, Trifluormethyl oder 1 oder 2 Halogenatomen;

$C_1$-$C_4$ -Mono- oder Dialkylaminocarbonylamino, gegebenenfalls substituiert am Stickstoffatom, das an den Ring gebunden ist mit $C_1$-$C_2$-Alkyl oder $C_1$-$C_4$-Alkoxy;

$C_1$-$C_4$ -Alkoxycarbonylamino, gegebenenfalls substituiert am Stickstoffatom mit $C_1$-$C_2$-Alkyl oder $C_1$-$C_4$-Alkoxy;

$C_1$-$C_4$ -Mono- oder Dialkylaminocarbonyloxy; sind,

mit der Maßgabe, daß mindestens einer der Reste Y und Z ist:

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_1$-$C_6$ gerad- oder verzweigtkettige Alkinylgruppe, gegebenenfalls substituiert mit Phenyl, $C_1$-$C_4$-

Alkoxy mit 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Diallylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy; oder

2(oder 4)-Pyridyloxy, gegebenenfalls substituiert mit $C_1$-$C_4$-Alkyl, Trifluormethyl oder 1 bis 2 Halogenatomen; und

$C_1$-$C_4$ -Mono- oder Dialkylaminocarbonylamino, gegebenenfalls substituiert am Stickstoffatom, das an den Ring gebunden ist mit $C_1$-$C_2$-Alkyl oder $C_1$-$C_4$-Alkoxy;

$C_1$-$C_4$ -Alkoxycarbonylamino, gegebenenfalls substituiert am Stickstoffatom, mit $C_1$-$C_2$-Alkyl oder $C_1$-$C_4$-Alkoxy;

$C_1$-$C_4$ -Mono- oder Dialkylaminocarbonyloxy;

mit der weiteren Maßgabe, daß wenn Y Hydroxy ist, Z nicht Wasserstoff sein kann;

die N-Oxide davon, mit der Maßgabe, daß $R_3$ nicht ungesättigtes Alkyl bedeutet und Y und Z nicht Alkylthioalkyl, Alkylaminoalkyl, Dialkylaminoalkyl, Trialkylammoniumalkyl, Amino, 2(oder 4)-Pyridyloxy, Alkoxyamino oder ungesättigtes Alkyl bedeuten können und die optischen Isomeren davon, wenn $R_1$ und $R_2$ nicht gleich sind;

die tautomeren und geometrischen Isomeren davon und die Säureadditionssalze davon, mit der Ausnahme, wenn $R_3$ ein salzbildendes Kation ist.

**14.** Verfahren zur Bekämpfung unerwünschter Pflanzenarten, umfassend: Anwenden auf das Blattwerk der Pflanzen oder auf den Boden oder das Wasser, enthaltend Samen oder andere Fortpflanzungsorgane davon, einer herbizid wirksamen Menge einer Verbindung der Struktur:

(I)

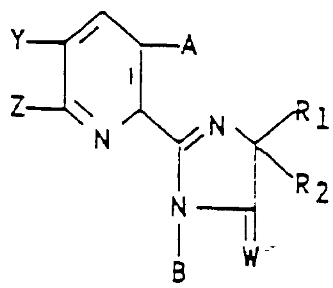

worin A, B, W, $R_1$ und $R_2$ wie in Anspruch 1 beschrieben sind, einer der Reste Y und Z Wasserstoff oder Methyl bedeutet und die anderen Reste Y und Z wiedergeben:

$C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen;

$C_2$-$C_6$ gerad- oder verzweigtkettige Alkinylgruppe, gegebenenfalls substituiert mit Phenyl, $C_1$-$C_4$-Alkoxy oder 1 bis 3 Halogenatomen;

$C_3$-$C_6$ -Cycloalkyl, gegebenenfalls substituiert mit Methyl, Halogen oder $C_1$-$C_4$-Alkoxy,

$C_1$-$C_4$ -Alkylcarbonyl, gegebenenfalls substituiert mit $C_1$-$C_4$-Alkoxy oder 1 bis 3 Halogenatomen;

$C_1$-$C_4$ -Alkylcarbonylamino, gegebenenfalls substituiert am Stickstoffatom mit $C_1$-$C_2$-Alkyl oder $C_1$-$C_4$-Alkoxy und am Kohlenstoffatom mit $C_1$-$C_4$-Alkoxy oder 1 bis 3 Halogenatomen;

$C_1$-$C_4$ -Alkyl, substituiert mit $C_1$-$C_4$-Alkoxy, $C_1$-$C_4$-Alkylthio, $C_1$-$C_4$-Alkylamino, $C_1$-$C_4$-Dialkylamino, $C_1$-$C_4$-Trialkylammonium oder $C_1$-$C_4$-Alkylcarbonyloxy, 2(oder 3)-Furyl, gegebenenfalls substituiert mit Halogen oder $C_1$-$C_3$-Alkyl, 2(oder 3)-Thienyl, gegebenenfalls substituiert mit Halogen oder $C_1$-$C_3$-Alkyl, N-Methyl-2(oder 3)-pyrrolyl;

Hydroxy;

Amino, gegebenenfalls substituiert mit $C_1$-$C_4$-Alkoxy oder $C_1$-$C_2$-Alkyl und $C_1$-$C_4$-Alkoxy;

2(oder 4)-Pyridyloxy, gegebenenfalls substituiert mit $C_1$-$C_4$-Alkyl, Trifluormethyl oder 1 oder 2

Halogenatomen;

$C_1$-$C_4$ -Mono- oder Dialkylaminocarbonylamino, gegebenenfalls substituiert am Stickstoffatom, das an den Ring gebunden ist, mit $C_1$-$C_2$-Alkyl oder $C_1$-$C_4$-Alkoxy;

$C_1$-$C_4$ -Alkoxycarbonylamino, gegebenenfalls substituiert am Stickstoffatom mit $C_1$-$C_2$-Alkyl oder $C_1$-$C_4$-Alkoxy;

$C_1$-$C_4$ -Mono- oder Dialkylaminocarbonyloxy;

die N-Oxide davon, mit der Maßgabe, daß $R_3$ keine ungesättigte Alkylgruppe bedeutet, die optischen Isomeren davon, wenn $R_1$ und $R_2$ nicht dieselben sind;

die Tautomeren davon und die Säureadditionssalze davon,mitder Ausnahme, wenn $R_3$ ein salzbildendes Kation ist.

**Patentansprüche für folgende Vertragsstaaten : AT, ES**

1. Verfahren zur Herstellung einer Verbindung der Strukturformel

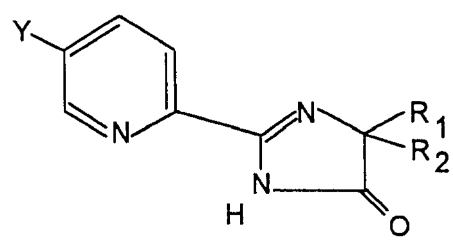

worin $R_1$ und $R_2$ jeweils unabhängig voneinander $C_1$-$C_4$-Alkyl darstellen; und Y eine $C_2$-$C_6$ gerad- oder verzweigtkettige Alkenylgruppe, gegebenenfalls substituiert mit Phenyl, $C_1$-$C_4$-Alkoxy oder 1 bis 3 Halogenatomen, bedeutet; umfassend Oxidieren einer Hydroxymethylverbindung der Strukturformel

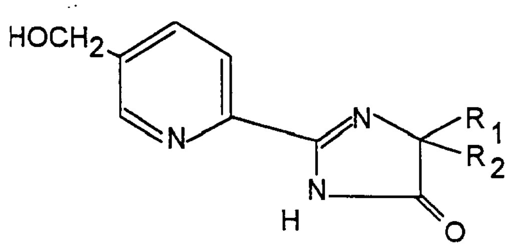

worin $R_1$ und $R_2$ wie vorstehend beschrieben sind, mit Mangandioxid bei erhöhter Temperatur in Gegenwart eines inerten organischen Lösungsmittels zu einer Carboxaldehydverbindung der Strukturformel

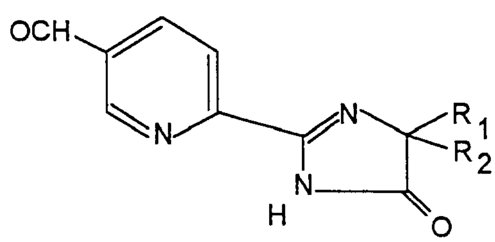

worin $R_1$ und $R_2$ wie vorstehend beschrieben sind, Umsetzen der Carboxaldehydverbindung mit einer geeigneten Alkyltriphenylphosphoniumhalogenidverbindung in Gegenwart eines Alkalimetallhydrids, Mineralöls und Dimethylsulfoxid zu einer 5-Alkenylverbindung der Strukturformel

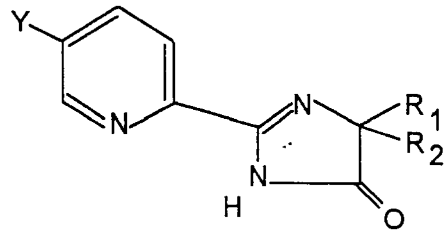

worin Y wie vorstehend beschrieben ist und Umsetzen der 5-Alkenylverbindung mit Methyllithium und Kohlendioxid in Gegenwart von Tetrahydrofuran.

2. Verfahren zur Herstellung von 2-(4-Isopropyl-4-methyl-5-oxo-2-imidazolin-2-yl)-5-(methoxymethyl)-nicotinsäure, umfassend Umsetzung von 5-(Brommethyl)-2-(4-Isopropyl-4-methyl-5-oxo-2-imidazolin-2-yl)-nicotinsäuremethylester mit Kaliummethoxid zu 2-(4-Isopropyl-4-methyl-5-oxo-2-imdazolin-2-yl)-5-(methoxymethyl)nicotinsäuremethylester, Umsetzen des 5-(Methoxymethyl)nicotinsäureesters mit Natriumhydroxid in Gegenwart von Methanol zu einem 5-(Methoxymethyl)nicotinsäuresalz und Umsetzen des sauren Salzes mit Salzsäure.

**Revendications**

**Revendications pour les Etats contractants suivants : BE, CH, LI, DE, FR, GB, GR, IT, LU, NL, SE**

**1.** Composés de structure :

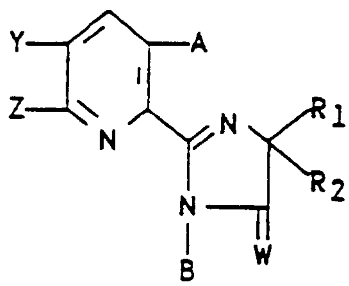

(I)

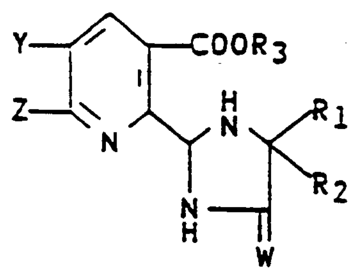

(II)

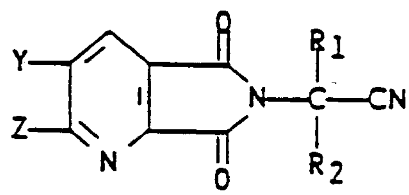

(III)

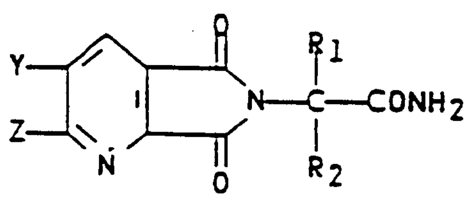

(IV)

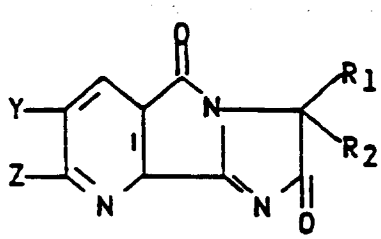

(V)

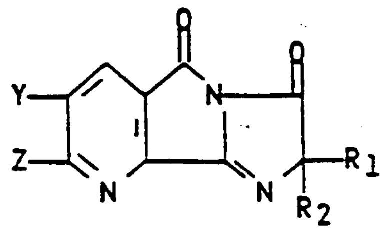

(VI)

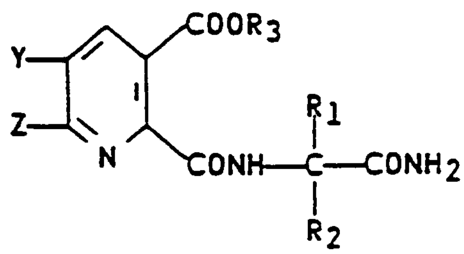

(VII)

où

R1 est un groupe alkyle en $C_1$-$C_4$ ;

R2 est un groupe alkyle en $C_1$-$C_4$ ;

A est $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

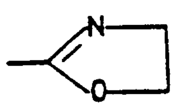

ou

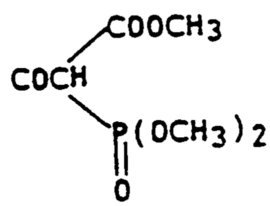

;

R3 est un atome d'hydrogène,

un groupe

/alkyle inférieur<br>
-N=C<br>
\alkyle inférieur

un groupe alkyle en $C_1$-$C_{12}$ facultativement substitué avec un des. groupes suivants : alcoxy en $C_1$-$C_3$, alkylthio en $C_1$-$C_3$, halogéno, hydroxyle, cycloalkyle en $C_3$-$C_6$, benzyloxy, furyle, phényle, halogénophényle, (alkyl inférieur)phényle, (alcoxy inférieur)phényle, nitrophényle, carboxyle, (alcoxy inférieur) carbonyle, cyano ou halogénure de tri(alkyl inférieur) ammonium ;

un groupe alcényle en $C_3$-$C_{12}$ facultativement substitué avec un des groupes suivants : alcoxy en $C_1$-$C_3$, phényle, halogéno ou (alcoxy inférieur)carbonyle ou avec deux groupes alcoxy en $C_1$-$C_3$ ou deux groupes halogéno ;

un groupe cycloalkyle en $C_3$-$C_6$ facultativement substitué avec un ou deux groupes alkyle en $C_1$-$C_3$ ;

un groupe alcynyle en $C_3$-$C_{16}$ facultativement substitué avec un ou deux groupes alkyle en $C_1$-$C_3$ ; ou
un cation ;

$R_6$ est un atome d'hydrogène, un groupe hydroxyle, alcényle en $C_3$, alcynyle en $C_3$ ou alkyle en $C_1$-$C_4$ facultativement substitué avec un groupe hydroxyle ou un groupe chloro ;

B est H, $COR_4$ ou $SO_2R_5$, sous réserve que, lorsque B est $COR_4$ ou $SO_2R_5$, A soit $COOR_3$ où $R_3$ est autre que H ou un cation, $CH_3$ ou CN ; W soit O ; et Y et Z ne soient pas un groupe alkylamino, hydroxyle ou hydroxyalkyle inférieur ;

$R_4$ est un groupe alkyle en $C_1$-$C_{11}$, chlorométhyle ou phényle facultativement substitué avec un groupe chloro, un groupe nitro ou un groupe méthoxy ;

$R_5$ est un groupe alkyle en $C_1$-$C_4$ ou phényle facultativement substitué avec un groupe méthyle ;

W est O ;

$R_8$ est un groupe alkyle en $C_1$-$C_4$ ou phényle ;

Y et Z sont chacun un atome d'hydrogène ou un groupe halogéno, alkyle en $C_1$-$C_6$, hydroxyalkyle inférieur en $C_1$-$C_4$, alcoxy en $C_1$-$C_6$, alkylthio en $C_1$-$C_4$, phénoxy, halogénoalkyle en $C_1$-$C_4$, nitro, cyano, alkylamino en $C_1$-$C_4$, formyle, di(alkyl inférieur en $C_1$-$C_4$) amino, alkylsulfonyle en $C_1$-$C_4$, difluorométhoxy, trifluorométhoxy, 1,1,2,2-tétrafluoroéthoxy ou phényle facultativement substitué avec un groupe alkyle en $C_1$-$C_4$, alcoxy en $C_1$-$C_4$ ou halogéno ;

un groupe alcényloxy en $C_3$-$C_8$ droit ou ramifié facultativement substitué avec un à trois groupes halogéno ;

un groupe alcynyloxy en $C_3$-$C_8$ droit ou ramifié facultativement substitué avec un à trois groupes halogéno ;

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe (alkyl en $C_1$-$C_4$)carbonyloxy facultativement substitué avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$)amino, tri(alkyl en $C_1$-$C_4$)ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$ ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitute sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ;

sous réserve qu'au moins un de Y et de Z soit :

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$) amino, tri(alkyl en $C_1$-$C_4$)ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$ ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitué sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ;

sous réserve également que, lorsque Y est un groupe hydroxy, Z ne soit pas un atome d'hydrogène ;

leurs N-oxydes, sous réserve que $R_3$ ne soit pas un groupe alkyle insaturé et Y et Z ne soient pas un groupe alkylthioalkyle, alkylaminoalkyle, dialkylaminoalkyle, trialkylammoniumalkyle, amino, 2(ou 4)-pyridyloxy, alcoxyamino ou alkyle insaturé ;

leurs isomères optiques lorsque $R_1$ et $R_2$ ne sont pas identiques ;

leurs tautomères et leurs isomères géométriques ; et

leurs sels d'addition d'acides, sauf lorsque $R_3$ est un cation formant un sel.

**2.** Composés selon la revendication 1 ayant la structure :

(I) 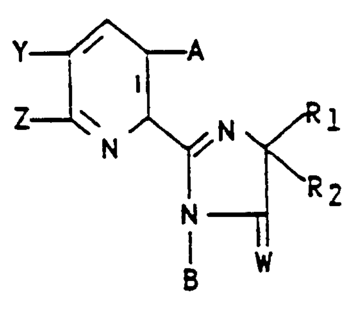

dans laquelle A, B, W, $R_1$, $R_2$, Y et Z sont comme décrit dans la revendication 1.

3. Composés selon la revendication 2 de structure :

(I)

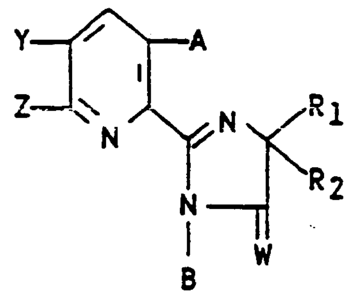

dans laquelle A, B, W, $R_1$ et $R_2$ sont comme décrit dans la revendication 1, un de Y et Z représente un atome d'hydrogène ou un groupe méthyle et l'autre de Y et Z représente

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$)amino, tri(alkyl en $C_1$-$C_4$)ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$ ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitué sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ;

leurs N-oxydes, sous réserve que $R_3$ ne soit pas un groupe alkyle insaturé ;

leurs isomères optiques lorsque $R_1$ et $R_2$ ne sont pas identiques ;

leurs tautomères et leurs isomères géométriques ; et

leurs sels d'addition d'acides sauf lorsque $R_3$ est un cation formant un sel.

4. Composés selon la revendication 2, qui sont l'acide 5-isopropényl-2-(4-isopropyl-4-méthyl-5-oxo-2-imidazoline-2-yl)nicotinique et ses sels et esters.

5. Composés selon la revendication 2, qui sont l'acide 2-(4-isopropyl-4-méthyl-5-oxo-2-imidazoline-2-yl)-5-(méthoxyméthyl) nicotinique ou ses esters ou ses sels.

6. Composés selon la revendication 2, qui sont l'acide 2-(4-isopropyl-4-méthyl-5-oxo-2-imidazoline-2-yl)-5-styrylnicotinique, l'acide 2-(4-isopropyl-4-méthyl-5-oxo-2-imidazoline-2-yl)-5-propénylnicotinique, l'acide 5-acétyl-2-(4-isopropyl-4-méthyl-5-oxo-2-imidazoline-2-yl)nicotinique, l'acide 5-acétyl-2-(4-isopropyl-4-méthyl-5-oxo-2-imidazoline-2-yl)-6-méthylnicotinique, l'acide 2-(4-isopropyl-4-méthyl-5-oxo-2-imidazoline-2-yl)-5-[(méthylthio)méthyl]nicotinique, l'acide 6-(2-furyl)-2-(4-isopropyl-4-méthyl-5-oxo-2-imidazoline-2-yl)nicotinique, l'acide 2-(4-isopropyl-4-méthyl-5-oxo-2-imidazoline-2-yl)-5-(2-thiényl) nicotinique, l'acide 2-(4-isopropyl-4-méthyl-5-oxo-2-imidazoline-2-yl)-6-(2-thiényl)nicotinique, l'acide 2-(4-isopropyl-4-méthyl-5-oxo-2-imidazoline-2-yl)-5-(3-thiényl)nicotinique, l'acide 1,6-dihydro-2-(4-isopropyl-4-méthyl-5-oxo-2-imidazoline-2-yl)-6-oxynicotinique et l'acide 2-(4-isopropyl-4-méthyl-5-oxo-2-imidazoline-2-yl)-6-[-(méthylthio)méthyl]nicotinique et leurs sels et leurs esters.

**7.** Composés selon la revendication 2, qui sont l'ester acétate du 2-(1-acétyl-4-isopropyl-4-méthyl-5-oxo-2-imidazoline-2-yl)-6-(hydroxyméthyl)nicotinate de méthyle et le 6-(2-furyl)-2-(4-isopropyl-4-méthyl-5-oxo-2-imidazoline-2-yl)nicotinate d'isopropylammonium.

**8.** Composés selon la revendication 1 ayant la structure :

(III)

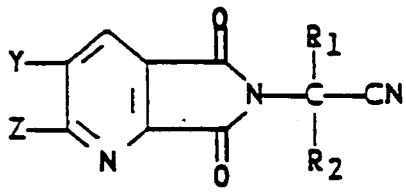

dans laquelle $R_1$, $R_2$, Y et Z sont comme décrit dans la revendication 1.

**9.** Composés selon la revendication 1 ayant la structure :

(IV)

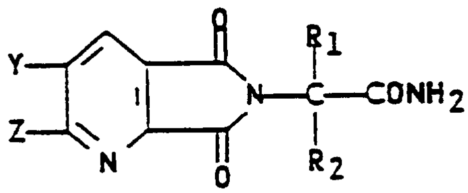

dans laquelle $R_1$, $R_2$, Y et Z sont comme décrit dans la revendication 1.

**10.** Composés selon la revendication 1 ayant la structure :

(V)

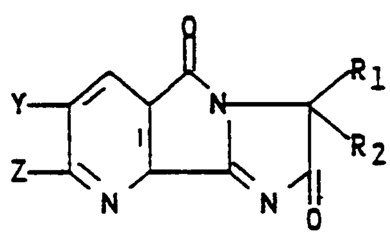

dans laquelle $R_1$, $R_2$, Y et Z sont comme décrit dans la revendication 1.

**11.** Composés selon la revendication 1 ayant la structure :

(VI)

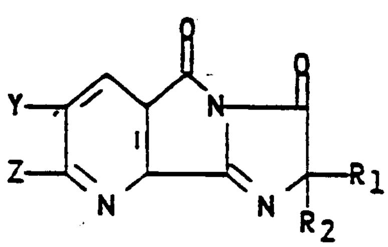

dans laquelle $R_1$, $R_2$, Y et Z sont comme décrit dans la revendication 1.

**12.** Composé selon la revendication 1 ayant la structure :

(VII)

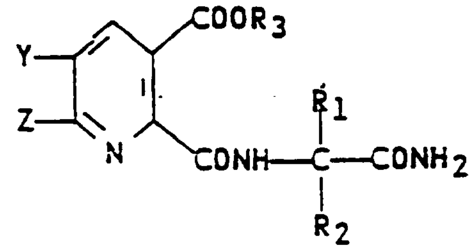

dans laquelle $R_1$, $R_2$, $R_3$, Y et Z sont comme décrit dans la revendication 1.

**13.** Procédé de maîtrise des espèces de plantes indésirables comprenant : l'application, au feuillage desdites plantes ou au sol ou à l'eau contenant des graines ou d'autres organes de leur propagation, d'une quantité herbicide efficace d'un composé de structure :

I

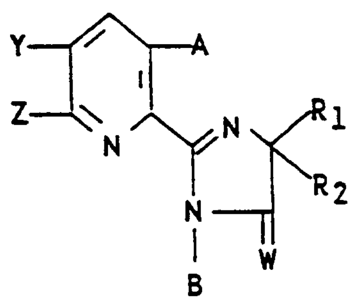

ou

II.

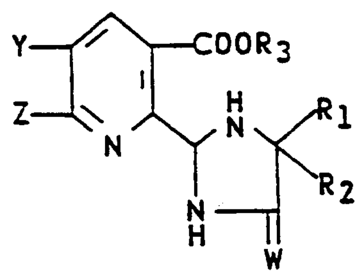

où

- $R_1$ est un groupe alkyle en $C_1$-$C_4$ ;
- $R_2$ est un groupe alkyle en $C_1$-$C_4$ ;
- A est $COOR_3$, $CONHR_6$, CHO, $CH_2OH$, $COCH_3$, $COC_6H_5$, CN, $CH_3$, CH=NOH, $CH_2COOH$, CONHOH, $CH_2CH_2COOH$, $CHR_8OH$,

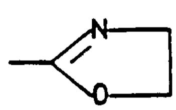

ou

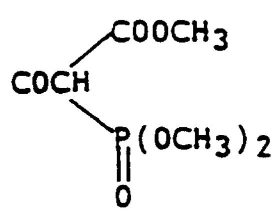

;

- $R_3$ est un atome d'hydrogène,
  un groupe

/alkyle inférieur
-N=C
\alkyle inférieur

un groupe alkyle en $C_1$-$C_{12}$ facultativement substitué avec un des groupes suivants : alcoxy en $C_1$-$C_3$, alkylthio en $C_1$-$C_3$, halogéno, hydroxyle, cycloalkyle en $C_3$-$C_6$, benzyloxy, furyle, phényle, halogénophényle, (alkyl inférieur)phényle, (alcoxy inférieur)phényle, nitrophényle, carboxyle, (alcoxy inférieur) carbonyle, cyano ou halogénure de tri(alkyl inférieur) ammonium ;

un groupe alcényle en $C_3$-$C_{12}$ facultativement substitué avec un des groupes suivants : alcoxy en $C_1$-$C_3$, phényle, halogéno ou (alcoxy inférieur)carbonyle ou avec deux groupes alcoxy en $C_1$-$C_3$ ou deux groupes halogéno ;

un groupe cycloalkyle en $C_3$-$C_6$ facultativement substitué avec un ou deux groupes alkyle en $C_1$-$C_3$ ;

un groupe alcynyle en $C_3$-$C_{16}$ facultativement substitué avec un ou deux groupes alkyle en $C_1$-$C_3$ ; ou

un cation ;

$R_6$ est un atome d'hydrogène, un groupe hydroxyle, alcényle en $C_3$, alcynyle en $C_3$ ou alkyle en $C_1$-$C_4$ facultativement substitué avec un groupe hydroxyle ou un groupe chloro ;

B est H, $COR_4$ ou $SO_2R_5$, sous réserve que, lorsque B est $COR_4$ ou $SO_2R_5$, A soit $COOR_3$ où $R_3$ est autre que H ou un cation, $CH_3$ ou CN ; W soit O ; et Y et Z ne soient pas un groupe alkylamino, hydroxyle ou hydroxyalkyle inférieur ;

$R_4$ est un groupe alkyle en $C_1$-$C_{11}$, chlorométhyle ou phényle facultativement substitué avec un groupe chloro, un groupe nitro ou un groupe méthoxy ;

$R_5$ est un groupe alkyle en $C_1$-$C_4$ ou phényle facultativement substitué avec un groupe méthyle ;

W est O ;

$R_8$ est un groupe alkyle en $C_1$-$C_4$ ou phényle ;

Y et Z sont chacun un atome d'hydrogène ou un groupe halogéno, alkyle en $C_1$-$C_6$, hydroxyalkyle inférieur en $C_1$-$C_4$, alcoxy en $C_1$-$C_6$, alkylthio en $C_1$-$C_4$, formyle, phénoxy, halogénoalkyle en $C_1$-$C_4$, nitro, cyano, alkylamino en $C_1$-$C_4$, di(alkyl inférieur en $C_1$-$C_4$) amino, alkylsulfonyle en $C_1$-$C_4$, difluorométhoxy, trifluorométhoxy$_1$ 1,1,2,2-tétrafluoroéthoxy ou phényle facultativement substitué avec un groupe alkyle en $C_1$-$C_4$, alcoxy en $C_1$-$C_4$ ou halogéno ;

un groupe alcényloxy en $C_3$-$C_8$ droit ou ramifié facultativement substitué avec un à trois groupes halogéno ;

un groupe alcynyloxy en $C_3$-$C_8$ droit ou ramifié facultativement substitué avec un à trois groupes halogéno ;

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe (alkyl en $C_1$-$C_4$)carbonyloxy facultativement substitué avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$) amino, tri(alkyl en $C_1$-$C_4$)ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$

ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitué sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ;

sous réserve qu'au moins un de Y et de Z soit :

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$) amino, tri(alkyl en $C_1$-$C_4$) ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$ ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitué sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ;

sous réserve également que, lorsque Y est un groupe hydroxy, Z ne soit pas un atome d'hydrogène ;

ses N-oxydes, sous réserve que $R_3$ ne soit pas un groupe alkyle insaturé et Y et Z ne soient pas un groupe alkylthioalkyle, alkylaminoalkyle, dialkylaminoalkyle, trialkylammoniumalkyle, amino, 2(ou 4)-pyridyloxy, alcoxyamino ou alkyle insaturé ;

ses isomères optiques lorsque $R_1$ et $R_2$ ne sont pas identiques ;

ses tautomères et ses isomères géométriques ; et

ses sels d'addition d'acides, sauf lorsque $R_3$ est un cation formant un sel.

**14.** Procédé selon la revendication 13 pour maîtriser des espèces de plantes indésirables comprenant : l'application, au feuillage desdites plantes ou au sol ou à l'eau contenant des graines ou d'autres organes de leur propagation, d'une quantité herbicide efficace d'un composé de structure :

(I)

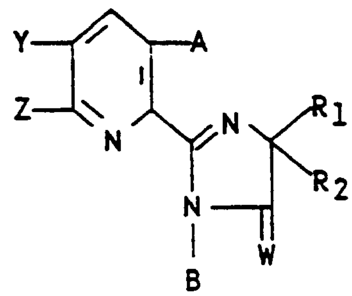

dans laquelle A, B, W, $R_1$ et $R_2$ sont comme décrit dans la revendication 1, un de Y et Z représente un atome d'hydrogène ou un groupe méthyle et l'autre de Y et Z représente

un groupe alcényle en $C_2$-$C_6$ droit ou ramifié facultativement substitué avec un groupe phényle ou alcoxy en $C_1$-$C_4$ ou un à trois groupes halogéno ;

un groupe alcynyle en $C_2$-$C_6$ droit ou ramifié facultativement substitué avec un groupe phényle ou alcoxy en $C_1$-$C_4$ ou un à trois groupes halogéno ;

un groupe cycloalkyle en $C_3$-$C_6$ facultativement substitué avec un groupe méthyle, halogéno ou alcoxy en $C_1$-$C_4$ ;

un groupe (alkyl en $C_1$-$C_4$)carbonyle facultativement substitué avec un groupe alcoxy en $C_1$-$C_4$ ou un à trois groupes halogéno ;

un groupe (alkyl en $C_1$-$C_4$)carbonylamino facultativement substitué sur l'azote avec un groupe alkyle en $C_1$-$C_2$ ou alcoxy en $C_1$-$C_4$ et sur le carbone avec un groupe alcoxy en $C_1$-$C_4$ ou un à trois groupes halogéno ;

un groupe alkyle en $C_1$-$C_4$ substitué avec un groupe alcoxy en $C_1$-$C_4$, alkylthio en $C_1$-$C_4$, alkylamino en $C_1$-$C_4$, di(alkyl en $C_1$-$C_4$)amino, tri(alkyl en $C_1$-$C_4$)ammonium ou (alkyl en $C_1$-$C_4$) carbonyloxy ;

un groupe 2(ou 3)-furyle facultativement substitué avec un groupe halogéno ou alkyle en $C_1$-$C_3$ ;

un groupe 2(ou 3)-thiényle facultativement substitué avec un groupe halogéno ou alkyle en $C_1$-$C_3$ ;

un groupe N-méthyl-2(ou 3)-pyrrolyle ;

un groupe hydroxy ;

un groupe amino facultativement substitué avec un groupe alcoxy en $C_1$-$C_4$, alkyle en $C_1$-$C_2$ ou alcoxy en $C_1$-$C_4$ ;

un groupe 2(ou 4)-pyridyloxy facultativement substitué avec un groupe alkyle en $C_1$-$C_4$ ou trifluorométhyle ou un ou deux groupes halogéno ;

un groupe mono- ou di(alkyl en $C_1$-$C_4$)aminocarbonylamino facultativement substitué sur l'azote fixé au cycle avec un groupe alkyle en $C_1$-$C_2$ ou alcoxy en $C_1$-$C_4$ ;

un groupe (alcoxy en $C_1$-$C_4$)carbonylamino facultativement substitué sur l'azote avec un groupe alkyle en $C_1$-$C_2$ ou alcoxy en $C_1$-$C_4$ ;

un groupe mono- ou di(alkyl en $C_1$-$C_4$)aminocarbonyloxy ; ses N-oxydes, sous réserve que $R_3$ ne soit pas un groupe alkyle insaturé ;

ses isomères optiques lorsque $R_1$ et $R_2$ ne sont pas identiques ;

ses tautomères ; et

ses sels d'addition d'acides sauf lorsque $R_3$ est un cation.

**Revendications pour les Etats contractants suivants : AT, ES**

**1.** Procédé pour la préparation d'un composé de formule développée :

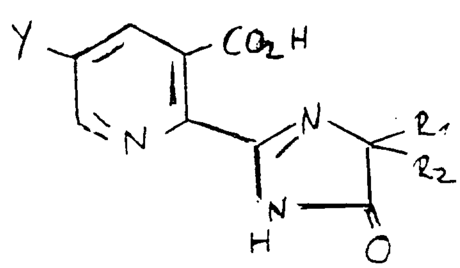

dans laquelle $R_1$ et $R_2$ représentent indépendamment un groupe alkyle en $C_1$-$C_4$, et Y représente un groupe alcényle en $C_2$-$C_6$ linéaire ou ramifié éventuellement substitué par phényle, alcoxy en $C_1$-$C_4$ ou 1 à 3 atomes d'halogène, comprenant les étapes consistant à oxyder un composé hydroxyméthylé de formule développée :

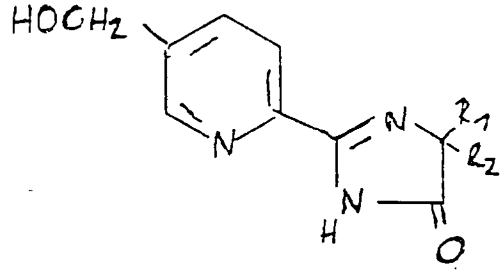

dans laquelle $R_1$ et $R_2$ sont tels que définis ci-dessus avec du dioxyde de manganèse, à température élevée, en présence d'un solvant organique inerte de façon à former un dérivé carboxaldéhyde de formule développée :

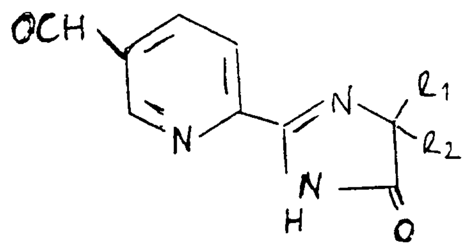

dans laquelle $R_1$ et $R_2$ sont tels que définis ci-dessus, faire réagir le dérivé carboxaldéhyde avec un dérivé halogénure d'alkyl-triphénylphosphonium approprié en présence d'un hydrure de métal alcalin, d'une huile minérale et de diméthylsulfoxide de façon à former un dérivé 5-alcényle de formule développée :

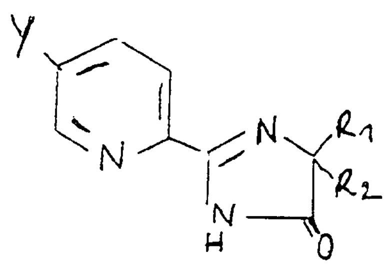

dans laquelle Y est tel que défini ci-dessus, et faire réagir le dérivé 5-alcényle avec du méthyl-lithium et du dioxyde de carbone en présence de tétrahydrofurane.

**2.** Procédé pour la préparation d'acide de 2-(4-isopropyl-4-méthyl-5-oxo-2-imidazolin-2-yl)-5-(methoxyméthyl)nicotinique comprenant les étapes consistant à faire réagir le 5-(bromométhyl)-2-(4-isopropyl-4-méthyl-5-oxo-2-imidazolin-2-yl)nicotinate de méthyle avec du méthylate de potassium de façon à conduire à du 2-(4-isopropyl-4-méthyl-5-oxo-2-imidazolin-2-yl)-5-(méthoxméthyl)nicotinate de méthyle, faire réagir le 5-(méthoxyméthyle)nicotinate avec de l'hydroxyde de sodium en présence de méthanol de façon à conduire à un sel de l'acide 5-(méthoxyméthyl)nicotinique, et faire réagir le sel de l'acide avec de l'acide chlorhydrique..